(19) Europäisches Patentamt
European Patent Office
Office européen des brevets

(11) Veröffentlichungsnummer: **0 014 470**
**B1**

(12) # EUROPÄISCHE PATENTSCHRIFT

(45) Veröffentlichungstag der Patentschrift: **10.07.85**

(21) Anmeldenummer: **80100610.7**

(22) Anmeldetag: **06.02.80**

(51) Int. Cl.⁴: **C 07 D 487/04**, C 07 D 239/26, C 07 D 239/30, C 07 D 239/42, A 61 K 31/55 // C07D317/16, C07D317/30, C07D223/16, C07D405/10, C07C69/76, C07C121/52 ,(C07D487/04, 239:00, 223:00)

(54) **Benzazepin-Derivate, Verfahren und Zwischenprodukte zu deren Herstellung, deren Verwendung sowie diese enthaltende Arzneimittel.**

(30) Priorität: **07.02.79 US 10118**
**01.03.79 US 16709**
**22.01.80 CH 510/80**

(43) Veröffentlichungstag der Anmeldung: **20.08.80 Patentblatt 80/17**

(45) Bekanntmachung des Hinweises auf die Patenterteilung: **10.07.85 Patentblatt 85/28**

(84) Benannte Vertragsstaaten: **AT BE CH DE FR IT LU NL SE**

(56) Entgegenhaltungen:
**DE - A - 2 524 048**

**JOURNAL OF THE CHEMICAL SOCIETY, Perkin Transactions I, Nr. 8, August 1978 London, G.B. G.R. PROCTOR: "Azabenzocycloheptenones. Part 19. Formation of Some Heterocyclic Annelated Compounds from 1,2,3,4-Tetrahydro-1-benzazepine Derivatives"**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Fryer, Rodney Ian, 5 Eton Drive, North Caldwell, N.J. (US)**
Erfinder: **Gilman, Norman W., 5 Normandy Drive, Wayne, N.J. (US)**
Erfinder: **Trybulski, Eugene J., 345 Claremont Avenue, Montclair, N.J. (US)**
Erfinder: **Walser, Armin, 19 Crane Avenue, West Caldwell, N.J. (US)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 014 470 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Pyrimido-2-benzazepine der allgemeinen Formel

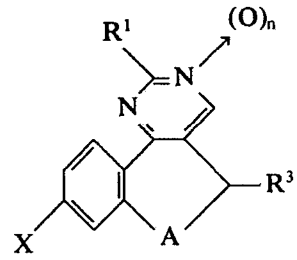

(I)

worin A eine der Gruppen

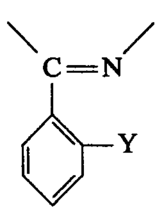
(a)

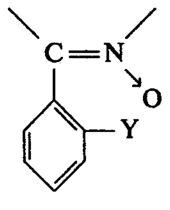
(b)

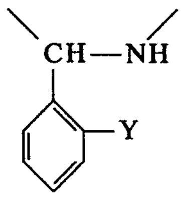
(c)

und

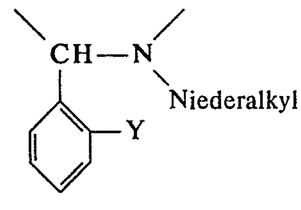
(d)

$R^1$ Wasserstoff, Chlor, Brom, niederes Alkyl, die Gruppe $NR^4R^5$, die Gruppe $-CH_2-CO-R^7$, die Gruppe $-NH(CH_2)_mNR^8R^9$, Hydroxy, niederes Alkoxy, Mercapto oder niederes Alkylmercapto, $R^3$ Wasserstoff, niederes Acyloxy oder Hydroxy, X Wasserstoff, Halogen, Trifluormethyl, Äthyl, $\alpha$-Hydroxyäthyl oder Acetyl, Y Wasserstoff oder Halogen, $R^4$ und $R^5$ je Wasserstoff oder niederes Alkyl oder, zusammen mit dem Stickstoffatom, einen 5- bis 7gliedrigen Heterocyclus, welcher ein Sauerstoff- oder Schwefelatom oder die Gruppe $>$N-Niederalkyl enthalten kann, $R^7$ Hydroxy, niederes Alkoxy oder $NR^8R^9$, $R^8$ und $R^9$ je Wasserstoff oder niederes Alkyl, n 0 oder 1 und m 1 bis 7 bedeuten, mit der Maßgabe, daß die als »nieder« bezeichneten Gruppen höchstens 7 Kohlenstoffatome aufweisen und mit den folgenden Maßgaben:

(i) wenn $R^3$ niederes Acyloxy oder Hydroxy bedeutet, dann bedeuten A Gruppe (a), X Wasserstoff, Halogen, Trifluormethyl, Äthyl oder Acetyl und, falls $R^1$ die Gruppe $-NH(CH_2)_m$ $NR^8R^9$ bedeutet, dann bedeuten $R^8$ und $R^9$ je niederes Alkyl;
(ii) wenn A Gruppe (d) und $R^1$ Gruppe $-NH(CH_2)_mNR^8R^9$ bedeuten, dann bedeuten $R^8$ und $R^9$ je niederes Alkyl; und
(iii) wenn n 1 bedeutet, dann bedeuten entweder A Gruppe (b) und $R^1$ Wasserstoff, niederes Alkyl, Hydroxy, niederes Alkoxy, $NR^{41}R^{51}$ (worin $R^{41}$ und $R^{51}$ je Wasserstoff oder niederes Alkyl oder, zusammen mit dem Stickstoffatom, einen 5- bis 7gliedrigen Heterocyclus, welcher ein Sauerstoffatom enthalten kann, bedeuten), Chlor, Brom oder die Gruppe $-CH_2-CO-R^7$, oder es bedeuten A Gruppe (a) und $R^1$ Wasserstoff, niederes Alkyl, niederes Alkoxy, Chlor, Brom oder Gruppe $-CH_2-CO-R^7$;

und pharmazeutisch akzeptable Säureadditionssalze davon. Diese Verbindungen zeigen pharmakologische Aktivität als Anxiolytica und Sedativa.

In DE-A1-2 524 048 sind 6-Aryl-pyrazolo[4,3-d] [2]benzazepine mit antidepressiven und anxiolytischen Eigenschaften beschrieben.

In der vorliegenden Beschreibung bezeichnet der Ausdruck »niederes Alkyl« geradkettige oder verzweigte Kohlenwasserstoffgruppen mit 1—7, vorzugsweise 1—4 Kohlenstoffatomen, wie Methyl, Äthyl, Propyl, Isopropyl und dergleichen.

Sofern nicht ausdrücklich anders angegeben, bezeichnet der Ausdruck »Halogen« die 4 Formen Fluor, Chlor, Jod und Brom.

Die Ausdrücke »niederes Alkoxy« und »niederes Alkylmercapto« bezeichnen Reste der Formel —O-Niederalkyl bzw. —S-Niederalkyl, wobei »Niederalkyl« im Sinne der obigen Definition aufzufassen ist.

Wenn der Ausdruck »Nucleophile des Heteroatom- oder Kohlenstoff-Typs« verwendet wird, so sind damit Agenzien gemeint, wie sie in der belgischen Patentschrift Nr. 833 249 beschrieben sind, deren Inhalt hierin als eingefügt anzusehen ist.

Der Ausdruck »pharmazeutisch akzeptable Salze« umfaßt Salze mit sowohl anorganischen als auch organischen pharmazeutisch akzeptablen starken Säuren, wie Schwefelsäure, Salzsäure, Salpetersäure, Methansulfonsäure und p-Toluolsulfonsäure. Solche Salze können vom Fachmann sehr leicht

hergestellt werden, wenn er sich den Stand der Technik und die Natur der in Salze zu überführenden Verbindung vergegenwärtigt.

Unter den Verbindungen der obigen Formel I sind diejenigen bevorzugt, worin A Gruppe (a) und n 0 bedeuten. Besonders bevorzugt sind diejenigen Verbindungen der Formel I, worin A Gruppe (a), n 0 und

- $R^3$ Wasserstoff und $R^1$ Wasserstoff, niederes Alkyl, die Gruppe $NR^4R^5$ (worin $R^4$ und $R^5$ je Wasserstoff oder niederes Alkyl bedeuten), Hydroxy, Chlor, Brom, die Gruppe

  $-NH-(CH_2)_mNR^8R^9$

  (worin $R^8$ und $R^9$ je niederes Alkyl bedeuten) oder die Gruppe $-CH_2-CO-R^7$ (worin $R^7$ obige Bedeutung besitzt) bedeuten, wobei als Bedeutungsmöglichkeiten von $R^1$ Wasserstoff, Amino und niederes Alkyl besonders bevorzugt sind; oder
- $R^3$ Hydroxy und $R^1$ Wasserstoff, niederes Alkyl oder die Gruppe $NR^4R^5$ (worin $R^4$ und $R^5$ je Wasserstoff oder niederes Alkyl sind) bedeuten.

Weiterhin sind unter den Verbindungen der Formel I diejenigen bevorzugt, worin X Halogen bedeutet, wobei Chlor besonders bevorzugt ist, und/oder worin Y Wasserstoff, Chlor oder Fluor bedeutet.

Eine besonders bevorzugte Verbindung im Rahmen der vorliegenden Erfindung ist das 9-Chlor-7-(2-chlorphenyl)-5H-pyrimido[5,4-d] [2]benzazepin.

Weitere Beispiele bevorzugter Verbindungen im Rahmen der vorliegenden Erfindung sind

- 9-Chlor-7-(2-fluorphenyl)-5H-pyrimido[5,4-d] [2]benzazepin;
- 9-Chlor-7-(2-chlorphenyl)-2-methyl-5H-pyrimido-[5,4-d] [2]benzazepin;
- 9-Chlor-7-(2-fluorphenyl)-5H-pyrimido[5,4-d] [2]benzazepin-2-ol und
- 9-Chlor-N,N-dimethyl-7-(2-fluorphenyl)-5H-pyrimido[5,4-d] [2]benzazepin-1-amin.

Die Verbindungen der Formel I und pharmazeutisch akzeptable Säureadditionssalze davon können erfindungsgemäß dadurch hergestellt werden, daß man

a) eine Verbindung der allgemeinen Formel

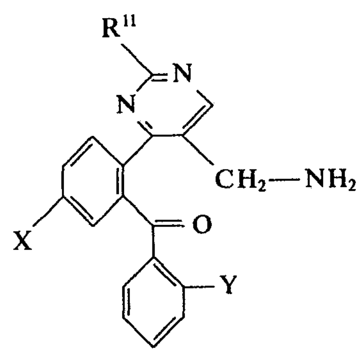

(II)

worin X und Y obige Bedeutung besitzen und $R^{11}$ Wasserstoff, niederes Alkyl oder $NR^8R^9$, worin $R^8$ und $R^9$ obige Bedeutung besitzen, bedeutet,
cyclisiert oder

b) eine Verbindung der allgemeinen Formel

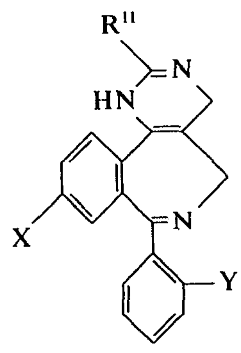

(III)

worin X, Y und $R^{11}$ obige Bedeutung besitzen,
dehydriert oder

c) eine Verbindung der allgemeinen Formel

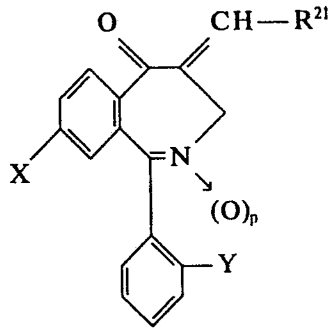

(IV)

worin X und Y obige Bedeutung besitzen und p 0 oder 1 und $R^{21}$ Di-(nieder)-alkylamino bedeuten, mit einer Verbindung der allgemeinen Formel

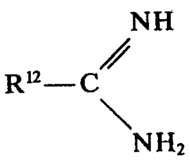

(V)

worin $R^{12}$ Wasserstoff, Mercapto, niederes Alkylmercapto, niederes Alkyl oder $NR^8R^9$, worin $R^8$ und $R^9$ obige Bedeutung besitzen, bedeutet,
umsetzt oder

d) eine Verbindung der allgemeinen Formel

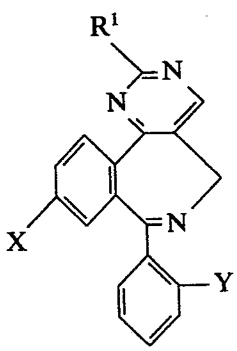

(Ia)

worin X, Y und $R^1$ obige Bedeutung besitzen,
reduziert oder

e) eine Verbindung der allgemeinen Formel

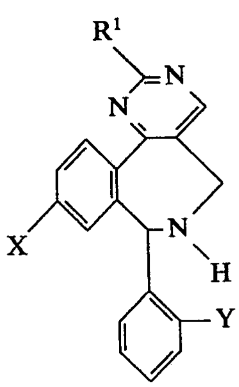

(Ib)

worin X, Y und $R^1$ obige Bedeutung besitzen,
niederalkyliert oder

f) eine Verbindung der allgemeinen Formel

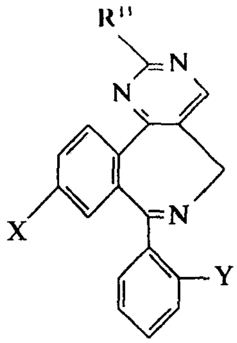

(Ic)

worin X und Y obige Bedeutung besitzen und $R^{13}$ Wasserstoff, niederes Alkyl, Hydroxy, niederes Alkoxy, $NR^{41}R^{51}$ (worin $R^{41}$ und $R^{51}$ je Wasserstoff oder niederes Alkyl oder, zusammen mit dem Stickstoffatom, einen 5- bis 7gliedrigen Heterocyclus, welcher ein Sauerstoffatom enthalten kann, bedeuten), Chlor, Brom oder die Gruppe $-CH_2-CO-R^7$ (worin $R^7$ obige Bedeutung besitzt) bedeutet,
oxidiert oder

g) eine Verbindung der allgemeinen Formel

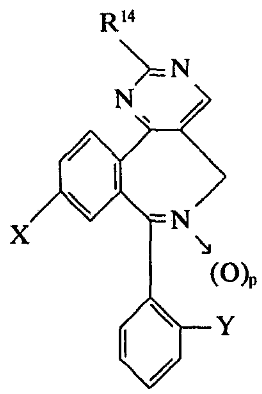

(Id)

worin X, Y und p obige Bedeutung besitzen und $R^{14}$ Mercapto oder Hydroxy bedeuten,
niederalkyliert oder

h) eine Verbindung der allgemeinen Formel

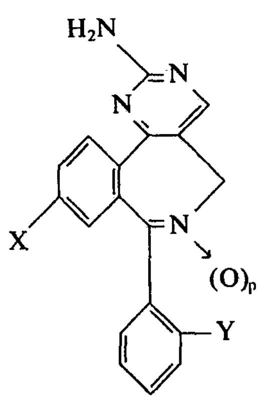

(Ie)

worin X, Y und p obige Bedeutung besitzen,
in die entsprechende 2-Hydroxyverbindung überführt oder

i) eine Verbindung der allgemeinen Formel

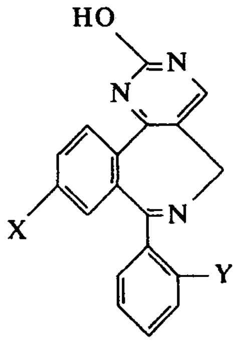

(If)

worin X und Y obige Bedeutung besitzen, in eine entsprechende 2-Chlor- oder 2-Bromverbindung überführt oder

k) eine Verbindung der allgemeinen Formel

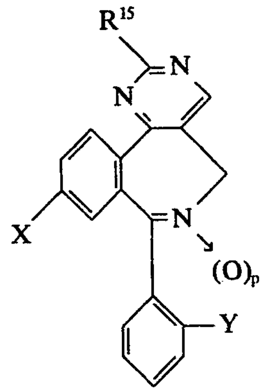

(Ig)

worin X, Y und p obige Bedeutung besitzen und $R^{15}$ Chlor oder Brom bedeutet, mit Schwefelwasserstoff, mit einem niederen Alkylmercaptan, mit einem niederen Alkanol, mit einer Verbindung der Formel $HNR^4R^5$, worin $R^4$ und $R^5$ obige Bedeutung besitzen, mit einer Verbindung der Formel $H_2N-(CH_2)_mNR^8R^9$, worin $R^8$, $R^9$ und m obige Bedeutung besitzen, oder mit dem Carbanion einer Verbindung der Formel

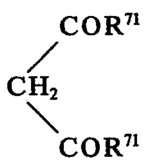 oder 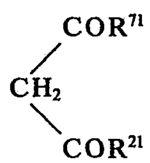

worin $R^{71}$ niederes Alkoxy bedeutet und $R^{21}$ obige Bedeutung besitzt, umsetzt oder

l) eine Verbindung der allgemeinen Formel

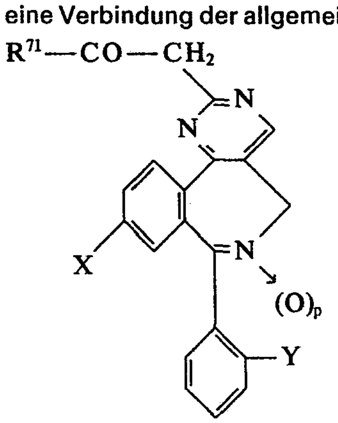

(Ih)

worin $R^{71}$, X, Y und p obige Bedeutung besitzen, in die entsprechende freie Säure oder in ein entsprechendes Amid, Niederalkylamid oder Di-niederalkylamid überführt oder

m) eine Verbindung der allgemeinen Formel

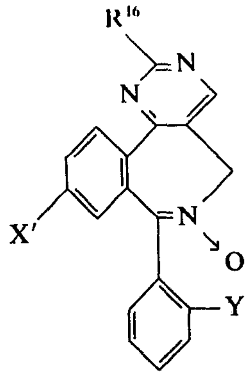

(Ii)

worin Y obige Bedeutung besitzt, $R^{16}$ Wasserstoff, Chlor, Brom, niederes Alkyl, die Gruppe $NR^4R^5$, die Gruppe $-CH_2-CO-R^7$, die Gruppe $-NH(CH_2)_mNR^{81}R^{91}$, Hydroxy, niederes Alkoxy, Mercapto oder niederes Alkylmercapto, X' Wasserstoff, Halogen, Trifluormethyl, Äthyl oder Acetyl und $R^{81}$ und $R^{91}$ je niederes Alkyl bedeuten und $R^4$, $R^5$ und $R^7$ obige Bedeutung besitzen, mit einem Niederacylierungsmittel zur Reaktion bringt oder

n) eine Verbindung der allgemeinen Formel

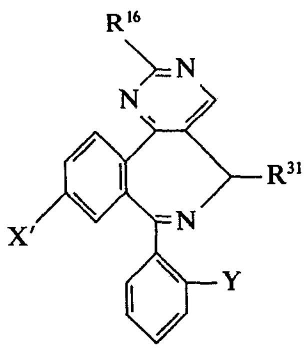

(Ik)

worin X', Y und $R^{16}$ obige Bedeutung besitzen und $R^{31}$ niederes Acyloxy bedeutet, hydrolysiert oder

o) eine Verbindung der allgemeinen Formel

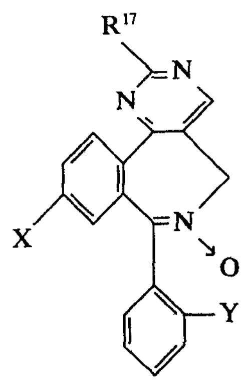

(Il)

worin X und Y obige Bedeutung besitzen, $R^{17}$ Wasserstoff, niederes Alkyl, die Gruppe $NR^4R^5$, die Gruppe $-CH_2-CO-R^7$, die Gruppe $NH-(CH_2)_mNR^8R^9$, Hydroxy, niederes Alkoxy, Mercapto oder niederes Alkylmercapto bedeutet und $R^4$, $R^5$, $R^7$, $R^8$ und $R^9$ obige Bedeutung besitzen, deoxygeniert oder

p) aus einer Verbindung der allgemeinen Formel

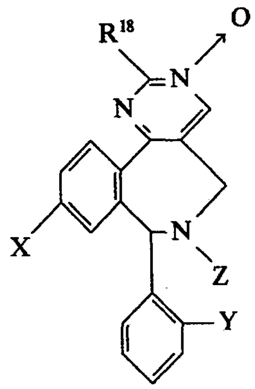

(III')

worin X und Y obige Bedeutung besitzen und $R^{18}$ Wasserstoff, niederes Alkyl, niederes Alkoxy, Chlor, Brom oder die Gruppe $-CH_2-CO-R^7$ (worin $R^7$ obige Bedeutung besitzt) und Z Wasserstoff oder eine leicht abspaltbare Acylgruppe bedeuten, die Elemente von H—Z entfernt oder

q) eine Verbindung der Formel I in ein pharmazeutisch akzeptables Säureadditionssalz überführt.

Es ist zu beachten und jedem Fachmann klar, daß, wenn die irgendeiner der vorerwähnten Reaktionen zu unterziehenden Verbindung zusätzlich zu der bzw. den in die Reaktion involvierten Gruppe(n) Gruppen enthält, welche unter den Bedingungen einer solchen Reaktion verwundbar sein könnten, entweder eine Methode zur Durchführung einer derartigen Reaktion verwendet werden muß, welche eine derartige verwundbare Gruppe nicht angreift (sofern eine solche Methode sich anbietet) oder eine derartige verwundbare Gruppe vor der Durchführung der Reaktion geschützt werden und hernach die Schutzgruppe wieder entfernt werden muß.

Die obigen Verfahrensvarianten a) und b) sowie die Herstellung der hierfür benötigten Ausgangsprodukte werden im Sinne eines Beispiels durch das folgende Reaktionsschema I illustriert, worin X, Y und $R^{11}$ obige Bedeutung besitzen:

Reaktionsschema I

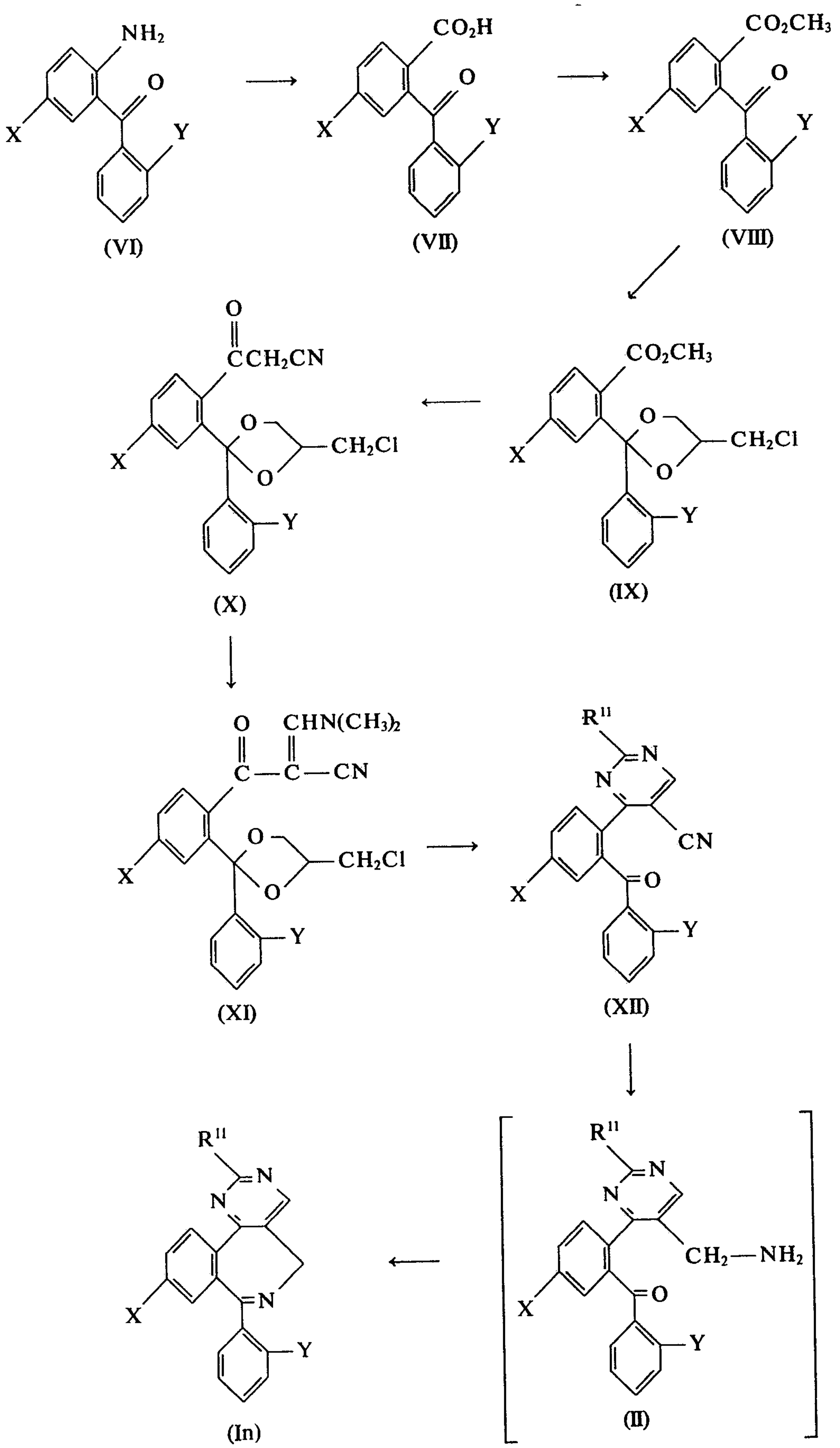

(VI) (VII) (VIII)

(X) (IX)

(XI) (XII)

(In) (II)

Die Verbindungen VI, VII und VIII gehören an sich bekannten Stoffklassen an. Eine Methode für deren Herstellung wird der Vollständigkeit halber erläutert.

VI → VII

Die wohlbekannte Verbindung der Formel VI wird mit Kupfer(I)-cyanid zur Reaktion gebracht, nachdem sie vorgängig nacheinander mit Schwefelsäure, Natriumnitrit und fakultativ Natriumtetrafluoroborat behandelt worden ist. Anschließend wird die Mischung mit einem Alkalimetallhydroxid, z. B. Natriumhydroxid oder Kaliumhydroxid, bei Rückflußtemperatur hydrolysiert.

VII → VIII

Die Verbindung der Formel VIII bildet sich bei der Reaktion der Carbonsäure der Formel VII mit Methanol in Gegenwart eines sauren Katalysators, wie Schwefelsäure, bei etwa der Rückflußtemperatur von Methanol während etwa 3—18 Stunden.

VIII → IX

Die Verbindung der Formel VIII behandelt man anschließend mit 1-Chlor-2,3-epoxypropan oder anderen geeigneten Ketalisierungsreagenzien von Epoxid-Typus, wie Äthylenoxid und 1,2-Epoxypropan, in Gegenwart einer Lewis-Säure, wie Aluminiumchlorid, Bortrifluorid oder vorzugsweise Zinntetrachlorid, in einem inerten organischen Lösungsmittel, wie Toluol, Tetrachlorkohlenstoff oder Benzol. Die Reaktionstemperatur liegt zweckmäßigerweise zwischen etwa 20° C und 110° C, vorzugsweise bei etwa 25° C.

IX → X

Hierauf behandelt man die Verbindung der Formel IX mit Acetonitril ($CH_3CN$) in Gegenwart eines Alkalimetallamids, wie Natriumamid oder Kaliumamid, in flüssigem Ammoniak in Gegenwart eines inerten organischen Lösungsmittels, wie Diäthyläther, Tetrahydrofuran oder Dioxan. Die Reaktionstemperatur kann zwischen etwa der Rückflußtemperatur von flüssigem Ammoniak und Raumtemperatur liegen.

X → XI

Die Verbindung der Formel X wird danach mit einem Dialkoxyacetal von Dimethylformamid, wie Dimethylformamid-dimethylacetal, zur Reaktion gebracht. Geeignete Reaktionstemperaturen liegen zwischen etwa 25° C und 120° C, wobei die Rückflußtemperatur des Dimethylacetals bevorzugt ist.

XI → XII

Die Umwandlung der Verbindungen der Formel XI in die Verbindungen der Formel XII umfaßt 2 Stufen, nämlich Reaktion mit einem geeigneten Säureadditionssalz eines Amidins oder Guanidins der Formel

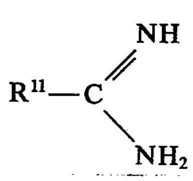

(Va)

worin $R^{11}$ obige Bedeutung besitzt,
und saure Hydrolyse der Ketalfunktion. Wenn X und Y beide Wasserstoff sind, so erfolgt die saure Hydrolyse der Ketalfunktion vorzugsweise vor der Reaktion mit dem Amidin- oder Guanidinsalz; ist dagegen X und/oder Y von Wasserstoff verschieden, so erfolgt die saure Hydrolyse der Ketalfunktion vorzugsweise nach der Reaktion mit dem Amidin- oder Guanidinsalz. Zwischenprodukte müssen aus dem Reaktionsgemisch entfernt werden, bevor weiter gearbeitet wird.

Die Reaktion mit einem geeigneten Säureadditionssalz eines Amidins oder Guanidins der Formel Va, wie Formamidin-acetat, Acetamidin-hydrochlorid, Guanidin-carbonat oder einem substituierten Guanidin-carbonat erfolgt zweckmäßigerweise in einem aprotischen polaren organischen Lösungsmittel, wie Dimethylsulfoxid, in einem Temperaturbereich von etwa 25° C bis 125° C, vorzugsweise in einem Bereich von etwa 90° C bis 95° C.

Die Hydrolyse der Ketalfunktion erfolgt mittels einer wäßrigen anorganischen Säure, wie Schwefelsäure, Phorphorsäure oder vorzugsweise einer Halogenwasserstoffsäure, wie Salzsäure, in Gegenwart eines niederen Alkylalkohols als Lösungsmittel, vorzugsweise Äthanol. Die Reaktion wird bei etwa Raumtemperatur durchgeführt.

XII → II

Die Verbindung der Formel II bildet sich bei der Hydrierung der Verbindung der Formel XII in Gegenwart eines Metallkatalysators, wie Platin, Palladium oder Raney-Nickel, und cyclisiert spontan zu Verbindung In. Die Hydrierung kann in einem geeigneten organischen Lösungsmittel durchgeführt werden, wie niedere Alkylalkohole oder niedere Alkylcarbonsäuren, z. B. Essigsäure. Je nach den für diese Hydrierung verwendeten Reaktionsbedingungen kann zusätzlich zur oder anstatt der Verbindung der Formel In eine Dihydropyrimidoverbindung der Formel III gebildet werden. Diese Dihydropyrimidoverbindung kann durch Behandlung mit einem milden Oxidationsmittel, wie Mangandioxid, Luft oder dergleichen, in die Verbindung der Formel In übergeführt werden.

Bei Verfahrensvariante c) handelt es sich um die Reaktion einer Verbindung der Formel IV mit einem Amidin- oder Guanidinsalz oder mit Thioharnstoff oder einem S-Niederalkyl-isothioharnstoff. Für die Reaktion mit dem Amidin- oder Guanidinsalz kann irgendein inertes organisches Lösungsmittel verwendet werden, wie Dioxan, Tetrahydrofuran oder Dimethylformamid, und die Reaktionstemperatur kann zwischen etwa Raumtemperatur und Rückflußtemperatur des Lösungsmittels liegen, wobei etwa Raumtemperatur bevorzugt ist. Die Reaktion mit Thioharnstoff oder mit einem S-Niederalkyl-isothioharnstoff kann in Gegenwart einer alkoholischen, z. B. methanolischen Lösung eines Alkalimetallalkoxids, z. B. Natriummethoxid, erfolgen. Die Reaktion kann zwischen etwa 0° C und 65° C durchgeführt werden, vorzugsweise bei etwa Raumtemperatur.

Verfahrensvarianten d) und e) werden im Sinne eines Beispiels durch das nachfolgende Reaktionsschema II illustriert, worin $R^{11}$ obige Bedeutung besitzt:

Reaktionsschema II

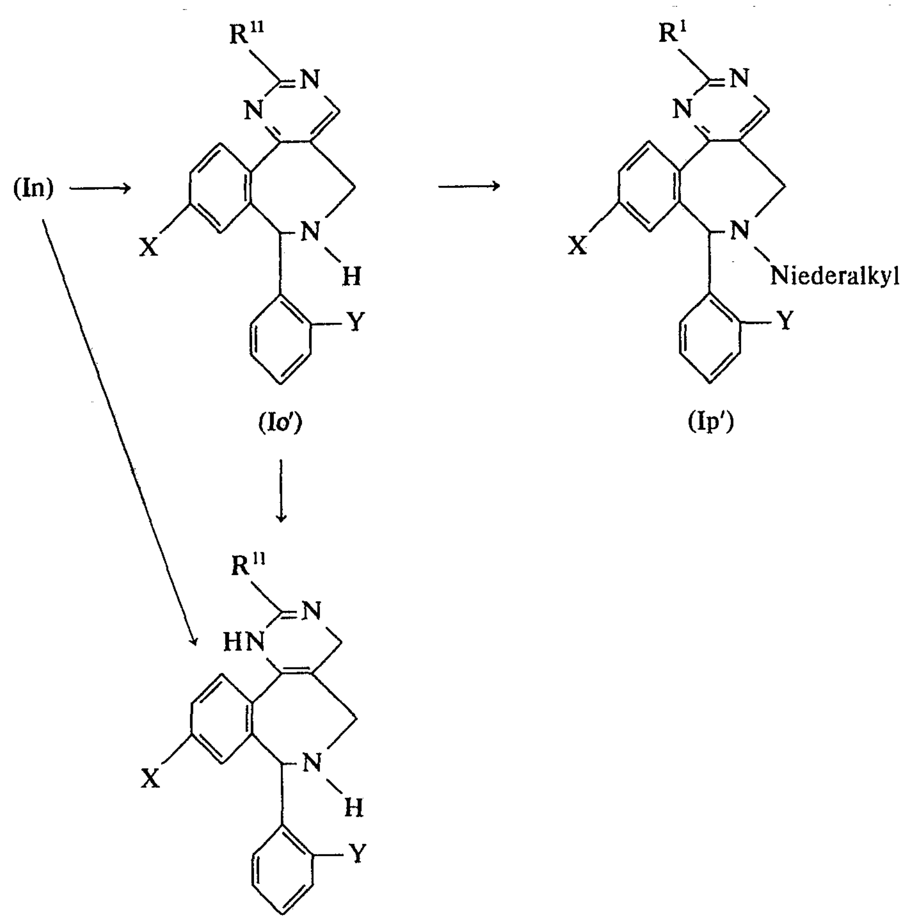

(In)

(Io')

(Ip')

(XIII)

In → Io' und In oder Io' → XIII

Durch Behandlung der Verbindung der Formel In (siehe Reaktionsschema I) mit einem Reduktionsmittel, wie Zink in Essigsäure bei einer Reaktionstemperatur von etwa −40° C bis 20° C erhält man Io'. Als Lösungsmittel kann man einen halogenierten Kohlenwasserstoff, wie Methylenchlorid, verwenden.

Durch Behandlung der Verbindung der Formel In oder Io' mit Wasserstoff in Gegenwart von Platinoxid oder vorhydriertem Platinoxid und in Essigsäure als Lösungsmittel erhält man XIII. Die Reaktion wird normalerweise bei etwa Raumtemperatur durchgeführt.

Andere für Verfahrensvariante d) geeignete Reduktionsmittel umfassen Natrium-cyanborhydrid, Natriumborhydrid und dergleichen.

In manchen Fällen erhält man Mischungen der 6,7-Dihydro- und der 4,5,6,7-Tetrahydroverbindungen. Die erwähnten 4,5,6,7-Tetrahydroverbindungen zeigen ebenfalls nützliche Wirksamkeiten auf das zentrale Nervensystem.

Io' → Ip'

Bei der Behandlung der Verbindung der Formel Io' mit Formaldehyd in Ameisensäure unter Erhitzen, beispielsweise auf etwa Rückflußtemperatur, wird die Dihydroverbindung methyliert. Die Methylierung kann auch beispielsweise mit Methyliodid, Dimethylsulfat oder dergleichen durchgeführt werden.

Von Methyl verschiedene Niederalkyl-Substituenten erhält man unter Verwendung geeigneter Alkyl-Halogenide, -Sulfate, -Alkylsulfonate, -Tosylate oder dergleichen in einem Lösungsmittel, wie Dimethylformamid, Tetrahydrofuran, Glyme und Diglyme oder ätherische Lösungsmittel, oder unter Verwendung geeigneter Aldehyde unter reduktiven Reaktionsbedingungen.

Verfahrensvariante f) wird im Sinne eines Beispiels durch das nachfolgende Reaktionsschema III illustriert, worin $R^{11}$ obige Bedeutung besitzt:

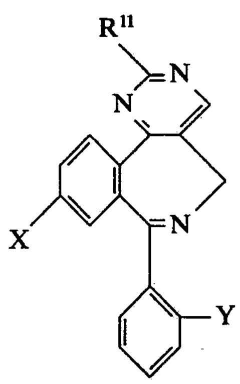

(In)

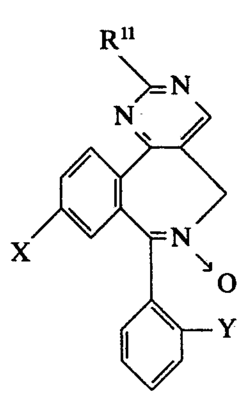

(Iq)

+

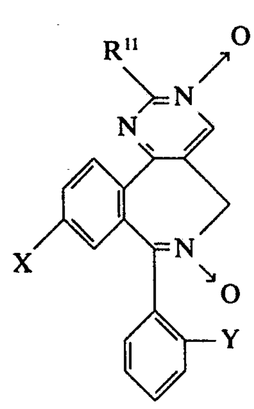

(Ir)

Die Verbindung der Formel In behandelt man mit einem geeigneten Oxidationsmittel, wie Metachlorperbenzoesäure, in einem inerten organischen Lösungsmittel, wie Methylenchlorid. Die Reaktion kann zwischen etwa 0°C und Raumtemperatur durchgeführt werden, vorzugsweise bei etwa Raumtemperatur. Die Reaktionszeit kann variiert werden, je nachdem, ob man als Produkt das N-Oxid oder das Di-N-oxid wünscht. Wenn die Reaktionszeit zwischen etwa 2 und 25 Stunden liegt, so erhält man überwiegend das N-Oxid Iq; liegt dagegen die Reaktionszeit zwischen etwa 40 und 60 Stunden, so entsteht überwiegend das Di-N-oxid Ir.

Gemäß Verfahrensvariante g) wird eine Mercapto- oder Hydroxygruppe nach an sich bekannten Methoden niederalkyliert. Die Alkylierung der Mercaptogruppe erfolgt zweckmäßigerweise mittels eines geeigneten Alkylhalogenids in Gegenwart einer Mischung eines Alkalimetallhydroxids, wie Natriumhydroxid, und eines Alkohols ($C_1-C_4$), wie Äthanol. Die Reaktion kann vorzugsweise bei etwa Raumtemperatur durchgeführt werden.

Die Alkylierung der Hydroxygruppe erfolgt zweckmäßigerweise mittels eines Dialkylsulfats, wie Dimethyl- oder Diäthylsulfat, unter basischen Bedingungen, beispielsweise in Gegenwart von Natriumhydroxid. Die Reaktion kann zwischen etwa 0°C und 65°C durchgeführt werden, vorzugsweise etwa bei Raumtemperatur.

Die Übeführung einer 2-Aminoverbindung in die entsprechende 2-Hydroxyverbindung gemäß Verfahrensvariante h) kann mittels einer Säure, wie Schwefelsäure, durchgeführt werden. Die Reaktionstemperatur kann zwischen etwa 25°C und 125°C variieren, wobei etwa 100°C als Reaktionstemperatur bevorzugt ist.

Andere Methoden, welche für diese Überführung verwendet werden können, umfassen alkalische Hydrolyse und Substitution von Diazoniumsalzen.

Die Umwandlung einer 2-Hydroxyverbindung in die entsprechende 2-Chlor- oder -Bromverbindung gemäß Verfahrensvaraínte i) kann mit einem geeigneten Chlorierungsmittel, wie Phosphortrichlorid bei Rückflußtemperatur der Mischung, bzw. mit einem geeigneten Bromierungsmittel, wie Phosphorylbromid oder Phosphorpentabromid, zwischen etwa Raumtemperatur und Rückflußtemperatur durchgeführt werden.

Gemäß Verfahrensvariante k) kann das Chlor- oder Bromatom in 2-Stellung der Verbindung der Formel Ig durch nucleophile Substitution mit verschiedenen Nucleophilen vom Heteroatom- und vom Kohlenstoff-Typ, wie Methanol, 3-Dimethylaminopropylamin, Methylamin, Dimethylamin, N-Methylpiperazin, das Carbanion von Malonsäurediäthylester und dergleichen, ersetzt werden; man kan in einem inerten polaren organischen Lösungsmittel, wie Dimethylformamid, zwischen etwa 0°C und Rückflußtemperatur des Lösungsmittels arbeiten, vorzugsweise bei Raumtemperatur.

Die Umwandlung einer Verbindung der Formel Ih in die entsprechende freie Säure gemäß Verfahrensvariante l) erfolgt zweckmäßigerweise durch Hydrolyse unter alkalischen Bedingungen, beispielsweise mittels eines Alkali- oder Erdalkalimetallhydroxids, wie Natriumhydroxid, Kaliumhydroxid oder dergleichen, in Gegenwart eines geeigneten Lösungsmittels, beispielsweise eines niederen Alkanols, wie Äthanol.

Die Umwandlung einer Verbindung der Formel Ih in ein entsprechendes Amid gemäß Verfahrensvariante l) kann entweder durch Behandlung mit Ammoniak oder einem geeigneten Mono- oder Di-nieder-alkylamin bei Raumtemperatur oder erhöhter Temperatur (zweckmäßigerweise etwa 50—130°C) in Gegenwart eines inerten organischen Lösungsmittels, wie ein niederes Alkanol, z. B. Äthanol, erfolgen oder durch Hydrolyse zur entsprechenden freien Säure, Umwandlung dieser freien Säure in ein reaktionsfähiges Derivat, wie ein Säurechlorid oder ein gemischtes Anhydrid, und anschließende Behandlung dieses reaktionsfähigen Derivats mit Ammoniak oder einem geeigneten Mono- oder Di-nieder-alkylamin.

Verfahrensvarianten m) und n) werden im Sinne eines Beispiels durch das nachfolgende Reaktionsschema IV illustriert, worin X', Y, $R^{11}$ und $R^{31}$ obige Bedeutung besitzen:

Reaktionsschema IV

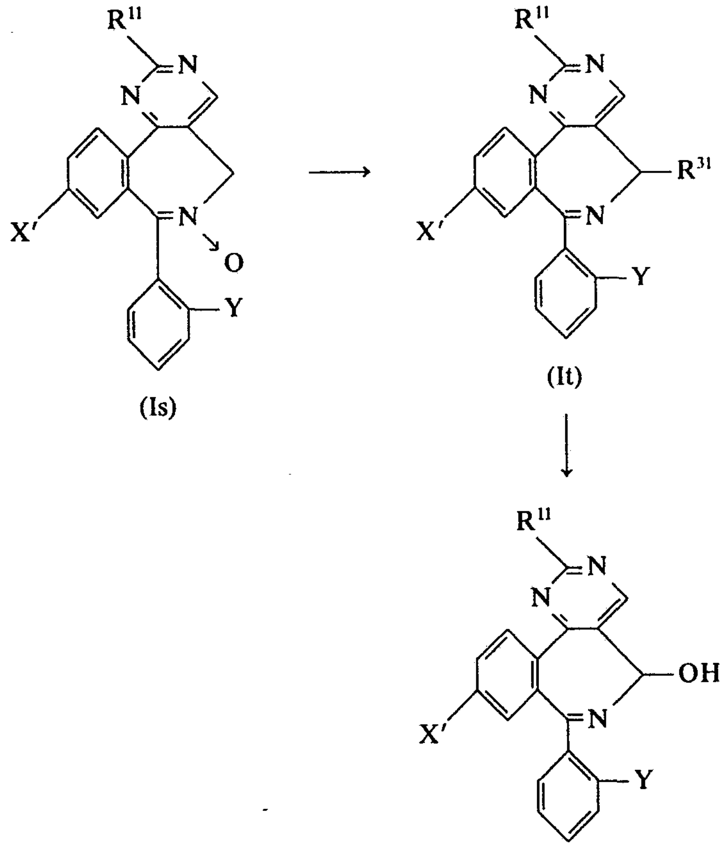

(Is) (It) (Iu)

Is → It

Die Verbindung der Formel Is kann mit einem Säureanhydrid einer geeigneten Carbonsäure, wie Essigsäure- oder Trifluoressigsäureanhydrid, zur Reaktion gebracht werden. Die Reaktion wird vorzugsweise bei etwa der Rückflußtemperatur des verwendeten Anhydrids ausgeführt. Anstelle eines Säureanhydrids kann auch ein Säurechlorid verwendet werden.

It → Iu

Die Verbindung der Formel It kann mit einem Alkalimetallcarbonat zur Reaktion gebracht werden, z. B. mit Natrium- oder Kaliumcarbonat in einem Alkohol ($C_1-C_4$), wie Methanol, bei einer Temperatur zwischen etwa 0° C und 65° C, vorzugsweise etwa bei Raumtemperatur. Andere für diesen Schritt geeignete Reaktionsbedingungen umfassen die Verwendung von Alkalimetallhydroxiden, beispielsweise Natrium- oder Kaliumhydroxid, und eines Alkohols ($C_1-C_5$) in Gegenwart von Wasser oder, andererseits, eine saure Hydrolyse unter Verwendung einer wäßrigen Mineralsäure, wie Salzsäure oder Schwefelsäure, in einem Lösungsmittel, wie Tetrahydrofuran oder Dioxan, und bei einer Reaktionstemperatur zwischen etwa 0° C und Raumtemperatur, vorzugsweise bei 0° C.

Die Deoxygenierung gemäß Verfahrensvariante o) kann nach an sich bekannten Methoden durchgeführt werden, beispielsweise mittels Reagenzien wie Phosphortrichlorid, Tri-nieder-alkyl-phosphite (z. B. Triäthylphosphit), Hexachlordisilan, Raney-Nickel und dergleichen.

Verfahrensvariante p) und die Herstellung der dafür benötigten Ausgangsprodukte werden im Sinne eines Beispiels durch das nachfolgende Reaktionsschema V illustriert, worin X, Y und $R^{18}$ obige Bedeutung besitzen und Z' eine leicht abspaltbare Acylgruppe bedeutet:

Reaktionsschema V

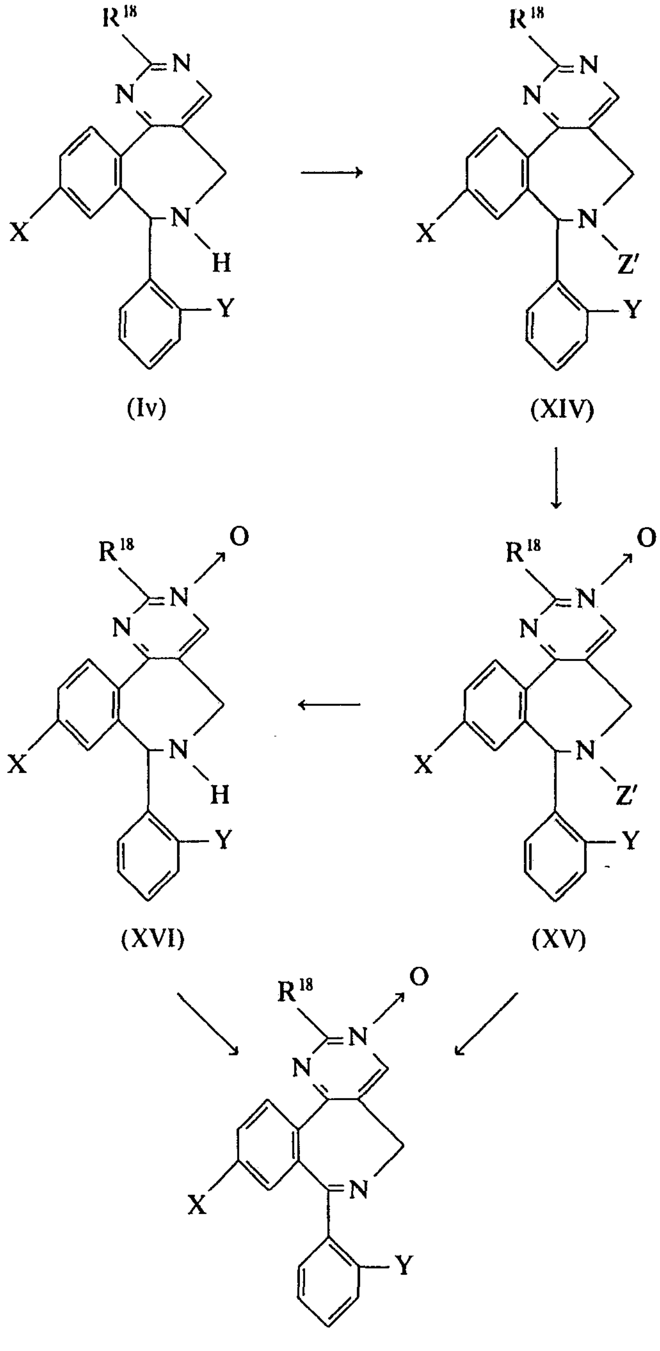

(Iv) (XIV) (XVI) (XV) (Iw)

Iv → XIV

Diese Umwandlung involviert eine Acylierung unter Verwendung eines geeigneten, die leicht abspaltbare Acylgruppe Z' liefernden Acylierungsmittels, wie Trifluoracetylchlorid, p-Toluolsulfonylchlorid, Methansulfonylchlorid, das gemischte Anhydrid von Ameisen- und Essigsäure, Chlorameisensäureäthylester, Benzyloxycarbonylchlorid und dergleichen. Die Bedingungen für die Durchführung einer derartigen Acylierung sind jedem Fachmann geläufig.

XIV → XV

Die Verbindung der Formel XIV wird mit einem geeigneten Oxydationsmittel zur Reaktion gebracht, wie Metachlorperbenzoesäure in einem inerten organischen Lösungsmittel, wie Methylenchlorid. Die Reaktion kann zwischen etwa 0°C und etwa Raumtemperatur durchgeführt werden, vorzugsweise etwa bei Raumtemperatur. Die Reaktionszeit beträgt etwa 40—60 Stunden.

XV → XVI

Die Acylgruppe Z' wird nach an sich bekannten, jedem Fachmann geläufigen Methoden abgespalten. Die Wahl der zur Anwendung gelangenden Methode hängt selbstverständlich von der Natur der Acylgruppe Z' ab. So kann beispielsweise die Trifluoracetyl- oder Äthoxycarbonylgruppe durch alkalische Hydrolyse entfernt werden, die Formylgruppe durch saure Hydrolyse, die Benzyloxycarbonylgruppe mittels Bromwasserstoff usw.

XVI oder XV → Iw

Die Umwandlung von XVI in Iw involviert eine Dehydrierung, welche nach an sich bekannten Methoden durchgeführt werden kann, beispielsweise mittels Azodicarbonsäurediäthylester oder durch N-Halogenierung (vorzugsweise N-Bromierung) und anschließende Dehydrohalogenierung unter alkalischen Bedingungen, beispielsweise mittels eines tert. Amins, wie Triäthylamin.

Wenn Z' in Formel XV eine Gruppe wie Tosyl oder Mesyl ist, so kann man eine Elimination von H—Z' unter alkalischen Bedingungen durchführen, beispielsweise mittels eines Alkalimetallalkoxids im entsprechenden Alkanol, wie methanolisches Natriummethoxid.

Falls sich das 7H-Isomere der Verbindung der Formel Iw gebildet hat, so kann es durch Behandlung mit einer geeigneten Base, beispielsweise mit methanolischem Natriummethoxid, zur Verbindung der Formel Iw isomerisiert werden.

Der Vollständigkeit halber wird die Herstellung von denjenigen unter den Zwischenprodukten der Formel IV, worin p 1 ist, im Sinne eines Beispiels durch das nachfolgende Reaktionsschema VI illustriert, worin X und Y obige Bedeutung besitzen:

Reaktionsschema VI

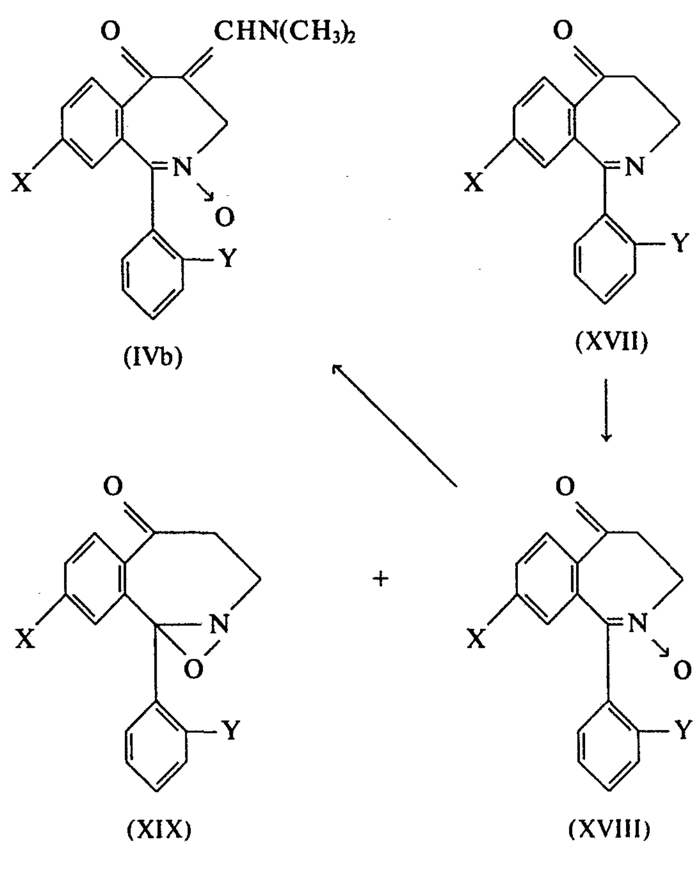

(IVb) (XVII)

(XIX) + (XVIII)

Die Verbindung der Formel XVII kann mit einer Persäure, wie Metachlorbenzoesäure, in einem inerten organischen Lösungsmittel, z. B. einem halogenierten Kohlenwasserstoff, wie Methylenchlorid, oder einem Äther, zur Reaktion gebracht werden. Die Reaktion kann zwischen etwa 0° C und 40° C erfolgen, wobei Raumtemperatur bevorzugt ist. Das Produktegemisch kann danach durch fraktionierte Kristallisation aufgetrennt werden. Dünnschichtchromatographische Analyse zeigt die Anwesenheit beider Produkte an.

Die Verbindung der Formel XVIII kann mit Dimethylformamid-dimethylacetal in einem inerten Lösungsmittel, wie halogenierte Kohlenwasserstoffe, z. B. Methylenchlorid, Dimethylformamid oder hochsiedende Äther, zur Reaktion gebracht werden. Die Reaktionstemperatur kann zwischen etwa 0° C und 100° C liegen, wobei Raumtemperatur bevorzugt ist.

Die Pyrimido-2-benzazepine der obigen Formel I und deren pharmazeutisch akzeptable Säureadditionssalze sind nützlich als Heilmittel und zeichnen sich durch sedative und anxiolytische Aktivität aus. Diese Verbindungen können in Form üblicher pharmazeutischer Zubereitungen verwendet werden; beispielsweise können sie mit üblichen organischen oder anorganischen, inerten, für parenterale oder enterale Verabreichung geeigneten Trägerstoffen vermischt werden, beispielsweise mit Wasser, Gelatine, Milchzucker, Stärke, Magnesiumstearat, Talk, vegetabilen Ölen, Gummi arabicum, Polyalkylenglykolen, Vaseline oder dergleichen. Sie können als übliche pharmazeutische Formen verabreicht werden, beispielsweise als feste Formen, wie Tabletten, Dragées, Kapseln, Suppositorien oder dergleichen, oder als flüssige Formen, wie Lösungen, Suspensionen oder Emulsionen. Darüber hinaus können die erfindungsgemäße Verbindungen enthaltenden pharmazeutischen Zubereitungen üblichen pharmazeutischen Operationen, wie Sterilisierung, unterzogen werden, und sie können übliche pharmazeutische Hilfsstoffe enthalten, wie Konservierungsmittel, Stabilisierungsmittel, Netzmittel, Emulgiermittel, Salze zur Veränderung des osmotischen Drucks oder Puffer. Die Zubereitungen können auch noch andere therapeutisch wertvolle Stoffe enthalten.

Die nachfolgende Tabelle zeigt die Resultate, welche erhalten wurden, als die nachfolgend aufgezählten Verbindungen bestimmten wohlbekannten Versuchen, wie dem Test an der geneigten Ebene, dem Kampftest, dem Test an der unanästhesierten Katze und dem Antipentamethylentetrazoltest (Metrazoltest) unterzogen wurden, ebenso auch Angaben über ihre Toxizität:

A: 9-Chlor-7-(2-chlorphenyl)-2-methyl-5H-pyrimido[5,4-d] [2]benzazepin
B: 9-Chlor-7-(2-chlorphenyl)-5H-pyrimido[5,4-d] [2]benzazepin
C: 9-Chlor-7-(2-chlorphenyl)-6,7-dihydro-2-methyl-5H-pyrimido[5,4-d] [2]benzazepin-dihydrochlorid
D: 9-Chlor-7-(2-chlorphenyl)-6,7-dihydro-2,6-dimethyl-5H-pyrimido[5,4-d] [2]benzazepin-methansulfonat (1 : 2)
E: 9-Chlor-7-(2-fluorphenyl)-5H-pyrimido[5,4-d] [2]benzazepin-2-ol
F: 9-Chlor-7-(2-fluorphenyl)-5H-pyrimido[5,4-d] [2]benzazepin
G: 9-Chlor-7-(2-fluorphenyl)-2-methoxy-5H-pyrimido[5,4-d] [2]benzazepin
H: 9-Chlor-7-(2-fluorphenyl)-N,N-dimethyl-5H-pyrimido[5,4-d] [2]benzazepin-2-amin

Tabelle

| Verbindung | Geneigte Ebene Maus ($ED_{50}$) | Kampf-Test Maus (wirksame Dosis) | Unanästh. Katze (kleinste wirksame Dosis) | Metrazol Maus ($ED_{50}$) | Toxizität Maus ($DL_{50}$) | |
|---|---|---|---|---|---|---|
| A | >400 mg/kg p.o. | 10 mg/kg p.o. | 0,5 mg/kg p.o. | 0,74 mg/kg p.o. | >1000 mg/kg p.o., | 450 mg/kg i.p. |
| B | 215 mg/kg p.o. | 2,5 mg/kg p.o. | 2,5 mg/kg p.o. | 0,59 mg/kg p.o. | >1000 mg/kg p.o., | >1000 mg/kg i.p. |
| C | >400 mg/kg p.o. | 50 mg/kg p.o. | | 2,3 mg/kg p.o. | >1000 mg/kg p.o., | 350 mg/kg i.p. |
| D | >400 mg/kg p.o. | >100 mg/kg p.o. | >20 mg/kg p.o. | 7,7 mg/kg p.o. | >1000 mg/kg p.o., | 450 mg/kg i.p. |
| E | >400 mg/kg p.o. | 50 mg/kg p.o. | 20 mg/kg p.o. | 6,3 mg/kg p.o. | >1000 mg/kg p.o., | >1000 mg/kg i.p. |
| F | >400 mg/kg p.o. | 2 mg/kg p.o. | 1 mg/kg p.o. | 0,37 mg/kg p.o. | 900 mg/kg p.o., | >1000 mg/kg i.p. |
| G | >400 mg/kg p.o. | 30 mg/kg p.o. | 20 mg/kg p.o. | 2,6 mg/kg p.o. | >1000 mg/kg p.o., | >1000 mg/kg i.p. |
| H | >400 mg/kg p.o. | 10 mg/kg p.o. | 10 mg/kg p.o. | 1,1 mg/kg p.o. | >1000 mg/kg p.o., | >1000 mg/kg i.p. |

Eine geeignete pharmazeutische Dosierungseinheit kann zwischen etwa 1 bis etwa 500 mg einer Verbindung der Formel I oder eines pharmazeutisch akzeptablen Säureadditionssalzes davon enthalten, wobei ein Dosierungsbereich von etwa 1 mg bis etwa 100 mg für orale Verabreichung und ein Dosierungsbereich von etwa 1 mg bis etwa 50 mg für parenterale Verabreichung bevorzugt ist. In jedem Einzelfall muß jedoch die Person, welche die Verabreichung der fraglichen Verbindungen durchführt oder überwacht, die Dosierung nach ihrem fachlichen Urteil den individuellen Erfordernissen anpassen. Es ist zu beachten, daß die vorstehenden Dosierungen lediglich im Sinn von Beispielen angegeben sind und daß sie den Umfang und die Ausführung der vorliegenden Erfindung in keiner Weise einschränken.

Der Ausdruck »Dosierungseinheit«, wie er in der vorliegenden Beschreibung verwendet wird, bezeichnet einzelne pharmazeutische Einheiten, welche sich als Einheitsdosen für Säuger eignen und von welchen jede eine vorbestimmte Menge des Wirkstoffs enthält, welche so berechnet wurde, daß sie zusammen mit dem erforderlichen pharmazeutischen Verdünnungsmittel, Träger oder Vehikel den erwünschten therapeutischen Effekt zur Folge hat.

Die nachfolgenden Beispiele sollen die Erfindung illustrieren, jedoch nicht einschränken. Alle Temperaturen sind in Celsiusgraden angegeben, sofern nicht anders angegeben.

## Beispiel 1

Eine unter Argon gerührte Suspension von 10 g (0,032 Mol) 8-Chlor-3,4-dihydro-4-[(dimethylamino)methylen]-1-phenyl-5H-2-benzazepin-5-on und 8,5 g (0,047 Mol) Guanidincarbonat in 250 ml Methanol wird bei Raumtemperatur in einer Portion mit 5,1 g (0,094 Mol) Natriummethylat versetzt. Nach 10 Minuten gibt man 150 ml Methylenchlorid hinzu und nach 2 Stunden bzw. 4 Stunden jeweils dieselben Portionen an Natriummethylat und Guanidincarbonat. Nach Rühren über Nacht wird die Mischung mit 250 ml Methylenchlorid verdünnt, mit Wasser gewaschen, über Natriumsulfat getrocknet und im Vakuum eingedampft. Der Rückstand kristallisiert aus Äthanol und ergibt bräunliche Kristalle vom Smp. 209—210° C. Durch Umkristallisieren einer Probe 2-Amino-9-chlor-7-phenyl-5H-pyrimido[5,4-d] [2]benzazepin aus Methylenchlorid/Essigester erhält man weißliche Prismen vom Smp. 210—211°.

## Beispiel 2

Eine unter Argon gerührte Suspension von 16 g (0,047 Mol) 8-Chlor-1-(2-fluorphenyl)-3,4-dihydro-4-[(dimethylamino)methylen]-5H-benzazepin-5-on und 12,5 g (0,07 Mol) Guanidincarbonat in 460 ml Methanol wird bei Raumtemperatur in einer Portion mit 7,5 g (0,14 Mol) Natriummethylat versetzt. Nach 10 Minuten gibt man 290 ml Methylenchlorid hinzu und nach 2 Stunden bzw. 4 Stunden jeweils dieselben Portionen Natriummethylat und Guanidincarbonat. Nach Rühren über Nacht verdünnt man die Mischung mit 460 ml Methylenchlorid, wäscht mit Wasser, trocknet über Natriumsulfat und dampft im Vakuum ein. Der Rückstand kristallisiert aus Äthanol/Methylenchlorid und ergibt 2-Amino-9-chlor-7-(2-fluorphenyl)-5H-pyrimido[5,4-d] [2]benzazepin als weißliche Kristalle vom Smp. 245—248°.

Eine warme Lösung des obigen Produktes in 100 ml Methanol/Methylenchlorid (1 : 1) wird filtriert und auf dem Dampfbad auf das halbe Volumen eingeengt. Das Filtrat wird mit 5 ml einer 5,7 N äthanolischen Salzsäurelösung behandelt und während 2 Stunden bei Raumtemperatur stehengelassen. Das entsprechende Dihydrochlorid wird abfiltriert, mit Äthanol gewaschen und an der Luft getrocknet. Die schwach gelben Kristalle haben einen Smp. von 232—237°.

## Beispiel 3

Eine unter Argon gerührte Suspension von 1,6 g (0,005 Mol) 8-Chlor-3,4-dihydro-4-[(dimethylamino)methylen]-1-phenyl-5H-2-benzazepin-5-on und 0,7 g (0,0075 Mol) Acetamidinhydrochlorid in 50 ml Methanol wird bei Raumtemperatur in einer Portion mit 0,8 g (0,015 Mol) Natriummethylat versetzt. Nach 10 Minuten gibt man 30 ml Methylenchlorid hinzu und nach 2 Stunden bzw. 4 Stunden jeweils dieselben Portionen an Natriummethylat und Acetamidinhydrochlorid. Nach Rühren über Nacht verdünnt man die Mischung mit 50 ml Methylenchlorid, wäscht mit Wasser, trocknet über Natriumsulfat und dampft im Vakuum ein. Kristallisation erfolgt durch Aufnehmen des Rückstandes in 20 ml warmem Hexan und abkühlen lassen. Durch Entfernen des Lösungsmittels erhält man noch eine zweite Portion 9-Chlor-2-methyl-7-phenyl-5H-pyrimido[5,4-d] [2]benzazepin. Durch Umkristallisieren aus Hexan (Aktivkohle) erhält man weißliche Kristalle vom Smp. 120—122°.

Beispiel 4

Eine unter Argon gerührte Suspension von 5,1 g (0,015 Mol) 8-Chlor-1-(2-fluorphenyl)-3,4-dihydro--4-[(dimethylamino)methylen]-5H-2-benzazepin-5-on und 2,1 g (0,0225 Mol) Acetamidinhydrochlorid in 150 ml Methanol wird bei Raumtemperatur in einer Portion mit 2,4 g (0,045 Mol) Natriummethylat versetzt. Nach 10 Minuten gibt man 90 ml Methylenchlorid hinzu und nach 2 Stunden bzw. 4 Stunden jeweils dieselben Portionen an Natriummethylat und Acetamidinhydrochlorid. Nach Rühren über Nacht verdünnt man die Mischung mit 150 ml Methylenchlorid, wäscht mit Wasser, trocknet über Natriumsulfat und dampft im Vakuum ein. Durch Umkristallisieren des Rückstandes aus Hexan (Aktivkohle) erhält man 9-Chlor-7-(2-fluorphenyl)-2-methyl-5H-pyrimido[5,4-d] [2]benzazepin als weiße Kristalle vom Smp. 104—107°.

Beispiel 5

Eine Lösung von 2,0 g (7,19 mMol) [2-(2-Fluorbenzoyl)-4-chlorbenzoesäure] in 40 ml Methanol und 0,3 ml Schwefelsäure wird während 10 Stunden unter Rückfluß zum Sieden erhitzt und anschließend eingedampft. Der Rückstand wird zwischen 50 ml Methylenchlorid und 30 ml verdünnter Ammoniumhydroxidlösung verteilt, die organische Phase abgetrennt, getrocknet und eingedampft. Das so erhaltene Öl wird in 20 ml Methylenchlorid gelöst und über 25 g Florisil filtriert unter Eluieren mit Methylenchlorid. Das nach Eindampfen des Lösungsmittels erhaltene Öl wird aus Äther kristallisiert und aus Methylenchlorid/Äther/Petrol umkristallisiert. Dabei erhält man 2-(2-Fluorbenzoyl)-4-chlorbenzoesäure-methylester als weiße Stäbchen vom Smp. 115—116°.

Beispiel 6

Eine Lösung von 47 g (0,16 Mol) 2-(2-Fluorbenzoyl)-4-chlorbenzoesäure-methylester in 350 ml trockenem Toluol wird mit 21,4 ml (0,18 Mol) Zinntetrachlorid versetzt. Nach 5 Stunden wird unter Rühren eine Lösung von 24 ml (0,308 Mol) 1-Chlor-2,3-epoxypropan in 25 ml Toluol über einen Zeitraum von 30 Minuten zugegeben. Nach 18 Stunden gibt man weitere 12 ml (0,154 Mol) 1-Chlor-2,3-epoxypropan über einen Zeitraum von 15 Minuten hinzu. Nach 4 Stunden wird der Ansatz in einem Eisbad gekühlt und mit konzentrierter Ammoniumhydroxidlösung basisch gestellt. Anschließend filtriert man über Celit unter Nachwaschen mit Toluol. Die vereinigten Filtrate werden mit 200 ml Wasser gewaschen, über Natriumsulfat getrocknet und eingedampft. Das so erhaltene Öl wird in 100 ml Methylenchlorid aufgenommen und an 500 g Alox chromatographiert. Durch Eluieren mit 4 l Methylenchlorid/Petroläther (2 : 1) erhält man ein Öl, dessen Reinheitsgrad (DC) etwa 90 bis 95% beträgt. Eine Probe wird aus Äther/Petrol umkristallisiert und ergibt 4-Chlor-1-[4-chlormethyl-2-(2-fluorphenyl)-1,3-dioxolan-2-yl]benzoesäure-methylester als weiße Prismen vom Smp. 117—122°.

Beispiel 7

Zu 800 ml flüssigem Ammoniak gibt man ein kleines Stück Natrium und einige Kristalle Eisen(II)-nitrat hinzu. Anschließend werden über einen Zeitraum von 30 Minuten 8,7 g (0,378 Mol) Natrium unter Rühren zugegeben und 15 Minuten später eine Lösung von 20,1 ml (0,378 Mol) Acetonitril in 70 ml Äther über einen Zeitraum von 15 Minuten. Nach 10 Minuten wird eine Lösung von 56 g (0,145 Mol) des Endproduktes aus Beispiel 6 in 250 ml Äther über einen Zeitraum von 10 Minuten zugegeben. Nach 2 Stunden gibt man 700 ml Äther hinzu und läßt den Ammoniak über Nacht abdampfen. Nach Zugabe von Eis wird der Ansatz mit Essigsäure sauer gestellt und mit gesättigter Natriumbicarbonatlösung neutralisiert. Die wäßrige Phase wird abgetrennt und mit 500 ml Äther ausgeschüttelt. Die vereinigten ätherischen Phasen werden mit 200 ml halbgesättigter Kochsalzlösung gewaschen, über Natriumsulfat getrocknet und zur Trockene eingedampft. Der Rückstand wird in 150 ml Methylenchlorid aufgenommen und über 400 g Florisil filtiert unter Nachspülen mit 1,5 l Methylenchlorid. Das so erhaltene Öl weist einen Reinheitsgrad (DC) von 90—95% auf.

48 g dieses Produktes werden mit N,N-Dimethylformamiddimethylacetat während 90 Minuten unter Rückfluß zum Sieden erhitzt und anschließend zur Trockene eingedampft. Nach Zerreiben des Rückstandes mit 300 ml Eiswasser wird abdekantiert und das verbleibende Öl in 300 ml Methylenchlorid aufgenommen, mit 200 ml Wasser gewaschen und über Natriumsulfat getrocknet. Durch Eindampfen der Lösung und Kristallisieren des Rückstandes aus Methylenchlorid/Äther erhält man das Endprodukt. Die Mutterlaugen werden eingedampft, in 100 ml Methylenchlorid aufgenommen und über 300 g Florisil filtriert, unter Eluieren mit 400 ml Methylenchlorid und 1,5 l Äther. Aus Methylenchlorid/Äther erhält man dabei kristallines 2-[4-Chlormethyl-2-(2-fluorphenyl)-1,3-dioxolan-2-yl]-$\alpha$-[(dimethylamino)-methylen]-$\beta$-oxo-(4-chlorphenyl)propionitril. Umkristallisieren einer analytischen Probe ergibt weißliche Prismen vom Smp. 143—147°. Die Mutterlaugen enthalten das Endprodukt als Öl, dessen Reinheitsgrad (DC) etwa 85% beträgt.

## Beispiel 8

Eine Lösung von 1,0 g (0,00223 Mol) des Endproduktes aus Beispiel 7 in 8 ml trockenem Dimethylsulfoxid wird mit 4 g Molekularsieb (Typ 5A) und 0,7 g (0,00389 Mol) Guanidincarbonat versetzt. Der Ansatz wird während 5 Stunden unter Rühren bei 90—95° C gehalten, abgekühlt und mit 50 ml Methylenchlorid versetzt. Die Lösung wird vom Festkörper abdekantiert, unter mehrmaligem Nachwaschen mit Methylenchlorid und Wasser. Nach Abtrennen der wäßrigen Phase wird die organische Phase 2mal mit verdünnter Kochsalzlösung gewaschen, über Natriumsulfat getrocknet und eingedampft. Der Rückstand wird an 4 Silicagel-Dickschichtplatten chromatographiert (Methylenchlorid/Essigester, 3 : 1, rf 0,4). 2-Amino-4-[2-[4-(chlormethyl)-2-(2-fluorphenyl)-1,3-dioxolan-2-yl]-4-chlorphenyl]pyrimidin-5-carbonitril kristallisiert aus Methanol und ergibt weißliche Prismen vom Smp. 184—191°.

## Beispiel 9

Eine Lösung von 0,2 g (0,448 mMol) des Endproduktes aus Beispiel 8 in 20 ml Methanol und 10 ml 3 N Salzsäure wird während 20 Minuten unter Rückfluß zum Sieden erhitzt und anschließend eingedampft. Nach Verteilen des Rückstandes zwischen 50 ml Methylenchlorid und 30 ml verdünnter Ammoniumhydroxidlösung, wird die organische Phase getrocknet, eingeengt und über 15 g Florisil, unter Eluieren mit 200 ml Äther, filtriert. Das so erhaltene 2-Amino-4-[2-(2-fluorbenzoyl)-4-chlorphenyl]-pyrimidin-5-carbonitril wird aus Methylenchlorid/Äther/Petrol umkristallisiert und ergibt weiße Prismen vom Smp. 151—157°.

## Beispiel 10

Eine Lösung von 50 ml (0,142 mMol) des Endproduktes aus Beispiel 9 in 10 ml Essigsäure wird mit wenig Raneynickel versetzt und während 2,5 Stunden hydriert. Nach Filtrieren über Celit und eindampfen, wird der Rückstand zwischen 30 ml Methylenchlorid und 15 ml verdünnter Ammoniumhydroxidlösung verteilt. Die organische Phase wird abgetrennt, über Natriumsulfat getrocknet und eingedampft. Anschließend wird der Rückstand in Äthanol aufgenommen, während 1 Stunde unter Rückfluß zum Sieden erhitzt und zur Trockene eingedampft. Der so erhaltene Festkörper wird an einer Silicagel-Dickschichtplatte chromatographiert (Essigester/Äthanol, 20 : 1, rf 0,3). 2-Amino-9-chlor-7-(2-fluorphenyl)-5H-pyrimido[5,4-d] [2]benzazepin kristallisiert aus Äther und weist einen Smp. von 243—248° auf, der Mischschmelzpunkt mit authentischem Material aus Beispiel 2 beträgt 244—248°.

## Beispiel 11

Eine Lösung von 33 g (0,138 Mol) 2-Benzoyl-benzoesäure-methylester in 200 ml trockenem Tetrachlorkohlenstoff wird mit 10,1 ml (0,13 Mol) 1-Chlor-3,4-epoxypropan versetzt. Nach Abkühlen in einem Eisbad gibt man eine Lösung von 1,5 ml (0,013 Mol) Zinntetrachlorid in 10 ml Tetrachlorkohlenstoff unter Rühren über einen Zeitraum von 20 Minuten hinzu. Nach Stehen übers Wochenende gibt man jeweils dieselben Portionen an 1-Chlor-2,3-epoxypropan und Zinntetrachlorid hinzu. Nach weiteren 18 Stunden wird die Reaktionsmischung in einem Eisbad abgekühlt und mit konzentrierter Ammoniumhydroxidlösung neutralisiert. Der Niederschlag wird unter Nachwaschen mit Methylenchlorid abfiltriert, die vereinigten Filtrate werden mit 150 ml Wasser gewaschen, über Natriumsulfat getrocknet und eingedampft. Das entstandene Öl wird in 100 ml Methylenchlorid aufgenommen und an 500 g neutralem Alox chromatogrpahiert. Durch Eluieren mit 3 l Methylenchlorid erhält man 2-[4-(Chlormethyl)-2-phenyl-1,3-dioxolan-2-yl]benzoesäure-methylester als Öl, dessen Reinheitsgrad (DC) etwa 95% beträgt. Durch Kristallisieren und Umkristallisieren einer Probe aus Äther/Petroläther resultieren weiße Stäbchen vom Smp. 90—91°.

## Beispiel 12

Zu 75 ml flüssigem Ammoniak gibt man unter Rühren ein kleines Stück Natrium und einige Kristalle Eisen(II)-nitrat hinzu. Über einen Zeitraum von 20 Minuten versetzt man die Lösung mit 1,15 g (0,0502 Mol) Natrium und 5 Minuten später tropfenweise mit einer Lösung von 2,9 ml (0,050 Mol) Acetonitril in 10 ml Äther. Anschließend tropft man eine Lösung von 6,6 g (0,0198 Mol) des Endproduktes aus Beispiel 12 in 40 ml Äther zu. Nach 2 Stunden versetzt man mit 100 ml Äther, läßt den Ammoniak abdampfen, gibt 100 g Eis hinzu, säuert mit Essigsäure an und neutralisiert mit gesättigter Natriumbicarbonatlösung. Die organische Phase wird abgetrennt, und die wäßrige Phase mit Äther ausgeschüttelt. Die vereinigten organischen Auszüge werden mit 100 ml Wasser gewaschen, über Natriumsulfat getrocknet und eingedampft. Das verbleibende Öl nimmt man in 15 ml Methylenchlorid auf und

filtriert, unter Nachspülen mit Methylenchlorid, über 100 g Florisil. Das rohe 2-[4-(Chlormethyl)-2-phenyl-1,3-dioxolan-2-yl]-β-oxo-benzolpropionitril wird ohne weitere Reinigung im nächsten Schritt eingesetzt.

Eine Lösung von 20 g (0,0585 Mol) des obigen Produktes in 75 ml N,N-Dimethylformamid-dimethylacetal wird während 90 Minuten unter Rückfluß zum Sieden erhitzt und anschließend zur Trockene eingedampft. Nach Zerreiben des öligen Rückstandes mit Eiswasser wird abdekantiert und der Rückstand zwischen 150 ml Methylenchlorid und 150 ml Wasser verteilt. Die organische Phase wird über Natriumsulfat getrocknet, eingeengt und über 150 g Florisil, unter Eluieren mit Äther, filtriert. Das so erhaltene rohe 2-[4-(Chlormethyl)-2-phenyl-1,3-dioxolan-2-yl]-α-[(dimethylamino)-methylen]-β-oxo-benzolpropionitril kristallisiert aus Äthanol. Eine Probe wird aus Methylenchlorid/Äther umkristallisiert und ergibt weiße Prismen vom Smp. 107—110°.

## Beispiel 13

Eine Lösung von 2,0 g (0,00504 Mol) des Endproduktes aus Beispiel 12 in 10 ml Methylenchlorid wird mit 10 ml Methanol und 2 ml (0,0192 Mol) einer 9,6 N äthanolischen Salzsäure-Lösung versetzt. Nach 90 Minuten entfernt man das Lösungsmittel und verteilt den Rückstand zwischen 50 ml Methylenchlorid und 30 ml einer gesättigten Natriumbicarbonat-Lösung. Nach Abtrennen und Trocknen der organischen Phase über Natriumsulfat wird eingedampft. Der ölige Rückstand (1,5 g) wird in 30 ml Methanol aufgenommen und mit 1,5 g (0,00833 Mol) Guanidincarbonat versetzt. Die Lösung wird während 90 Minuten gerührt und anschließend noch 2 Stunden unter Rückfluß zum Sieden erhitzt. Nach Eindampfen und Verteilen des Rückstandes zwischen 50 ml Methylenchlorid und 30 ml verdünnter Ammoniumhydroxid-Lösung wird die organische Phase über Natriumsulfat getrocknet und über 50 g Florisil, unter Eluieren mit 200 ml Methylenchlorid und 300 ml Äther, filtriert. Aus der Methylenchlorid-Fraktion erhält man nach Umkristallisieren aus Methylenchlorid/Petroläther 1-Oxo-3-phenyl-1H-inden-2-carbonitril als gelbe Stäbchen vom Smp. 173—175°.

Aus der Äther-Fraktion erhält man durch Kristallisieren aus Methylenchlorid/Petroläther 2-Amino-4-(2-benzoylphenyl)pyrimidin-5-carbonitril vom Smp. 195—199°. Eine analytische Probe wird aus Methanol umkristallisiert und ergibt weißliche Prismen vom Smp. 197—200°.

## Beispiel 14

Eine Lösung von 3,1 g (0,0103 Mol) 2-Amino-4-(2-benzoylphenyl)pyrimidin-5-carbonitril in 60 ml Eisessig wird mit einem Teelöffel Raneynickel versetzt und während 8,5 Stunden hydriert, anschließend filtriert man über Celit und entfernt das Lösungsmittel. Nach Verteilen des Rückstandes zwischen 50 ml Methylenchlorid und 30 ml verdünnter Ammoniumhydroxid-Lösung wird die organische Phase über Natriumsulfat getrocknet und eingedampft. Das erhaltene Öl wird in 75 ml Methanol aufgenommen, während 15 Minuten unter Rückfluß zum Sieden erhitzt, eingedampft und in 100 ml Methylenchlorid gelöst. Anschließend gibt man unter Rühren 3 g aktiviertes Mangandioxid hinzu und erhitzt während 30 Minuten unter Rückfluß. Nach Filtrieren und Eindampfen wird der erhaltene Rückstand an 100 g Florisil chromatographiert. Zuerst eluiert man mit 300 ml Methylenchlorid anschließend mit 500 ml Äther und 1,5 l Essigester. Aus der Essigester-Fraktion erhält man durch Umkristallisieren aus Methylenchlorid/Äther weiße Stäbchen vom Smp. 239—242°. Die Mutterlaugen und die Äther-Fraktion ergeben nach Eindampfen und Chromatographieren an Silicagel-Dickschichtplatten mit Essigester/Methanol (20 : 1) als Fließmittel eine weitere Portion 7-Phenyl-5H-pyrimido[5,4-d] [2]benzazepin-2-amin. Durch Umkristallisieren einer analytischen Probe aus Äther erhält man weiße Prismen vom Smp. 201—205°, die sich nach dem Abkühlen zu Stäbchen vom Smp. 240—243° umordnen.

## Beispiel 15

In 5 gleichen Portionen gibt man 5,5 g (58 mMol) Acetamidinhydrochlorid und 15 ml (62 mMol) einer 4,12 M methanolischen Lösung von Natriummethylat, über einen Zeitraum von 3 Stunden, zu einer Lösung von 3,5 g (10 mMol) 8-Chlor-1-(2-chlorphenyl)-3,4-dihydro-4-[(dimethylamino)methylen]-5H-2-benzazepin-5-on in 140 ml Methanol und 140 ml Methylenchlorid. Die Mischung wird mit Wasser verdünnt und mit Methylenchlorid ausgeschüttelt. Nach Waschen der Methylenchlorid-Lösung mit Wasser, Trocknen über Natriumsulfat und Eindampfen im Vakuum erhält man ein gelbbraunes Öl. Durch Aufnehmen des Öles in 10 ml (10 mMol) einer 1 M Methansulfonsäure-Lösung in Methanol und Ausfällen des Salzes durch Zugabe von Äther bekommt man 9-Chlor-7-(2-chlorphenyl)-2-methyl-5H-pyrimido[5,4-d] [2]benzazepin als gelbe Prismen vom Smp. 193—197°. Nach Umkristallisieren auf Methanol/Äther rsultieren gelbe Prismen vom Smp. 197—198°.

## Beispiel 16

In 5 gleichen Portionen gibt man 21 g (200 mMol) Formamidinacetat und 32,5 ml (135 mMol) einer 4,12 M methanolischen Lösung von Natriummethylat, über einen Zeitraum von 3 Stunden, zu einer Lösung von 7,2 g (20 mMol) 8-Chlor-1-(2-chlorphenyl)-3,4-dihydro-4-[(dimethylamino)methylen]-5H-2-benzazepin-5-on in 270 ml Methanol und 270 ml Methylenchlorid. Nach Verdünnen der Lösung mit Wasser und Extrahieren mit Methylenchlorid wird die organische Phase mit Wasser gewaschen, über Natriumsulfat getrocknet und im Vakuum eingedampft, dabei erhält man ein gelbbraunes Öl. Die Reinigung von rohem 9-Chlor-7-(2-chlorphenyl)-5H-pyrimido[5,4-d] [2]benzazepin erfolgt durch Säulenchromatographie an 100 g Silicagel unter Eluieren mit Methylenchlorid/Essigester (1 : 1) und ergibt Kristalle vom Smp. 122—124°. Durch Umkristallisieren aus Äther erhält man blaßgelbe Prismen vom Smp. 122—125°.

## Beispiel 17

Eine Mischung aus 3,5 g (10 mMol) 1-(2-Chlorphenyl)-3,4-dihydro-4-[(dimethylamino)methylen]-5H--2-benzazepin-5-on, 4,8 g (40 mMol) Isobutyramidin-hydrochlorid, 10 ml (41 mMol) einer 4,12 M methanolischen Lösung von Natriummethylat und 100 ml Methanol wird während 2 Stunden bei Raumtemperatur gerührt. Die Mischung wird mit Wasser verdünnt und mit Methylenchlorid extrahiert. Die organische Phase wird über Natriumsulfat getrocknet und im Vakuum eingedampft, dabei erhält man ein gelbes Öl. Kristallisation aus Äther ergibt 9-Chlor-7-(2-chlorphenyl)-2-isopropyl-5H-pyrimido[5,4-d] [2]benzazepin als gelber Festkörper vom Smp. 127—129°. Durch Umkristallisieren aus Äther/Petroläther erhält man farblose Stäbchen vom Smp. 127—129°.

## Beispiel 18

In 2 gleichen Portionen gibt man 14,4 g (80 mMol) Guanidincarbonat und 20 ml (82 mMol) einer 4,12 M methanolischen Natriummethylat-Lösung, über einen Zeitraum von 90 Minuten, zu einer Lösung von 3,6 g (10 mMol) 8-Chlor-1-(2-chlorphenyl)-3,4-dihydro-4-[(dimethylamino)methylen]-5H-2-benzazepin-5-on in 100 ml Methanol. Nach Verdünnen der Mischung mit Wasser und Ausschütteln mit Methylenchlorid wird die organische Phase über Natriumsulfat getrocknet und im Vakuum eingedampft. Das so erhaltene gelbe Öl kristallisiert aus Methylenchlorid und ergibt einen weißen Festkörper vom Smp. 240—241°. Durch Umkristallisieren aus Äther/Methylenchlorid erhält man 2-Amino-9-Chlor-7-(2-chlorphenyl)-5H-pyrimido[5,4-d] [2]benzazepin als farblose Nadeln vom Smp. 240—241°.

## Beispiel 19

In 4 gleichen Portionen gibt man 7,2 g (76 mMol) Acetamidin-hydrochlorid und 18 ml (80 mMol) einer 4,46 M methanolischen Natriummethylat-Lösung, über einen Zeitraum von 3 Stunden, zu einer Lösung von 4,5 g (14 mMol) 1-(2-Chlorphenyl)-3,4-dihydro-4-[(dimethylamino)methylen]-5H-2-benzazepin-5-on in 180 ml Methanol und 180 ml Methylenchlorid. Nach Verdünnen der Mischung mit Wasser und Ausschütteln mit Methylenchlorid wäscht man die organische Phase mit Wasser, trocknet über Natriumsulfat und dampft im Vakuum ein. Nach Aufnehmen des gelbbraunen Öles in einer Mischung von 15 ml Isopropanol und 1,3 g (14 mMol) Methansulfonsäure dampft man im Vakuum ein. Der Rückstand wird aus Äther/Methylenchlorid kristallisiert, dabei erhält man 7-(2-Chlorphenyl)-2-methyl-5H-pyrimido[5,4-d] [2]benzazepin-methansulfonat als gelben Festkörper vom Smp. 147—151°. Durch Umkristallisieren aus Äther/Methylenchlorid bekommt man das entsprechende Halbhydrat als gelbe Prismen vom Smp. 159—160°.

## Beispiel 20

In 5 gleichen Portionen gibt man 9,0 g (95 mMol) Acetamidin-hydrochlorid und 22,5 ml (0,1 Mol) einer 4,46 M methanolischen Natriummethylat-Lösung, über einen Zeitraum von 3 Stunden, zu einer Lösung von 4,5 g (15 mMol) 1-Phenyl-3,4-dihydro-4-[(dimethylamino)methylen]-5H-2-benzazepin-5-on in 180 ml Methanol und 180 ml Methylenchlorid. Die Methylenchloridphase wird mit Wasser gewaschen, über Natriumsulfat getrocknet und im Vakuum eingedampft. Nach Lösen des erhaltenen Öls in überschüssiger 6proz. methanolischer Salzsäure-Lösung dampft man im Vakuum zur Trockene ein. Durch Kristallisieren des Rückstandes aus Äther/Methylenchlorid erhält man 2-Methyl-7-phenyl-5H-pyrimido[5,4-d] [2]benzazepin-dihydrochlorid als weißen Festkörper vom Smp. 211—221°. Beim Umkristallisieren aus Methanol/Äther erhält man weiße Plättchen vom Smp. 217—227°.

Beispiel 21

Eine Lösung von 2,0 g (5,6 mMol) 9-Chlor-7-(2-chlorphenyl)-2-methyl-5H-pyrimido[5,4-d] [2]benzazepin und 2,2 g (10,8 mMol) m-Chlor-perbenzoesäure (85%) in 100 ml Methylenchlorid wird während 21 Stunden bei Raumtemperatur gerührt. Nach Waschen mit kalter verdünnter Natriumhydroxid-Lösung wird die organische Phase über Natriumsulfat getrocknet und im Vakuum zur Trockene eingedampft. Die Reinigung von 9-Chlor-7-(2-chlorphenyl)-2-methyl-5H-pyrimido[5,4-d] [2]benzazepin-6-oxid erfolgt durch Filtration über 25 g Silicagel unter Eluieren mit 1000 ml Äther/Methylenchlorid (1 : 1), dabei erhält man einen farblosen Festkörper vom Smp. 215—216°.

Beispiel 22

Eine Lösung von 3,8 g (10,7 mMol) 9-Chlor-7-(2-chlorphenyl)-2-methyl-5H-pyrimido[5,4-d] [2]benzazepin und 9,6 g (47 mMol) m-Chlor-perbenzoesäure (85%) in 400 ml Methylenchlorid wird während 55 Stunden bei Raumtemperatur gerührt. Nach Waschen der organischen Phase mit kalter verdünnter Natriumhydroxid-Lösung wird über Natriumsulfat getrocknet und im Vakuum zur Trockene eingedampft. Die Reinigung von 9-Chlor-7-(2-chlorphenyl)-2-methyl-5H-pyrimido[5,4-d] [2]benzazepin-3,6-dioxid erfolgt durch Filtration über 25 g Silicagel unter Eluieren mit 400 ml Äther/Methylenchlorid (1 : 1) und 200 ml Methylenchlorid/Methanol (9 : 1), dabei erhält man einen farblosen Festkörper vom Smp. 241—243°.

Beispiel 23

Eine Mischung aus 3,7 g (10,5 mMol) 9-Chlor-7-(2-chlorphenyl)-2-methyl-5H-pyrimido[5,4-d] [2]benzazepin, 1,3 g Zinkstaub, 40 ml Essigsäure und 90 ml Methylenchlorid wird während 30 Minuten zwischen −15° und −20° gerührt. Nach Filtrieren über Hyflo wird die Mischung mit kalter verdünnter Natronlauge basisch gestellt und mit Methylenchlorid extrahiert. Die organische Phase wird über Natriumsulfat getrocknet und im Vakuum eingedampft, dabei erhält man ein gelbes Öl. Dieses Öl kristallisiert aus überschüssiger 6proz. methanolischer Salzsäure-Lösung und ergibt 9-Chlor-7-(2-chlorphenyl)-6,7-dihydro-2-methyl-5H-pyrimido[5,4-d] [2]benzazepin-dihydrochlorid als weißen Festkörper vom Smp. 272—274°. Durch Umkristallisieren aus Methanol erhält man farblose Stäbchen vom Smp. 272—274°.

Eine Probe des obigen Materials wird zwischen verdünnter Natronlauge und Methylenchlorid verteilt. Die organische Phase wird über Natriumsulfat getrocknet und im Vakuum zur Trockene eingedampft. Der Rückstand kristallisiert aus Äther und ergibt die freie Base als cremefarbene Prismen vom Smp. 176—177°.

Wird die Reaktion in Essigsäure mit einem vorhydrierten Platinoxid-Katalysator durchgeführt, so werden 2 äquivalente Wasserstoff aufgenommen, und man erhält nach dem Aufarbeiten die in Beispiel 39 beschriebene Tetrahydroverbindung. Solche Tetrahydroverbindungen entfalten ebenfalls nützliche Wirkungen auf das zentrale Nervensystem.

Beispiel 24

Eine Mischung aus 18,6 g (61 mMol) 8-Chlor-3,4-dihydro-1-(2-chlorphenyl)-5H-2-benzazepin-5-on und 149 ml Dimethylformamid-dimethylacetal wird während 12 Stunden auf ca. 50° erwärmt. Nach Entfernen des Lösungsmittels im Vakuum wird der Rückstand aus Äther/Methylenchlorid kristallisiert und ergibt 8-Chlor-1-(2-chlorphenyl)-3,4-dihydro-4-[(dimethylamino)-methylen]-5H-2-benzazepin-5-on als gelben Festkörper vom Smp. 170—171°. Durch Umkristallisieren aus Äther erhält man gelbe Prismen vom Smp. 170—171°.

Beispiel 25

Eine Mischung aus 3,4 g (12,5 mMol) 1-(2-Chlorphenyl)-3,4-dihydro-5H-2-benzazepin-5-on und 28 ml Dimethylformamid-dimethylacetal wird während 2 Stunden unter Rückfluß zum Sieden erhitzt. Nach Einengen der Mischung im Vakuum wird der erhaltene Festkörper mit Äther verrieben und ergibt einen braunen Festkörper vom Smp. 155—157°. Durch Umkristallisieren aus Methylenchlorid/Äther erhält man 1-(2-Chlorphenyl)-3,4-dihydro-4-[(dimethylamino)methylen]-5H-2-benzazepin-5-on als gelbe Prismen vom Smp. 158—159°.

Beispiel 26

Eine Mischung aus 5,2 g (22 mMol) 3,4-Dihydro-1-phenyl-5H-2-benzazepin-5-on und 43 ml Dimethylformamiddimethylacetal wird während 4 Stunden unter Rückfluß erhitzt. Die Mischung wird im Vakuum zur Trockene eingedampft. Nach Kristallisieren des Rückstandes aus Äther erhält man 3,4-Dihydro--1-phenyl-4-[(dimethylamino)methylen]-5H-2-benzazepin-5-on als gelben Festkörper vom Smp. 131—133°. Durch Umkristallisieren aus Äther erhält man gelbe Prismen vom Smp. 131—132°.

Beispiel 27

Eine Mischung aus 1,1 g (3,0 mMol) 9-Chlor-7-(2-chlorphenyl)-5H-pyrimido[5,4-d] [2]benzazepin-2-thiol, 1,0 ml (10 mMol) Dimethylsulfat, 20 ml 1 N Natronlauge und 10 ml Äthanol wird während 15 Minuten bei Raumtemperatur gerührt. Die Mischung wird mit Wasser verdünnt und mit Methylenchlorid ausgeschüttelt. Nach Trocknen der organischen Phase über Natriumsulfat und Eindampfen im Vakuum erhält man ein Öl, das aus Äther 9-Chlor-7-(2-chlorphenyl)-2-(methylhio)-5H-pyrimido[5,4-d] [2]benzazepin als farblose Prismen vom Smp. 187—188° ergibt.

Beispiel 28

Durch Lösen äquivalenter Mengen des Produktes aus Beispiel 27 und Methansulfonsäure in Methanol und anschließendem Ausfällen des Salzes durch Zugabe von Äther erhält man 9-Chlor-7-(2-chlorphenyl)-2-(methylthio)-5H-pyrimido[5,4-d] [2]benzazepin-methansulfonat. Nach Umkristallisieren aus Äthanol resultieren cremefarbene Nadeln vom Smp. 165—166°.

Beispiel 29

Eine Mischung aus 2,8 g (7,8 mMol) 8-Chlor-1-(2-chlorphenyl)-3,4-dihydro-4-[(dimethylamino)methylen]-5H-2-benzazepin-5-on, 2,8 g (37 mMol) Thioharnstoff, 8,0 ml (32 mMol) einer 4,0 M methanolischen Natriummethylat-Lösung und 80 ml Methanol wird während 18 Stunden bei Raumtemperatur gerührt. Nach Verdünnen der Mischung mit Wasser und Extrahieren mit Äther wird die wäßrige Phase mit Essigsäure neutralisiert und mit Methylenchlorid ausgeschüttelt. Die Methylenchloridphase wird über Natriumsulfat getrocknet und im Vakuum eingedampft, wobei ein gelber Festkörper vom Smp. 238—239° anfällt. Durch Umkristallisieren aus Tetrahydrofuran erhält man 9-Chlor-7-(2-chlorphenyl)-5H-pyrimido[5,4-d] [2]benzazepin-2-thiol als gelbe Kristalle vom Smp. 232—234°.

Beispiel 30

Eine Lösung von 1,2 g (0,00354 Mol) 9-Chlor-7-(2-fluorphenyl)-2-amino-5H-pyrimido[5,4-d] [2]benzazepin in 20 ml konzentrierter Schwefelsäure und 20 ml Wasser wird während 12 Stunden unter Rückfluß zum Sieden erhitzt und anschließend abgekühlt. Nach Zugabe von Eis wird der Ansatz mit Ammoniumhydroxid-Lösung basisch gestellt und mit 100 ml Methylenchlorid extrahiert. Die ausgefallenen Festkörper werden abfiltriert und aus Methylenchlorid/Methanol umkristallisiert, dabei erhält man 9-Chlor-7-(2-fluorphenyl)-5H-pyrimido[5,4-d] [2]benzazepin-2-ol als weiße Prismen vom Smp. 297—299° (Zers.). Nach Trocknen und Eindampfen des Methylenchloridextraktes wird der Rückstand aus Methylenchlorid/Methanol kristallisiert, wobei man eine zusätzliche Portion Produkt erhält.

Beispiel 31

Eine Lösung von 1,0 g (0,00294 Mol) 9-Chlor-7-(2-fluorphenyl)-5H-pyrimido[5,4-d] [2]benzazepin-2-ol in 5 ml Phosphoroxychlorid wird während 4 Stunden auf dem Dampfbad erwärmt und anschließend zur Trockene eingedampft. Der Festkörper wird aus Methylenchlorid/Äther kristallisiert und zwischen 50 ml Methylenchlorid und 40 ml gesättigter Natriumbicarbonat-Lösung verteilt. Nach Trocknen und Eindampfen der organischen Phase wird der Rückstand aus Äther kristallisiert. Nach Umkristallisieren aus Methylenchlorid/Äther erhält man 2,9-Dichlor-7-(2-fluorphenyl)-5H-pyrimido[5,4-d] [2]benzazepin als weißliche Prismen vom Smp. 157—160°.

Beispiel 32

Eine Lösung von 1 g (0,00279 Mol) 2,9-Dichlor-7-(2-fluorphenyl)-5H-pyrimido[5,4-d] [2]benzazepin in 4 ml N,N-Dimethylformamid wird in einem Eisbad gekühlt und mit Methylamin gesättigt. Nach 64 Stunden bei Raumtemperatur versetzt man mit 50 ml Eiswasser und filtriert den Niederschlag ab. Der Niederschlag wird zwischen 50 ml Methylenchlorid und 50 ml Wasser verteilt. Die organische Phase wird mit 25 ml halbgesättigter Kochsalzlösung gewaschen, getrocknet und zur Trockene eingedampft. Nach Kristallisieren des Öles aus Äther wird zuerst aus Methylenchlorid/Äther und dann Methanol umkristallisiert. Dabei erhält man 9-Chlor-N-methyl-7-(2-fluorphenyl)-5H-pyrimido[5,4-d] [2]benzazepin-2-amin als weiße Prismen vom Smp. 172—179°.

Beispiel 33

Eine Lösung von 4,0 g (0,0112 Mol) 2,9-Dichlor-7-(2-fluorphenyl)-5H-pyrimido[5,4-d] [2]benzazepin in 15 ml N,N-Dimethylformamid wird in einem Eisbad gekühlt und mit Dimethylamin gesättigt. Nach 18 Stunden bei Raumtemperatur versetzt man mit 80 ml Eiswasser und filtriert den Niederschlag ab. Durch 2maliges Umkristallisieren aus Methanol erhält man 9-Chlor-N,N-dimethyl-7-(2-fluorphenyl)-5H-pyrimido[5,4-d] [2]benzazepin-2-amin als weiße Stäbchen vom Smp. 175—179°.

Beispiel 34

Zu 3,5 g (0,00978 Mol) 2,9-Dichlor-7-(2-fluorphenyl)-5H-pyrimido[5,4-d] [2]benzazepin in 7 ml N,N-Dimethylformamid gibt man unter Eiskühlung 2,7 ml (0,0215 Mol) 3-Dimethylamino-propylamin hinzu. Nach 66 Stunden bei Raumtemperatur versetzt man mit 50 ml Eiswasser und filtriert ab. Der Niederschlag wird in 50 ml Methylenchlorid aufgenommen, mit 40 ml Wasser gewaschen und eingedampft. Nach Aufnehmen des Öles in verdünnter Salzsäure wird die Lösung mit Ammoniumhydroxid auf pH 2 eingestellt. Nach Extrahieren der sauren Lösung mit 2mal je 50 ml Methylenchlorid wird die wäßrige Phase mit Ammoniumhydroxid basisch gestellt und mit 75 ml Methylenchlorid ausgeschüttelt. Nach Trocknen und Eindampfen der Methylenchloridphase wird der Rückstand aus Äther/Petroläther kristallisiert. Durch Umkristallisieren aus demselben Lösungsmittelgemisch erhält man 9-Chlor-7-(2-fluorphenyl)-N-(3-N,N-dimethylaminopropyl)-5H-pyrimido[5,4-d] [2]benzazepin-2-amin als weiße Nadeln vom Smp. 90—101°.

Beispiel 35

Zu 1,0 g (0,00279 Mol) 2,9-Dichlor-7-(2-fluorphenyl)-5H-pyrimido[5,4-d] [2]benzazepin in 25 ml Methanol gibt man 0,18 g (0,00335 Mol) Natriummethylat und rührt während 18 Stunden. Nach Eindampfen wird der feste Rückstand in 50 ml Methylenchlorid aufgenommen, mit 40 ml Wasser gewaschen, getrocknet und im Vakuum zur Trockene eingedampft. Nach Umkristallisieren des Rückstandes aus Äther/Petroläther und Äther erhält man 9-Chlor-2-methoxy-7-(2-fluorphenyl)-5H-pyrimido[5,4-d] [2]benzazepin als weiße Prismen vom Smp. 137—141°.

Beispiel 36

Eine Mischung aus 34,2 g (0,1 Mol) 8-Chlor-3,4-dihydro-1-(2-fluorphenyl)-4-[(dimethylamino)methylen]-5H-2-benzazepin-5-on, 62,4 g (0,6 Mol) Formamidinacetat, 35 g (0,63 Mol) Natriummethylat und 700 ml Methanol wird während 3 Stunden bei Raumtemperatur gerührt, unter gleichzeitigem Einleiten von Stickstoff. Nach Verdünnen der Mischung mit Wasser und Extrahieren mit Methylenchlorid wird die organische Phase über Natriumsulfat getrocknet und im Vakuum eingedampft. Das so erhaltene rote Öl wird in siedendem Hexan suspendiert. Nach Abdekantieren der noch heißen Hexan-Lösung läßt man abkühlen, wobei 9-Chlor-7-(2-fluorphenyl)-5H-pyrimido[5,4-d] [2]benzazepin ausfällt und abfiltriert wird. Durch Umkristallisieren aus Cyclohexan erhält man weißliche Kristalle vom Smp. 123—125°.

Beispiel 37

Eine Lösung von 3,2 g (10 mMol) 9-Chlor-7-(2-fluorphenyl)-5H-pyrimido[5,4-d] [2]benzazepin, 3 g (15 mMol) m-Chlorperbenzoesäure (85%) in 100 ml Methylenchlorid wird während 4 Stunden bei Raumtemperatur gerührt. Die Reaktionsmischung wird mit einem Überschuß kalter, verdünnter Natronlauge gewaschen, über Natriumsulfat getrocknet und über Hyflo filtriert. Das Filtrat wird im

Vakuum zur Trockene eingedampft. Durch Kristallisieren des Rückstandes aus Methylenchlorid/Äther erhält man rohes 9-Chlor-7-(2-fluorphenyl)-5H-pyrimido[5,4-d] [2]benzazepin-6-oxid vom Smp. 185—186°. Nach Umkristallisieren aus Methylenchlorid/Äther erhält man das reine Produkt vom Smp. 187—188°.

Beispiel 38

Eine Mischung aus 2,5 g (7,4 mMol) 9-Chlor-7-(2-fluorphenyl)-5H-pyrimido[5,4-d] [2]benzazepin-6-oxid und 50 ml Essigsäureanhydrid wird auf dem Dampfbad während 24 Stunden erwärmt. Nach Eindampfen des Ansatzes und Kristallisieren des Rückstandes aus Methylenchlorid/Äther erhält man das Rohprodukt vom Smp. 197—198°. Durch Umkristallisieren aus Methylenchlorid/Äther erhält man das reine 9-Chlor-7-(2-fluorphenyl)-5H-pyrimido[5,4-d] [2]benzazepin-5-ol-acetat als cremefarbene Prismen vom Smp. 200—201°.

Beispiel 39

Eine Lösung von 3,8 g (10,7 mMol) 9-Chlor-7-(2-chlorphenyl)-2-methyl-5H-pyrimido[5,4-d] [2]benzazepin in 50 ml Essigsäure wird bei Raumtemperatur und Normaldruck hydriert, wobei 0,5 g vorhydriertes Platinoxid als Katalysator verwendet werden. Nach 3 Stunden und einer Aufnahme von etwa 500 ml Wasserstoff (ca. 2 Äquivalente) wird vom Katalysator abfiltriert. Das Filtrat wird im Vakuum zur Trockene eingedampft und der Rückstand in Methylenchlorid aufgenommen, mit einem Überschuß verdünnter, kalter Natronlauge gewaschen und über Natriumsulfat getrocknet. Die Methylenchlorid-Phase wird mit überschüssiger methanolischer Salzsäure-Lösung verdünnt und im Vakuum eingedampft. Der Rückstand wird mit Isopropanol verrieben und abfiltriert. Durch Umkristallisieren aus Methanol erhält man reines 9-Chlor-7-(2-chlorphenyl)-4,5,6,7-tetrahydro-2-methyl-1H-pyrimido[5,4-d] [2]benzazepin-hydrochlorid vom Smp. 275—276°.

Beispiel 40

Über einen Zeitraum von 2 Stunden gibt man 28 g (150 mMol) Guanidincarbonat und 38 ml (150 mMol) einer 4,09 M methanolischen Natriummethylat-Lösung in 2 gleichen Portionen zu einer Lösung von 7,0 g (20 mMol) 8-Chlor-1-(2-fluorphenyl)-3,4-dihydro-4-[(dimethylamino)methylen]-5H-benzazepin-5-on-2-oxid in 210 ml Methanol. Nach Verdünnen der Mischung mit Wasser und Extrahieren mit Methylenchlorid wird die organische Phase über Natriumsulfat getrocknet und im Vakuum eingedampft, wobei ein roter Festkörper anfällt. Durch Umkristallisieren aus Methanol/Essigester erhält man 2-Amino-9-Chlor-7-(2-fluorphenyl)-5H-pyrimido[5,4-d] [2]benzazepin-6-oxid als feine, gelbe Nadeln vom Smp. 320—323°.

Beispiel 41

Eine Mischung aus 4 g (11 mMol) 9-Chlor-7-(2-chlorphenyl)-6,7-dihydro-2-methyl-5H-pyrimido[5,4-d] [2]benzazepin, 2 ml 88proz. Ameisensäure und 2 ml 37,5proz. Formaldehyd-Lösung wird auf dem Dampfbad während 3 Stunden erwärmt. Das Reaktionsgemisch wird auf einen Überschuß verdünnter, eiskalter Natronlauge gegossen und mit Methylenchlorid extrahiert. Die organische Phase wird über Natriumsulfat getrocknet und im Vakuum zur Trockene eingedampft. Der Rückstand kristallisiert aus Äther und ergibt das Rohprodukt vom Smp. 155—156°. Durch Umkristallisieren aus Äther erhält man reines 9-Chlor-7-(2-chlorphenyl)-6,7-dihydro-2,6-dimethyl-5H-pyrimido[5,4-d] [2]benzazepin als farblose Prismen vom Smp. 156—157°.

Beispiel 42

Methode A

Eine Mischung aus 7,2 g (25 mMol) 8-Chlor-1-(2-fluorphenyl)-3,4-dihydro-5H-2-benzazepin-5-on und 50 ml Dimethylformamid-dimethylacetal wird während 1 Stunde unter Rückfluß zum Sieden erhitzt. Durch Eindampfen der Mischung im Vakuum erhält man bräunliche Kristalle. Durch Umkristallisieren aus Äther erhält man 8-Chlor-1-(2-fluorphenyl)-3,4-dihydro-4-[(dimethylamino)methylen]-5H-2-benzazepin-5-on als gelbe Prismen vom Smp. 228—233°.

Methode B

Eine Mischung aus 10 g (35 mMol) rohem 8-Chlor-3,4-dihydro-1-(2-fluorphenyl)-5H-2-benzazepin-5-on und 10 g (84 mMol) Dimethylformamid-dimethylacetal in 10 ml Dimethylformamid wird während 12 Stunden bei Raumtemperatur gerührt. Der erhaltene Niederschlag wird abfiltriert und nacheinander mit Äthanol und Äther gewaschen, wobei bräunliche Kristalle anfallen, die in allen Belangen mit authentischem Material identisch sind.

Beispiel 43

Die Herstellung von 8-Chlor-1-phenyl-3,4-dihydro-4-[(dimethylamino)methylen]-5H-2-benzazepin-5-on erfolgt auf die gleiche Weise wie die Herstellung von 8-Chlor-1-(2-fluorphenyl)-3,4-dihydro-4-[(dimethylamino)methylen]-5H-2-benzazepin-5-on (Methode A) und ergibt gelbe Prismen vom Smp. 180—183°.

Beispiel 44

Eine Mischung aus 6,4 g (22 mMol) 8-Chlor-1-(2-fluorphenyl)-3,4-dihydro-5H-2-benzazepin-5-on und 6,4 g (34 mMol) m-Chlorperbenzoesäure in 350 ml Methylenchlorid wird während 2 Stunden bei Raumtemperatur gerührt. Die Methylenchlorid-Lösung wird mit gesättigter Natriumbicarbonat-Lösung und Wasser gewaschen, über Natriumsulfat getrocknet und im Vakuum eingedampft. Das gelbe Öl wird aus Äther/Petroläther kristallisiert und ergibt weißliche Prismen vom Smp. 166—168°. Durch Umkristallisieren aus Äther/Methylenchlorid erhält man 8-Chlor-1-(2-fluorphenyl)-3,4-dihydro-5H-2-benzazepin-5-on-2-oxid als farblose Prismen vom Smp. 168—170°.

Beispiel 45

Die Herstellung von 8-Chlor-1-(2-chlorphenyl)-3,4-dihydro-5H-2-benzazepin-5-on-2-oxid erfolgt auf die gleiche Weise wie die Herstellung von 8-Chlor-1-(2-fluorphenyl)-3,4-dihydro-5H-2-benzazepin-5-on-2-oxid und ergibt gelbe Prismen vom Smp. 184—187°.

Beispiel 46

Eine Mischung aus 3,4 g (11 mMol) 8-Chlor-1-(2-fluorphenyl)-3,4-dihydro-5H-2-benzazepin-5-on-2-oxid und 26 ml Dimethylformamid-dimethylacetal wird während 12 Stunden bei Raumtemperatur gerührt. Nach Verdünnen der Mischung mit Äther und Abfiltrieren des Niederschlages erhält man einen gelben Festkörper vom Smp. 175—178°. Durch Umkristallisieren aus Äther/Essigester bekommt man 8-Chlor-1-(2-fluorphenyl)-3,4-dihydro-4-[(dimethylamino)methylen]-5H-2-benzazepin-5-on-2-oxid als gelbe Nadeln vom Smp. 193—194°.

Beispiel 47

Die Herstellung von 8-Chlor-1-(2-chlorphenyl)-3,4-dihydro-4-[(dimethylamino)methylen]-5H-2-benzazepin-5-on-2-oxid erfolgt auf die gleiche Weise wie die Herstellung von 8-Chlor-1-(2-fluorphenyl)-3,4-dihydro-4-[(dimethylamino)methylen]-5H-2-benzazepin-5-on-2-oxid und ergibt gelbe Prismen vom Smp. 196—198°.

Beispiel 48

Eine Mischung aus 1 g (2,8 mMol) 8-Chlor-1-(2-chlorphenyl)-4-[(dimethylamino)methylen]-3,4-dihydro-5H-2-benzazepin-5-on-2-oxid, 1,0 g (11 mMol) Acetamidin-hydrochlorid und 2,0 ml (9,9 mMol) einer 4,46 M methanolischen Natriummethylat-Lösung in einer Mischung von 20 ml Methanol und 20 ml Methylenchlorid wird während 2 Stunden bei Raumtemperatur gerührt. Nach Verdünnen der Mischung mit Wasser und Extrahieren mit Methylenchlorid wird die organische Phase mit Wasser gewaschen, über Natriumsulfat getrocknet und im Vakuum zur Trockene eingedampft. Umkristallisieren des Rückstandes aus Äther/Methylenchlorid ergibt 9-Chlor-7-(2-chlorphenyl)-2-methyl-5H-pyrimido[5,4-d] [2]benzazepin-6-oxid als farblosen Festkörper vom Smp. 215—216°.

Beispiel 49

Eine Lösung von 3,7 g (9,7 mMol) 9-Chlor-7-(2-fluorphenyl)-5H-pyrimido[5,4-d] [2]benzazepin-5-ol-acetat in 25 ml Tetrahydrofuran, 50 ml Methanol und 2 ml 3 N Natronlauge wird während 0,5 Stunden bei Raumtemperatur gerührt. Das Reaktionsgemisch wird auf Eiswasser gegossen und mit Methylenchlorid extrahiert. Die organische Phase wird abgetrennt, über Natriumsulfat getrocknet und im Vakuum zur Trockene eingedampft. Der Rückstand kristallisiert aus Methylenchlorid/Äther und ergibt rohes 9-Chlor-7-(2-fluorphenyl)-5H-pyrimido[5,4-d] [2]benzazepin-5-ol vom Smp. 186—188°. Durch Umkristallisieren aus Äther/Methylenchlorid resultieren cremefarbene Prismen vom Smp. 196—198°.

Beispiel 50

Eine Lösung von 6,8 g (20 mMol) 9-Chlor-7-(2-chlorphenyl)-5H-pyrimido[5,4-d] [2]benzazepin, 6 g (30 mMol) m-Chlorperbenzoesäure (85%) in 200 ml Methylenchlorid wird während 4 Stunden bei Raumtemperatur gerührt. Die Mischung wird mit überschüssiger, kalter, verdünnter Natronlauge gewaschen, über Natriumsulfat getrocknet und über Hyflo filtriert. Nach Eindampfen des Filtrates im Vakuum wird der Rückstand aus Methylenchlorid/Äther kristallisiert und ergibt das Rohprodukt vom Smp. 228—229°. Durch Umkristallisieren aus Methylenchlorid/Äther erhält man 9-Chlor-7-(2-chlorphenyl)-5H-pyrimido[5,4-d] [2]benzazepin-6-oxid vom Smp. 216—217°.

Beispiel 51

Eine Mischung aus 3 g (8 mMol) 9-Chlor-7-(2-chlorphenyl)-5H-pyrimido[5,4-d] [2]benzazepin-6-oxid und 50 ml Essigsäureanhydrid wird während 22 Stunden auf dem Dampfbad erwärmt. Nach Eindampfen der Reaktionsmischung wird der Rückstand aus Methylenchlorid/Äther kristallisiert, dabei erhält man 9-Chlor-7-(2-chlorphenyl)-5H-pyrimido[5,4-d] [2]benzazepin-5-ol-acetat vom Smp. 211—212°. Umkristallisieren aus demselben Lösungsmittelgemisch ergibt reines Produkt als farblose Prismen vom Smp. 211—212°.

Beispiel 52

Eine Lösung von 4,8 g (12 mMol) 6-Chlor-7-(2-chlorphenyl)-5H-pyrimido[5,4-d] [2]benzazepin-5-ol-acetat in 50 ml Tetrahydrofuran, 50 ml Methanol und 4 ml 3 N Natronlauge wird während 30 Minuten bei Raumtemperatur gerührt. Das Reaktionsgemisch wird auf Eiswasser gegossen und mit Methylenchlorid extrahiert. Die organische Phase wird abgetrennt, über Natriumsulfat getrocknet und im Vakuum zur Trockene eingedampft. Durch Kristallisieren des Rückstandes aus Methylenchlorid/Äther erhält man 9-Chlor-7-(2-chlorphenyl)-5H-pyrimido[5,4-d] [2]benzazepin-5-ol vom Smp. 105—117°. Das reine Produkt erhält man durch Umkristallisieren aus Methylenchlorid/Aceton, es resultieren farblose Prismen vom Smp. 174—175°.

Beispiel 53

Eine Lösung von 3,0 g (0,00838 mMol) 2,9-Dichlor-7-(2-fluorphenyl)-5H-pyrimido[5,4-d] [2]benzazepin in 8 ml N,N-Dimethylformamid wird mit 2 g (0,020 Mol) N-Methylpiperazin versetzt. Nach 20 Stunden gibt man 40 ml Eiswasser hinzu und filtriert den entstandenen Niederschlag ab. Nach Verteilen des Festkörpers zwischen 50 ml Methylenchlorid und 50 ml 1 N Salzsäure wird der pH der wäßrigen Phase mit Ammoniumhydroxid auf 1—2 eingestellt. Der entstandene Niederschlag wird abfiltriert und 2mal aus Methanol umkristallisiert, dabei erhält man weiße Prismen vom Smp. 187—194°. Die saure, wäßrige Phase wird mit Ammoniumhydroxid basisch gestellt und mit 100 ml Methylenchlorid extrahiert. Die Methylenchlorid-Lösung wird getrocknet und eingedampft. Das erhaltene Öl wird mit 1 N Salzsäure angesäuert und der pH mit Ammoniumhydroxid auf 1—2 eingestellt. Nach Abkühlen und Abfiltrieren wird der Niederschlag aus Methanol umkristallisiert, dabei erhält man eine weitere Portion 9-Chlor-7-(2-fluorphenyl)-2-(4-methyl-1-piperazinyl)-5H-pyrimido[5,4-d] [2]benzazepin-hydrochlorid.

Beispiel 54

Unter Stickstoff gibt man zu 20 ml Diäthylmalonat unter Rühren 1,9 g (16,8 mMol) Kalium-tert.-butylat und 15 Minuten später 2,0 g (5,59 mMol) 2,9-Dichlor-7-(2-fluorphenyl)-5H-pyrimido[5,4-d] [2]benzazepin. Die Reaktionsmischung wird während 2 Stunden bei 110° und während 4 Stunden bei 140° gehalten. Nach Zugabe von Eis wird mit konzentrierter Salzsäure angesäuert und mit 100 ml Äther

extrahiert. Die ätherische Phase wird mit 25 ml 3 N Salzsäure extrahiert. Die vereinigten sauren Auszüge werden mit Ammoniumhydroxid basisch gestellt und 2mal mit je 100 ml Äther ausgeschüttelt. Die ätherische Lösung wird getrocknet, über Aktivkohle filtriert und auf ein kleines Volumen eingeengt. Nach Zugabe von Petroläther wird der 9-Chlor-7-(2-fluorphenyl)-5H-pyrimido[5,4-d] [2]benzazepin-2-essigsäure-äthylester abfiltriert und aus denselben Lösungsmitteln umkristallisiert, dabei erhält man weiße Stäbchen vom Smp. 98—103°.

Beispiel 55

Eine Lösung von 4,0 g (9,76 mMol) 9-Chlor-7-(2-fluorphenyl)-5H-pyrimido[5,4-d] [2]benzazepin-2-essigsäure-äthylester in 30 ml Äthanol und 25 ml 1 N Natronlauge wird während 3 Stunden auf dem Dampfbad erwärmt. Die Reaktionsmischung wird zwischen 75 ml Wasser und 75 ml Äther verteilt. Der basische Auszug wird mit Essigsäure sauergestellt und 2mal mit je 100 ml Methylenchlorid extrahiert. Nach Eindampfen wird das erhaltene Öl aus Methanol kristallisiert und aus Methylenchlorid/Äther/Petroläther umkristallisiert. Man erhält 9-Chlor-7-(2-fluorphenyl)-5H-pyrimido[5,4-d] [2]benzazepin-2-essigsäure als weißliche Prismen vom Smp. 138—140°.

Beispiel 56

Während 10 Minuten leitet man Methylamin durch eine Lösung von 2,6 g (6,34 mMol) 9-Chlor-7-(2-fluorphenyl)-5H-pyrimido[5,4-d] [2]benzazepin-2-essigsäure-äthylester in 60 ml Äthanol. Man läßt während 18 Stunden stehen, dampft die Reaktionsmischung ein und verteilt den Rückstand zwischen 75 ml Methylenchlorid und 50 ml Wasser. Nach Trocknen und Eindampfen der organischen Phase wird der Rückstand aus Methylenchlorid/Äther kristallisiert. Durch Umkristallisieren aus demselben Lösungsmittelgemisch erhält man 9-Chlor-7-(2-fluorphenyl)-N-methyl-5H-pyrimido[5,4-d] [2]benzazepin-2-acetamid als weiße Stäbchen vom Smp. 175—177°.

Beispiel 57

Eine Mischung aus 90,5 g (0,25 Mol) 8-Chlor-1-(2-chlorphenyl)-3,4-dihydro-4-[(dimethylamino)methylen]-5H-2-benzazepin-5-on, 100 g (0,96 Mol) Formamidinacetat und 1,0 l Formamid wird während 16 Stunden auf dem Dampfbad erwärmt. Nach Abkühlen der Reaktionsmischung auf 0° wird der erhaltene Niederschlag abfiltriert, mit Wasser gewaschen und bis zum konstanten Gewicht getrocknet. Man erhält 9-Chlor-7-(2-chlorphenyl)-5H-pyrimido[5,4-d] [2]benzazepin als weißliche Kristalle vom Smp. 120—121°.

Beispiel 58

Eine Mischung aus 0,4 g (1,1 mMol) 8-Chlor-1-(2-fluorphenyl)-3,4-dihydro-4-[(dimethylamino)methylen]-5H-2-benzazepin-5-on-2-oxid, 1,0 g (9,6 mMol) Formamidinacetat und 20 ml Formamid wird während 6 Stunden auf dem Dampfbad erwärmt. Die Reaktionsmischung wird auf Eis gegossen und mit Methylenchlorid ausgeschüttelt. Die Methylenchlorid-Lösung wird mit Wasser gewaschen, über Natriumsulfat getrocknet und im Vakuum eingedampft. Durch Zugabe von Äther/Methylenchlorid kristallisiert der Rückstand und ergibt 9-Chlor-7-(2-fluorphenyl)-5H-pyrimido[5,4-d] [2]benzazepin-6-oxid als weißliche Kristalle vom Smp. 186—188°.

Beispiel 59

Eine Mischung aus 0,4 g (1,1 mMol) 8-Chlor-1-(2-chlorphenyl)-3,4-dihydro-4-[(dimethylamino)methylen]-5H-2-benzazepin-5-on-2-oxid und 1,0 g (9,6 mMol) Formamidinacetat in 20 ml Formamid wird während 7 Stunden auf dem Dampfbad erwärmt. Die Mischung wird auf Eis gegossen und mit Methylenchlorid extrahiert. Die Methylenchlorid-Lösung wird mit Wasser gewaschen, über Natriumsulfat getrocknet und im Vakuum eingedampft. Der Rückstand wird mit Äther verrieben, dabei erhält man 9-Chlor-7-(2-chlorphenyl)-5H-pyrimido[5,4-d] [2]benzazepin-6-oxid als weißlicher Festkörper vom Smp. 215—217°.

Beispiel 60

Eine Mischung aus 0,5 g (1,3 mMol) 9-Chlor-7-(2-chlorphenyl)-5H-pyrimido[5,4-d] [2]benzazepin-6-oxid, 1,0 ml (10 mMol) Phosphortrichlorid und 20 ml Methylenchlorid wird während 3 Stunden unter Rückfluß zum Sieden erhitzt. Nach Abkühlen gibt man auf Eis, stellt mit Ammoniumhydroxid basisch und extrahiert mit Methylenchlorid. Die Methylenchloridphase wird mit Wasser gewaschen, über Natriumsulfat getrocknet und im Vakuum eingedampft. Der Rückstand kristallisiert durch Zugabe von Äther und ergibt 9-Chlor-7-(2-chlorphenyl)-5H-pyrimido[5,4-d] [2]benzazepin als farblose Prismen vom Smp. 121—123°.

Beispiel 61

Eine Mischung aus 0,5 g (1,5 mMol) 9-Chlor-7-(2-fluorphenyl)-5H-pyrimido[5,4-d] [2]benzazepin-6-oxid, 1,0 ml (10 mMol) Phosphortrichlorid und 20 ml Methylenchlorid wird während 2 Stunden unter Rückfluß zum Sieden erhitzt. Man gießt auf Eis, stellt mit Ammoniumhydroxid basisch und extrahiert mit Methylenchlorid. Die Methylenchlorid-Lösung wird über Natriumsulfat getrocknet und im Vakuum eingedampft. Aus Äther resultiert 9-Chlor-7-(2-fluorphenyl)-5H-pyrimido[5,4-d] [2]benzazepin als weißliche Kristalle vom Smp. 122—124°.

Beispiel 62

Eine Mischung aus 1,5 g (4 mMol) 8-Chlor-1-(2-fluorphenyl)-3,4-dihydro-4-[(dimethylamino)methylen]-5H-2-benzazepin-5-on-2-oxid, 1,5 g (20 mMol) Thioharnstoff und 5 ml einer 4 M methanolischen Natriummethylat-Lösung in 30 ml Methanol wird während 5 Stunden bei Raumtemperatur gerührt. Man gießt die Reaktionsmischung auf Wasser und extrahiert mit Äther. Die wäßrige Phase wird mit Essigsäure sauergestellt und mit Methylenchlorid ausgeschüttelt. Die Methylenchlorid-Lösung wird über Natriumsulfat getrocknet und im Vakuum eingedampft. Der Rückstand wird mit Methylenchlorid zerrieben und ergibt einen orangefarbenen Festkörper. Durch Umkristallisieren aus Methylenchlorid erhält man 9-Chlor-7-(2-fluorphenyl)-5H-pyrimido[5,4-d] [2]benzazepin-2-thiol-6-oxid als orangefarbene Kristalle vom Smp. 323—325°.

Beispiel 63

Eine Mischung aus 68 g (0,2 Mol) 9-Chlor-7-(2-chlorphenyl)-5H-pyrimido[5,4-d] [2]benzazepin, 27 g Zinkstaub, 250 ml Essigsäure und 600 ml Methylenchlorid wird während 2 Stunden bei —30° gerührt. Die Mischung wird über Hyflo in eine gerührte Mischung von 600 ml konzentrierter Ammoniumhydroxid-Lösung und 500 ml Eis filtriert. Die Methylenchloridphase wird abgetrennt, über Natriumsulfat getrocknet und im Vakuum eingedampft. Der Rückstand wird aus Methylenchlorid/Äther kristallisiert und ergibt 9-Chlor-7-(2-chlorphenyl)-6,7-dihydro-5H-pyrimido[5,4-d] [2]benzazepin als farbloser Festkörper. Nach Umkristallisieren aus Äther/Methylenchlorid resultieren farblose Nadeln vom Smp. 169—170°.

Beispiel 64

Eine Lösung von 14,5 g (42 mMol) 9-Chlor-7-(2-chlorphenyl)-6,7-dihydro-5H-pyrimido[5,4-d] [2]benzazepin, 14,5 g (76 mMol) p-Toluolsulfonsäurechlorid, 30 ml Pyridin und 0,3 g 4-Dimethylaminopyridin in 300 ml Methylenchlorid wird während 24 Stunden bei Raumtemperatur gerührt. Die Mischung wird mit einem Überschuß an kalter, verdünnter Salzsäure und verdünnter Natronlauge gewaschen. Nach Trocknen der Methylenchlorid-Lösung über Natriumsulfat und Eindampfen im Vakuum wird der Rückstand aus Methylenchlorid/Äther kristallisiert. Dabei erhält man 9-Chlor-7-(2-chlorphenyl)-6,7-dihydro-6-[(4-methylphenyl)-sulfonyl]-5H-pyrimido[5,4-d] [2]benzazepin als weißen Festkörper vom Smp. 200—201°. Durch Umkristallisieren aus Äther/Methylenchlorid bekommt man das reine Produkt als farblose Prismen vom Smp. 200—201°.

Beispiel 65

Bei 0° wird eine Lösung von 6,4 g (19 mMol) 9-Chlor-7-(2-chlorphenyl)-6,7-dihydro-5H-pyrimido[5,4-d] [2]benzazepin und 10 ml (12,8 mMol) Pyridin in 75 ml Methylenchlorid tropfenweise mit 5,0 ml (35 mMol) Trifluoressigsäureanhydrid versetzt. Man rührt während 1 Stunde und gießt auf kalte, verdünnte Salzsäure. Die Methylenchlorid-Lösung wird abgetrennt, mit gesättigter Kochsalzlösung

gewaschen, über Natriumsulfat getrocknet und im Vakuum eingedampft. Der Rückstand wird aus Äther kristallisiert und ergibt rosafarbene Kristalle vom Smp. 178—179°. Durch Umkristallisieren aus Äther erhält man 9-Chlor-7-(2-chlorphenyl)-6,7-dihydro-6-(trifluoracetyl)-5H-pyrimido[5,4-d] [2]benzazepin als weißliche Kristalle vom Smp. 179—180°.

Beispiel 66

Eine Lösung von 7,6 g (15 mMol) 9-Chlor-7-(2-chlorphenyl)-6,7-dihydro-6-[(4-methylphenyl)sulfonyl]-5H-pyrimido[5,4-d] [2]benzazepin und 4,0 g (20 mMol) m-Chlorperbenzoesäure in 300 ml Methylenchlorid wird während 48 Stunden bei Raumtemperatur gerührt. Die Mischung wird mit kalter, verdünnter Natronlauge und gesättigter Kochsalzlösung gewaschen. Die Methylenchlorid-Lösung wird über Natriumsulfat getrocknet und im Vakuum zur Trockene eingedampft. Die Reinigung des Rückstandes (8,2 g) erfolgt durch Säulenchromatographie an 20 g Silicagel, unter Eluieren mit Methylenchlorid und anschließend mit Essigester. Aus der Methylenchlorid-Fraktion erhält man 2,0 g des Ausgangsmaterials als farblosen Festkörper. Die Essigester-Fraktion ergibt 2,1 g 9-Chlor-7-(2-chlorphenyl)-6,7-dihydro-6-[(4-methylphenyl)sulfonyl]-5H-pyrimido[5,4-d] [2]benzazepin-3-oxid als weißlichen Festkörper vom Smp. 242—243°. Durch Umkristallisieren aus Äther/Methylenchlorid erhält man farblose Kristalle vom Smp. 243—244°.

Beispiel 67

Methode A

Eine Mischung aus 2,0 g (3,9 mMol) 9-Chlor-7-(2-chlorphenyl)-6,7-dihydro-6-[(4-methylphenyl)sulfonyl]-5H-pyrimido[5,4-d] [2]benzazepin-3-oxid und 8 ml einer 4 M methanolischen Natriummethylat-Lösung in 130 ml Tetrahydrofuran und 180 ml Methanol wird während 19 Stunden bei Raumtemperatur gerührt. Die Mischung wird auf kalte Kochsalzlösung gegossen und mit Methylenchlorid extrahiert. Die organische Phase wird mit Kochsalzlösung gewaschen, über Natriumsulfat getrocknet und im Vakuum eingedampft. Die Reinigung des Rückstandes erfolgt durch Säulenchromatographie an 20 g Silicagel, unter Eluieren mit Äther/Methylenchlorid (1 : 4) und Methanol/Methylenchlorid (1 : 9). Durch Eindampfen der Äthanol/Methylenchlorid-Fraktion erhält man 9-Chlor-7-(2-chlorphenyl)-5H-pyrimido[5,4-d] [2]benzazepin-3-oxid als weißen Festkörper. Durch Umkristallisieren aus Methylenchlorid/Äther erhält man lange, farblose Prismen vom Smp. 189—190°.

Methode B

Eine Mischung aus 1,5 g (4,3 mMol) 9-Chlor-7-(2-chlorphenyl)-6,7-dihydro-5H-pyrimido[5,4-d] [2]benzazepin-3-oxid, 6 ml einer 5proz. Methylenchlorid-Lösung von Brom und 600 ml Methylenchlorid wird während 30 Minuten bei Raumtemperatur gerührt. Die Mischung wird durch Zugabe von 4,5 ml (32 mMol) Triäthylamin basisch gestellt und während 10 Minuten gerührt. Die Mischung wird mit gesättigter Kochsalzlösung gewaschen, über Natriumsulfat getrocknet und im Vakuum eingedampft. Der Rückstand wird durch Säulenchromatographie an 50 g Silicagel unter Eluieren mit Methylenchlorid, Essigester und schließlich mit Tetrahydrofuran/Methylenchlorid (3 : 7), gereinigt. Die Essigester-Fraktion enthält einen farblosen Festkörper vom Smp. 242—244°. Es handelt sich dabei um eine zum gewünschten Produkt isomere Verbindung, bezüglich der Iminbindung. Die Tetrahydrofuran/Methylenchlorid-Fraktion enthält 9-Chlor-7-(2-chlorphenyl)-5H-pyrimido[5,4-d] [2]benzazepin-3-oxid als farblose Prismen vom Smp. 189—190°.

### Beispiel 68

Die Herstellung von 9-Chlor-7-(2-chlorphenyl)-6,7-dihydro-6-(trifluoracetyl)-5H-pyrimido[5,4-d] [2]benzazepin-3-oxid erfolgt auf die gleiche Weise wie die Herstellung von 9-Chlor-7-(2-chlorphenyl)-6,7-dihydro-6-[(4-methylphenyl)sulfonyl]-5H-pyrimido[5,4-d] [2]benzazepin-3-oxid und ergibt farblose Kristalle vom Smp. 209—211°.

### Beispiel 69

Eine Mischung aus 4,9 g (11 mMol) 9-Chlor-7-(2-chlorphenyl)-6,7-dihydro-6-(trifluoracetyl)-5H-pyrimido[5,4-d] [2]benzazepin-3-oxid, 50 ml 3 N Natronlauge, 100 ml Äthanol und 100 ml Tetrahydrofuran wird während 30 Minuten bei Raumtemperatur gerührt. Die Mischung wird im Vakuum auf ein kleines Volumen eingeengt, und der entstandene Niederschlag abfiltriert. Man erhält einen farblosen Festkörper vom Smp. 259—260°. Durch Umkristallisieren aus Tetrahydrofuran erhält man 9-Chlor-7-(2-chlorphenyl)-6,7-dihydro-5H-pyrimido[5,4-d] [2]benzazepin-3-oxid als farblose Kristalle vom Smp. 263—264°.

### Beispiel A

Herstellung von Tabletten (Naßgranulierung)

| Bestandteile | mg/Tablette | mg/Tablette | mg/Tablette | mg/Tablette |
|---|---|---|---|---|
| 1. 2-Amino-8-chlor-7-(2-fluorphenyl)-5H-pyrimido[5,4-d] [2]benzazepin oder 7-Phenyl-5H-pyrimido[5,4-d]-benzazepin-2-amin | 1 | 5 | 10 | 25 |
| 2. Lactose | 202 | 232 | 261 | 280 |
| 3 Modifizierte Stärke | 25 | 35 | 45 | 55 |
| 4. Vorgelatinisierte Stärke | 20 | 25 | 30 | 35 |
| 5. Destilliertes Wasser q.s. | — | — | — | — |
| 6. Magnesiumstearat | 2 | 3 | 4 | 5 |
| Tablettengewicht | 250 mg | 300 mg | 350 mg | 400 mg |

Man mischt die Bestandteile 1—4 in einem geeigneten Mixer und granuliert mit genügend Wasser um eine gute Konsistenz zu erhalten. Nach dem Mahlen wird in einem geeigneten Ofen getrocknet. Anschließend mahlt und vermischt man während 3 Minuten mit Magnesiumstearat. Schließlich wird das Gemisch maschinell zu Tabletten entsprechender Größe verpreßt.

Beispiel B

Herstellung von Tabletten (Direkte Verpressung)

| Bestandteile | mg/Tablette | mg/Tablette | mg/Tablette | mg/Tablette |
|---|---|---|---|---|
| 1. 2-Amino-9-chlor-7-(2-fluorphenyl)-5H-pyrimido[5,4-d] [2]benzazepin oder 7-Phenyl-5H-pyrimido[5,4-d]-benzazepin-2-amin | 1 | 5 | 10 | 25 |
| 2. Lactose | 221 | 217 | 212 | 181 |
| 3. Avicel | 45 | 45 | 45 | 55 |
| 4. Stärke (Direkt verpreßbar) | 30 | 30 | 30 | 35 |
| 5. Magnesiumstearat | 3 | 3 | 3 | 4 |
| Tablettengewicht | 300 mg | 300 mg | 300 mg | 300 mg |

Man mischt Bestandteil 1 mit einer äquivalenten Menge Lactose. Anschließend wird diese Mischung mit den Bestandteilen 3, 4 und dem Rest von Bestandteil 2 gut vermischt. Nach Zugabe von Magnesiumstearat mischt man während 3 Minuten. Die Mischung wird schließlich maschinell zu Tabletten entsprechender Größe verpreßt.

Beispiel C

Herstellung von Kapseln

| Bestandteile | mg/Tablette | mg/Tablette | mg/Tablette | mg/Tablette |
|---|---|---|---|---|
| 1. 2-Amino-9-chlor-7-(2-fluorphenyl)-5H-pyrimido[5,4-d] [2]benzazepin oder 7-Phenyl-5H-pyrimido[5,4-d]-benzazepin-2-amin | 1 | 5 | 10 | 25 |
| 2. Lactose | 203 | 293,5 | 328 | 372,5 |
| 3. Stärke | 30 | 35 | 40 | 30 |
| 4. Talk | 15 | 15 | 20 | 20 |
| 5. Aerosol OT | 1 | 1,5 | 2 | 2,5 |
| Füllgewicht der Kapseln | 250 mg | 350 mg | 400 mg | 450 mg |

Die Bestandteile 1, 2, 3 und 5 werden in einem geeigneten Mixer gut vermischt. Nach Zugabe von Talk wird wiederum gut vermischt und die Mischung mit einer geeigneten Maschine in Kapseln abgefüllt.

**Patentansprüche für die Vertragsstaaten: BE, CH, DE, FR, IT, LI, LU, NL, SE**

1. Pyrimido-2-benzazepine der allgemeinen Formel

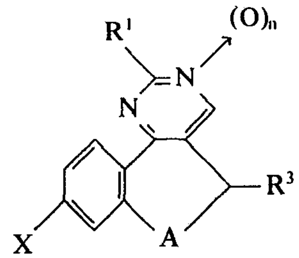

(I)

worin A eine der Gruppen

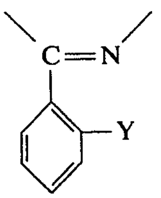

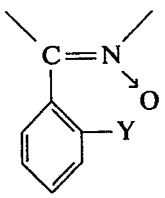

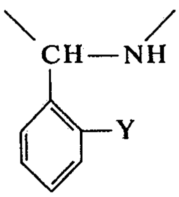

und

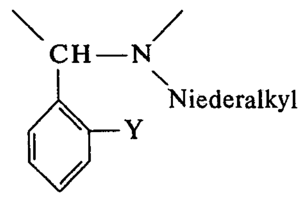

(a) (b) (c) (d)

$R^1$ Wasserstoff, Chlor, Brom, niederes Alkyl, die Gruppe $NR^4R^5$, die Gruppe $-CH_2-CO-R^7$, die Gruppe $-NH(CH_2)_mNR^8R^9$, Hydroxy, niederes Alkoxy, Mercapto oder niederes Alkylmercapto, $R^3$ Wasserstoff, niederes Acyloxy oder Hydroxy, X Wasserstoff, Halogen, Trifluormethyl, Äthyl, $\alpha$-Hydroxyäthyl oder Acetyl, Y Wasserstoff oder Halogen, $R^4$ und $R^5$ je Wasserstoff oder niederes Alkyl oder, zusammen mit dem Stickstoffatom, einen 5- bis 7gliedrigen Heterocyclus, welcher ein Sauerstoff- oder Schwefelatom oder die Gruppe $>$N-Niederalkyl enthalten kann, $R^7$ Hydroxy, niederes Alkoxy oder $NR^8R^9$, $R^8$ und $R^9$ je Wasserstoff oder niederes Alkyl, n 0 oder 1 und m 1 bis 7 bedeuten, mit der Maßgabe, daß die als »nieder« bezeichneten Gruppen höchstens 7 Kohlenstoffatome aufweisen und mit den folgenden Maßgaben:

(i) wenn $R^3$ niederes Acyloxy oder Hydroxy bedeutet, dann bedeuten A Gruppe (a), X Wasserstoff, Halogen, Trifluormethyl, Äthyl oder Acetyl und, falls $R^1$ die Gruppe $-NH(CH_2)_mNR^8R^9$ bedeutet, dann bedeuten $R^8$ und $R^9$ je niederes Alkyl;
(ii) wenn A Gruppe (d) und $R^1$ Gruppe $-NH(CH_2)_mNR^8R^9$ bedeuten, dann bedeuten $R^8$ und $R^9$ je niederes Alkyl; und
(iii) wenn n 1 bedeutet, dann bedeuten entweder A Gruppe (b) und $R^1$ Wasserstoff, niederes Alkyl, Hydroxy, niederes Alkoxy, $NR^{41}R^{51}$ (worin $R^{41}$ und $R^{51}$ je Wasserstoff oder niederes Alkyl oder, zusammen mit dem Stickstoffatom, einen 5- bis 7gliedrigen Heterocyclus, welcher ein Sauerstoffatom enthalten kann, bedeuten), Chlor, Brom oder die Gruppe $-CH_2-CO-R^7$ oder es bedeuten A Gruppe (a) und $R^1$ Wasserstoff, niederes Alkyl, niederes Alkoxy, Chlor, Brom oder Gruppe $-CH_2-CO-R^7$;

und pharmazeutisch akzeptable Säureadditionssalze davon.

2. Verbindungen nach Anspruch 1, worin A Gruppe (b), $R^1$ Amino, n 0, X Wasserstoff, Halogen mit einer Atomnummer von höchstens 35 oder Trifluormethyl und Y Wasserstoff oder Halogen mit einer Atomnummer von höchstens 35 bedeuten.

3. Verbindungen nach Anspruch 1, worin $R^1$ Wasserstoff, Chlor, Brom, niederes Alkyl, die Gruppe $NR^8R^9$ (worin $R^8$ und $R^9$ je Wasserstoff oder niederes Alkyl sind), die Gruppe $-CH_2-CO-R^7$ (worin $R^7$ niederes Alkoxy ist), Di-(nieder)-alkylamino-(nieder)-alkylamino, Hydroxy, niederes Alkoxy, Mercapto oder niederes Alkylmercapto bedeutet, mit den folgenden Maßgaben:

(i) wenn A Gruppe (b), (c) oder (d) ist oder wenn A Gruppe (a) und $R^3$ niederes Acyloxy oder Hydroxy sind, dann bedeutet $R^1$ Wasserstoff, niederes Alkyl oder $NR^8R^9$;
(ii) wenn n 1 ist, dann bedeutet A die Gruppe (b); und
(iii) wenn A die Gruppe (b), $R^1$ Amino und Y Wasserstoff oder Halogen mit einer Atomnummer von höchstens 35 sind, dann bedeutet X nicht Wasserstoff, Halogen mit einer Atomnummer von höchstens 35 oder Trifluormethyl.

4. Verbindungen nach Anspruch 1, worin A die Gruppe (a) und n 0 bedeuten.

5. Verbindungen nach Anspruch 4, worin $R^3$ Wasserstoff und $R^1$ Wasserstoff, niederes Alkyl, die

Gruppe $NR^4R^5$ (worin $R^4$ und $R^5$ je Wasserstoff oder niederes Alkyl sind), Hydroxy, Chlor, Brom, die Gruppe $-NH(CH_2)_mNR^8R^9$ (worin $R^8$ und $R^9$ je niederes Alkyl sind) oder die Gruppe $-CH_2-CO-R^7$ bedeuten.

6. Verbindungen nach Anspruch 5, worin $R^1$ Wasserstoff, Amino oder niederes Alkyl bedeutet.

7. Verbindungen nach Anspruch 4, worin $R^3$ Hydroxy und $R^1$ Wasserstoff, niederes Alkyl oder die Gruppe $NR^4R^5$ (worin $R^4$ und $R^5$ je Wasserstoff oder niederes Alkyl sind) bedeuten.

8. Verbindungen nach einem der Ansprüche 4—7, worin X Halogen bedeutet.

9. Verbindungen nach Anspruch 8, worin X Chlor bedeutet.

10. Verbindungen nach einem der Ansprüche 4—9, worin Y Wasserstoff, Chlor oder Fluor bedeutet.

11. 9-Chlor-7-(2-chlorphenyl)-5H-pyrimido[5,4-d] [2]benzazepin.

12. 9-Chlor-7-(2-fluorphenyl)-5H-pyrimido[5,4-d] [2]benzazepin.

13. 9-Chlor-7-(2-chlorphenyl)-2-methyl-5H-pyrimido[5,4-d] [2]benzazepin.

14. 9-Chlor-7-(2-fluorphenyl)-5H-pyrimido[5,4-d] [2]benzazepin-2-ol.

15. 9-Chlor-N,N-dimethyl-7-(2-fluorphenyl)-5H-pyrimido[5,4-d] [2]benzazepin-2-amin.

16. Verbindungen der allgemeinen Formeln

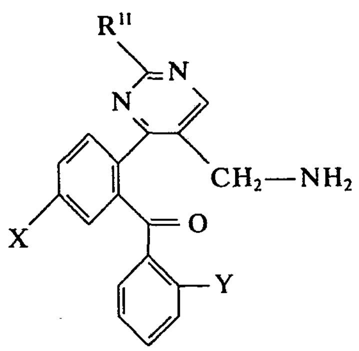

(II)

und

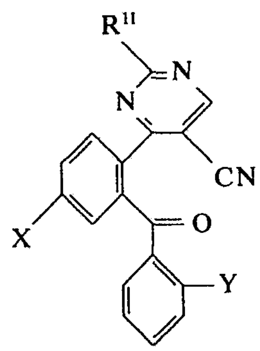

(XII)

worin X und Y die in Anspruch 1 angegebene Bedeutung besitzen und $R^{11}$ Wasserstoff, niederes Alkyl oder $NR^8R^9$ bedeutet, worin $R^8$ und $R^9$ die in Anspruch 1 angegebene Bedeutung besitzen.

17. Verbindungen nach Anspruch 16, worin X und Y je Chlor und $R^{11}$ Wasserstoff bedeuten.

18. Verbindungen nach einem der Ansprüche 1—10 und 12—15 als pharmazeutische Wirkstoffe.

19. Verbindungen nach einem der Ansprüche 1—10 und 12—15 als Anxiolytika und Sedativa.

20. 9-Chlor-7-(2-chlorphenyl)-5H-pyrimido[5,4-d] [2]benzazepin als pharmazeutischer Wirkstoff.

21. 9-Chlor-7-(2-chlorphenyl)-5H-pyrimido[5,4-d] [2]benzazepin als Anxiolytikum und Sedativum.

22. Verfahren zur Herstellung von Verbindungen nach einem der Ansprüche 1—15, dadurch gekennzeichnet, daß man

a) eine Verbindung der allgemeinen Formel

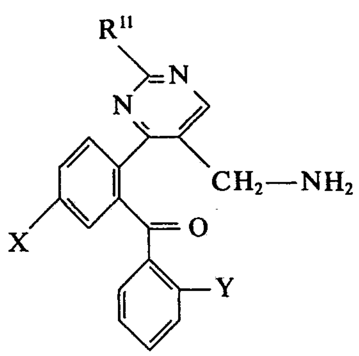

(II)

worin X und Y die in Anspruch 1 angegebene Bedeutung besitzen und $R^{11}$ Wasserstoff, niederes Alkyl oder $NR^8R^9$ bedeutet, worin $R^8$ und $R^9$ die in Anspruch 1 angegebene Bedeutung besitzen, cyclisiert oder

b) eine Verbindung der allgemeinen Formel

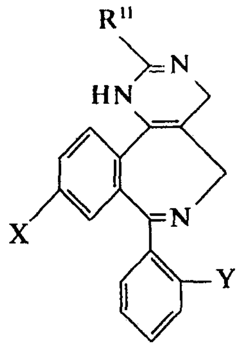

(III)

worin X und Y die in Anspruch 1 angegebene und $R^{11}$ obige Bedeutung besitzen, dehydriert oder

c) eine Verbindung der allgemeinen Formel

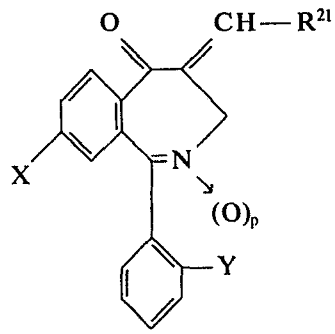

(IV)

worin X und Y die in Anspruch 1 angegebene Bedeutung besitzen und p 0 oder 1 und $R^{21}$ Di-niederalkylamino bedeuten,
mit einer Verbindung der allgemeinen Formel

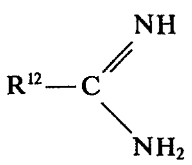

(V)

worin $R^{12}$ Wasserstoff, Mercapto, niederes Alkylmercapto, niederes Alkyl oder $NR^8R^9$ bedeutet, worin $R^8$ und $R^9$ die in Anspruch 1 angegebene Bedeutung besitzen,
zur Reaktion bringt oder

d) eine Verbindung der allgemeinen Formel

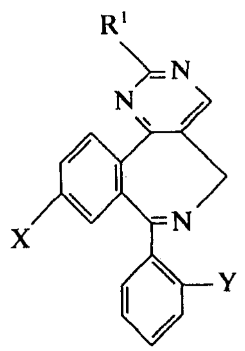

(Ia)

worin X, Y und $R^1$ die in Anspruch 1 angegebene Bedeutung besitzen, reduziert oder

e) eine Verbindung der allgemeinen Formel

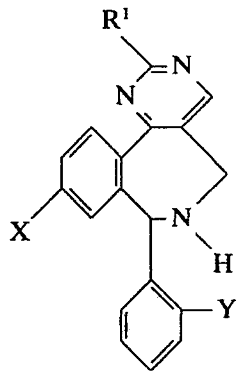

(Ib)

worin X, Y und $R^1$ die in Anspruch 1 angegebene Bedeutung besitzen, niederalkyliert oder

f) eine Verbindung der allgemeinen Formel

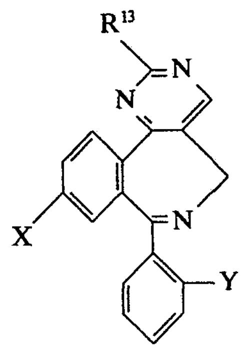

(Ic)

worin X und Y die in Anspruch 1 angegebene Bedeutung besitzen und $R^{13}$ Wasserstoff, niederes Alkyl, Hydroxy, niederes Alkoxy, $NR^{41}R^{51}$ (worin $R^{41}$ und $R^{51}$ je Wasserstoff oder niederes Alkyl oder, zusammen mit dem Stickstoffatom, einen 5- bis 7gliedrigen Heterocyclus, welcher ein Sauerstoffatom enthalten kann, sind), Chlor, Brom oder die Gruppe $-CH_2-CO-R^7$ (worin $R^7$ die in Anspruch 1 angegebene Bedeutung besitzt) bedeutet, oxidiert oder

g) eine Verbindung der allgemeinen Formel

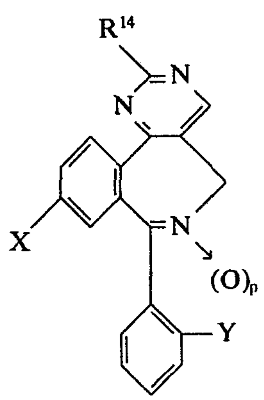

(Id)

worin X und Y die in Anspruch 1 angegebene und p obige Bedeutung besitzen und $R^{14}$ Mercapto oder Hydroxy bedeutet, niederalkyliert oder

h) eine Verbindung der allgemeinen Formel

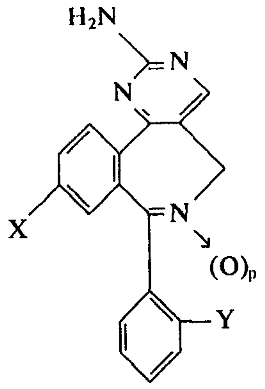

(Ie)

worin X und Y die in Anspruch 1 angegebene und p obige Bedeutung besitzen, in die entsprechende 2-Hydroxyverbindung überführt oder

i) eine Verbindung der allgemeinen Formel

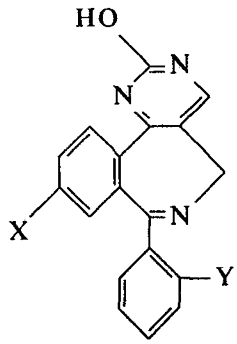

(If)

worin X und Y die in Anspruch 1 angegebene Bedeutung besitzen, in eine entsprechende 2-Chlor- oder 2-Brom-Verbindung überführt oder

k) eine Verbindung der allgemeinen Formel

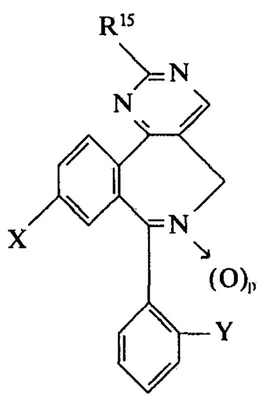

(Ig)

worin X und Y die in Anspruch 1 angegebene Bedeutung und p obige Bedeutung besitzen und $R^{15}$ Chlor oder Brom bedeutet,
mit Schwefelwasserstoff, mit einem niederen Alkylmercaptan, mit einem niederen Alkanol, mit einer Verbindung der Formel $HNR^4R^5$, worin $R^4$ und $R^5$ die in Anspruch angegebene Bedeutung besitzen, mit einer Verbindung der Formel $H_2N-(CH_2)_mNR^8R^9$, worin $R^8$, $R^9$ und m die in Anspruch 1 angegebene Bedeutung besitzen, oder mit dem Carbanion einer Verbindung der Formel

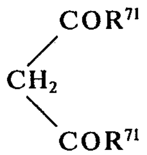 oder 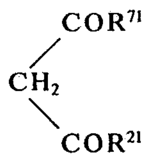

worin $R^{71}$ niederes Alkoxy bedeutet und $R^{21}$ obige Bedeutung besitzt,
behandelt oder

l) eine Verbindung der allgemeinen Formel

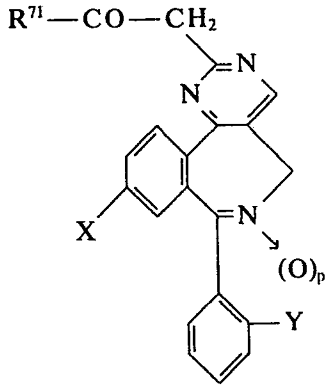

(Ih)

worin X und Y die in Anspruch 1 angegebene Bedeutung und $R^{71}$ und p obige Bedeutung besitzen, in die entsprechende freie Säure oder in ein entsprechendes Amid, Niederalkylamid oder D-nieder-alkylamid überführt oder

m) eine Verbindung der allgemeinen Formel

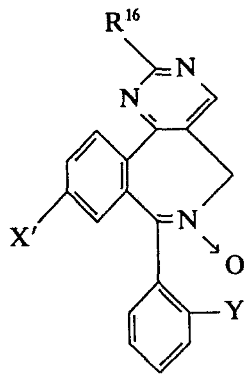

(Ii)

worin Y die in Anspruch 1 angegebene Bedeutung besitzt, $R^{16}$ Wasserstoff, Chlor, Brom, niederes Alkyl, die Gruppe $NR^4R^5$, die Gruppe $-CH_2-CO-R^7$, die Gruppe $NH(CH_2)_mNR^{81}R^{91}$, Hydroxy, niederes Alkoxy, Mercapto oder niederes Alkylmercapto, X' Wasserstoff, Halogen, Trifluormethyl, Äthyl oder Acetyl und $R^{81}$ und $R^{91}$ je niederes Alkyl bedeuten und $R^4$, $R^5$ und $R^7$ die in Anspruch 1 angegebene Bedeutung besitzen,
mit einem Niederacylierungsmittel zur Reaktion bringt oder

n) eine Verbindung der allgemeinen Formel

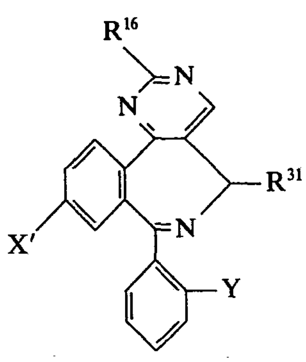

(Ik)

worin Y die in Anspruch 1 angegebene Bedeutung und X' und $R^{16}$ obige Bedeutung besitzen und $R^{31}$ niederes Acyloxy bedeutet,
hydrolysiert oder

o) eine Verbindung der allgemeinen Formel

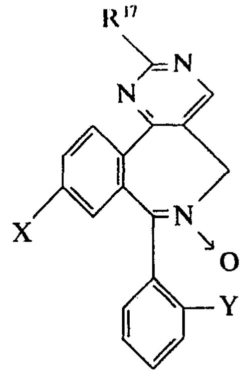

(II)

worin X und Y die in Anspruch 1 angegebene Bedeutung besitzen, $R^{17}$ Wasserstoff, niederes Alkyl, die Gruppe $NR^4R^5$, die Gruppe $-CH_2-CO-R^7$, die Gruppe $NH-(CH_2)_m-NR^8R^9$, Hydroxy, niederes Alkoxy, Mercapto oder niederes Alkylmercapto bedeutet und $R^4$, $R^5$, $R^7$, $R^8$ und $R^9$ die in Anspruch 1 angegebene Bedeutung besitzen,
desoxydiert oder

p) aus einer Verbindung der allgemeinen Formel

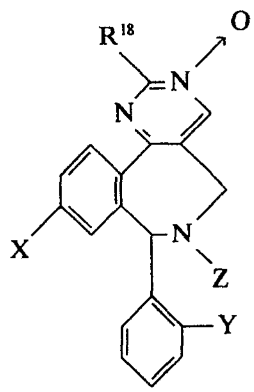

(III')

worin X und Y die in Anspruch 1 angegebene Bedeutung besitzen und $R^{18}$ Wasserstoff, niederes Alkyl, niederes Alkoxy, Chlor, Brom oder die Gruppe $-CH_2-CO-R^7$ (worin $R^7$ die in Anspruch 1 angegebene Bedeutung besitzt) und Z Wasserstoff oder eine leicht abspaltbare Acylgruppe bedeuten,
die Elemente von H—Z entfernt oder

q) eine Verbindung der in Anspruch 1 definierten Formel I in ein pharmazeutisch akzeptables Säureadditionssalz überführt.

23. Verfahren nach Anspruch 22, worin 9-Chlor-7-(2-chlorphenyl)-5H-pyrimido[5,4-d] [2]benzazepin hergestellt wird.

24. Arzneimittel, enthaltend eine Verbindung gemäß einem der Ansprüche 1—10 und 12—15.

25. Anxiolytikum und Sedativum, enthaltend eine Verbindung gemäß einem der Ansprüche 1—10 und 12—15.

26. Arzneimittel, enthaltend 9-Chlor-7-(2-chlorphenyl)-5H-pyrimido[5,4-d] [2]benzazepin oder ein pharmazeutisch akzeptables Säureadditionssalz davon.

27. Anxiolytikum und Sedativum, enthaltend 9-Chlor-7-(2-chlorphenyl)-5H-pyrimido[5,4-d] [2]benzazepin oder ein pharmazeutisch akzeptables Säureadditionssalz davon.

**Patentansprüche für den Vertragsstaat: AT**

1. Verfahren zur Herstellung von Pyrimido-2-benzazepinen der allgemeinen Formel

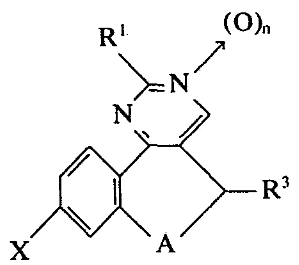

(I)

worin A eine der Gruppen

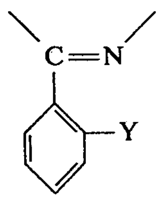 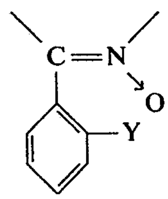 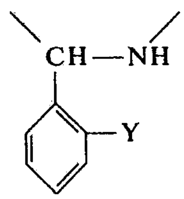 und 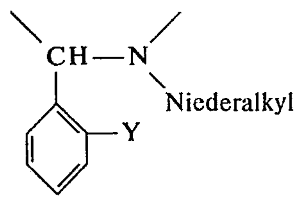

(a) (b) (c) (d)

$R^1$ Wasserstoff, Chlor, Brom, niederes Alkyl, die Gruppe $NR^4R^5$, die Gruppe $-CH_2-CO-R^7$, die Gruppe $-NH(CH_2)_mNR^8R^9$, Hydroxy, niederes Alkoxy, Mercapto oder niederes Alkylmercapto, $R^3$ Wasserstoff, niederes Acyloxy oder Hydroxy, X Wasserstoff, Halogen, Trifluormethyl, Äthyl, $\alpha$-Hydroxyäthyl oder Acetyl, Y Wasserstoff oder Halogen, $R^4$ und $R^5$ je Wasserstoff oder niederes Alkyl oder, zusammen mit dem Stickstoffatom, einen 5- bis 7gliedrigen Heterocyclus, welcher ein Sauerstoff- oder Schwefelatom oder die Gruppe $>$N-Niederalkyl enthalten kann, $R^7$ Hydroxy, niederes Alkoxy oder $NR^8R^9$, $R^8$ und $R^9$ je Wasserstoff oder niederes Alkyl, n 0 oder 1 und m 1 bis 7 bedeuten, mit der Maßgabe, daß die als »nieder« bezeichneten Gruppen höchstens 7 Kohlenstoffatome aufweisen und mit den folgenden Maßgaben:

(i) wenn $R^3$ niederes Acyloxy oder Hydroxy bedeutet, dann bedeuten A Gruppe (a), X Wasserstoff, Halogen, Trifluormethyl, Äthyl oder Acetyl und, falls $R^1$ die Gruppe $-NH(CH_2)_mNR^8R^9$ bedeutet, dann bedeuten $R^8$ und $R^9$ je niederes Alkyl;
(ii) wenn A Gruppe (d) und $R^1$ Gruppe $-NH(CH_2)_mNR^8R^9$ bedeuten, dann bedeuten $R^8$ und $R^9$ je niederes Alkyl; und
(iii) wenn n 1 bedeutet, dann bedeuten entweder A Gruppe (b) und $R^1$ Wasserstoff, niederes Alkyl, Hydroxy, niederes Alkoxy, $NR^{41}R^{51}$ (worin $R^{41}$ und $R^{51}$ je Wasserstoff oder niederes Alkyl oder, zusammen mit dem Stickstoffatom, einen 5- bis 7gliedrigen Heterocyclus, welcher ein Sauerstoffatom enthalten kann, bedeuten), Chlor, Brom oder die Gruppe $-CH_2-CO-R^7$ oder es bedeuten A Gruppe (a) und $R^1$ Wasserstoff, niederes Alkyl, niederes Alkoxy, Chlor, Brom oder Gruppe $-CH_2-CO-R^7$;

und von pharmazeutisch akzeptablen Säureadditionssalzen davon, dadurch gekennzeichnet, daß man

a) eine Verbindung der allgemeinen Formel

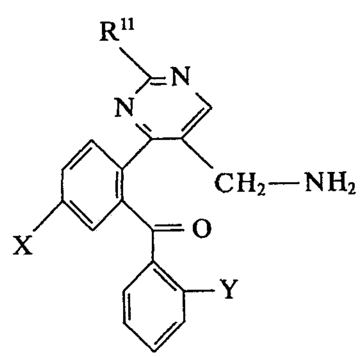

(II)

worin X und Y obige Bedeutung besitzen und $R^{11}$ Wasserstoff, niederes Alkyl oder $NR^8R^9$, worin $R^8$ und $R^9$ obige Bedeutung besitzen, bedeutet,
cyclisiert oder

b) eine Verbindung der allgemeinen Formel

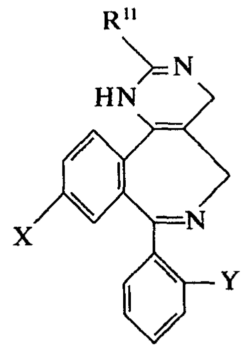

(III)

worin X, Y und $R^{11}$ obige Bedeutung besitzen,
dehydriert oder

c) eine Verbindung der allgemeinen Formel

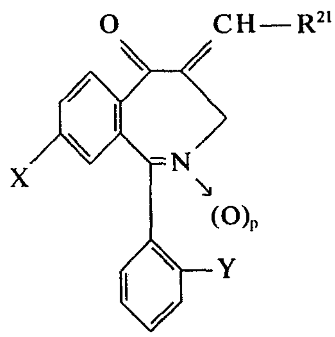

(IV)

worin X und Y obige Bedeutung besitzen und p 0 oder 1 und $R^{21}$ Di-(nieder)-alkylamino bedeuten, mit einer Verbindung der allgemeinen Formel

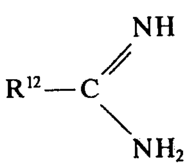

(V)

worin $R^{12}$ Wasserstoff, Mercapto, niederes Alkylmercapto, niederes Alkyl oder $NR^8R^9$, worin $R^8$ und $R^9$ obige Bedeutung besitzen, bedeutet,
umsetzt oder

d) eine Verbindung der allgemeinen Formel

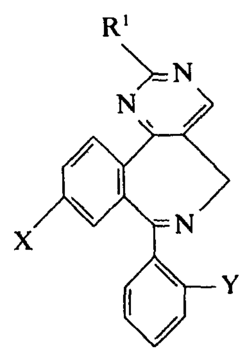

(Ia)

worin X, Y und $R^1$ obige Bedeutung besitzen,
reduziert oder

e) eine Verbindung der allgemeinen Formel

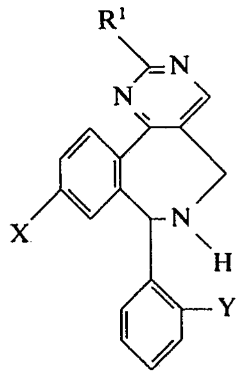

(Ib)

worin X, Y und $R^1$ obige Bedeutung besitzen, niederalkyliert oder

f) eine Verbindung der allgemeinen Formel

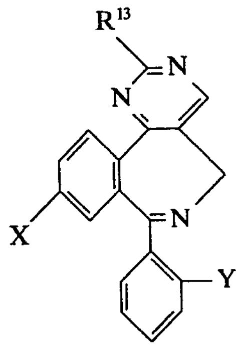

(Ic)

worin X und Y obige Bedeutung besitzen und $R^{13}$ Wasserstoff, niederes Alkyl, Hydroxy, niederes Alkoxy, $NR^{41}R^{51}$ (worin $R^{41}$ und $R^{51}$ je Wasserstoff oder niederes Alkyl oder, zusammen mit dem Stickstoffatom, einen 5- bis 7gliedrigen Heterocyclus, welcher ein Sauerstoffatom enthalten kann, bedeuten), Chlor, Brom oder die Gruppe $-CH_2-CO-R^7$ (worin $R^7$ obige Bedeutung besitzt) bedeutet, oxidiert oder

g) eine Verbindung der allgemeinen Formel

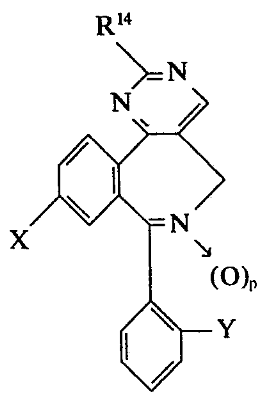

(Id)

worin X, Y und p obige Bedeutung besitzen und $R^{14}$ Mercapto oder Hydroxy bedeuten, niederalkyliert oder

h) eine Verbindung der allgemeinen Formel

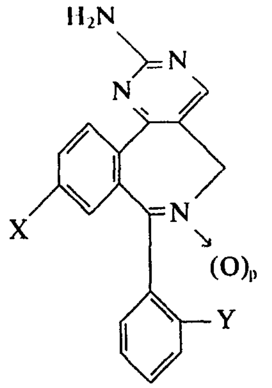

(Ie)

worin X, Y und p obige Bedeutung besitzen, in die entsprechende 2-Hydroxyverbindung überführt oder

i) eine Verbindung der allgemeinen Formel

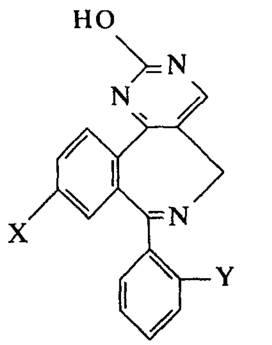

(If)

worin X und Y obige Bedeutung besitzen, in eine entsprechende 2-Chlor- oder 2-Bromverbindung überführt oder

k) eine Verbindung der allgemeinen Formel

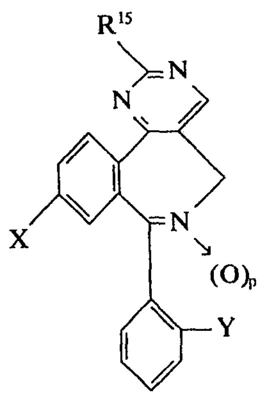

(Ig)

worin X, Y und p obige Bedeutung besitzen und $R^{15}$ Chlor oder Brom bedeutet, mit Schwefelwasserstoff, mit einem niederen Alkylmercaptan, mit einem niederen Alkanol, mit einer Verbindung der Formel $HNR^4R^5$, worin $R^4$ und $R^5$ obige Bedeutung besitzen, mit einer Verbindung der Formel $H_2N-(CH_2)_mNR^8R^9$, worin $R^8$, $R^9$ und m obige Bedeutung besitzen, oder mit dem Carbanion einer Verbindung der Formel

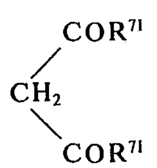 oder 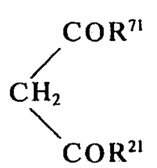

worin $R^{71}$ niederes Alkoxy bedeutet und $R^{21}$ obige Bedeutung besitzt, umsetzt oder

l) eine Verbindung der allgemeinen Formel

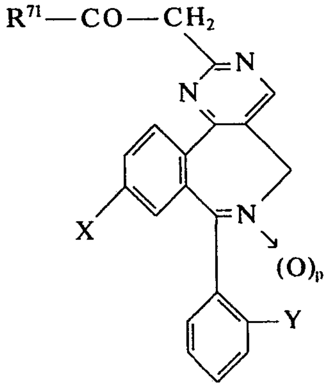

(Ih)

worin $R^{71}$, X, Y und p obige Bedeutung besitzen, in die entsprechende freie Säure oder in ein entsprechendes Amid, Niederalkylamid oder Di-niederalkylamid überführt oder

m) eine Verbindung der allgemeinen Formel

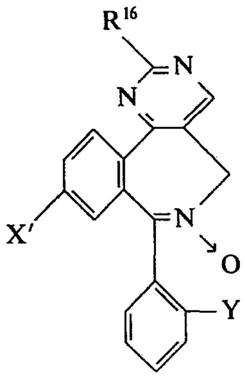

(Ii)

worin Y obige Bedeutung besitzt, $R^{16}$ Wasserstoff, Chlor, Brom, niederes Alkyl, die Gruppe $NR^4R^5$, die Gruppe $-CH_2-CO-R^7$, die Gruppe $-NH(CH_2)_mNR^{81}R^{91}$, Hydroxy, niederes Alkoxy, Mercapto oder niederes Alkylmercapto, X' Wasserstoff, Halogen, Trifluormethyl, Äthyl oder Acetyl und $R^{81}$ und $R^{91}$ je niederes Alkyl bedeuten und $R^4$, $R^5$ und $R^7$ obige Bedeutung besitzen, mit einem Niederacylierungsmittel zur Reaktion bringt oder

n) eine Verbindung der allgemeinen Formel

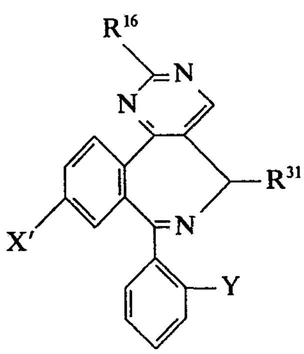

(Ik)

worin X', Y und $R^{16}$ obige Bedeutung besitzen und $R^{31}$ niederes Acyloxy bedeutet, hydrolysiert oder

o) eine Verbindung der allgemeinen Formel

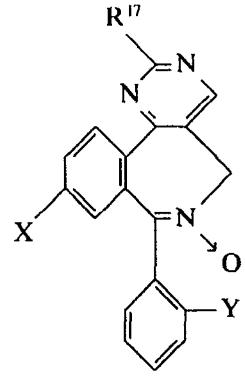

(II)

worin X und Y obige Bedeutung besitzen, $R^{17}$ Wasserstoff, niederes Alkyl, die Gruppe $NR^4R^5$, die Gruppe $-CH_2-CO-R^7$, die Gruppe $NH-(CH_2)_mNR^8R^9$, Hydroxy, niederes Alkoxy, Mercapto oder niederes Alkylmercapto bedeutet und $R^4$, $R^5$, $R^7$, $R^8$ und $R^9$ obige Bedeutung besitzen, deoxygeniert oder

p) aus einer Verbindung der allgemeinen Formel

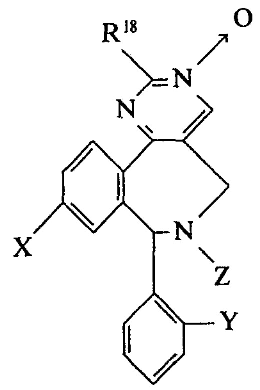

(III')

worin X und Y obige Bedeutung besitzen und $R^{18}$ Wasserstoff, niederes Alkyl, niederes Alkoxy, Chlor, Brom oder die Gruppe $-CH_2-CO-R^7$ (worin $R^7$ obige Bedeutung besitzt) und Z Wasserstoff oder eine leicht abspaltbare Acylgruppe bedeuten, die Elemente von $H-Z$ entfernt oder

q) eine Verbindung der Formel I in ein pharmazeutisch akzeptables Säureadditionssalz überführt.

2. Verfahren nach Anspruch 1, dadurch gekennzeichnet, daß man Verbindungen der Formel I, worin A die Gruppe (b), $R^1$ Amino, n 0, X Wasserstoff, Halogen mit einer Atomnummer von höchstens 35 oder Trifluormethyl und Y Wasserstoff oder Halogen mit einer Atomnummer von höchstens 35 bedeuten, herstellt und daß man diese Verbindungen gemäß Verfahrensvariante c), worin X und Y obige Bedeutung besitzen und $R^{21}$ Dimethylamino, p 1 und $R^{12}$ Amino bedeuten, herstellt.

3. Verfahren nach Anspruch 1, dadurch gekennzeichnet, daß man Verbindungen der Formel I, worin $R^1$ Wasserstoff, Chlor, Brom, niederes Alkyl, die Gruppe $NR^8R^9$ (worin $R^8$ und $R^9$ je Wasserstoff oder niederes Alkyl sind), die Gruppe $-CH_2-CO-R^7$ (worin $R^7$ niederes Alkoxy ist), Di-(nieder)-alkylamino-(nieder)-alkylamino, Hydroxy, niederes Alkoxy, Mercapto oder niederes Alkylmercapto bedeutet, mit den folgenden Maßgaben:

(i) wenn A Gruppe (b), (c) oder (d) ist oder wenn A Gruppe (a) und $R^3$ niederes Acyloxy oder Hydroxy sind, dann bedeutet $R^1$ Wasserstoff, niederes Alkyl oder $NR^8R^9$; und

(ii) wenn n 1 ist, dann bedeutet A die Gruppe (b);

oder pharmazeutisch akzeptable Säureadditionssalze davon herstellt und daß man nach Verfahrensvarianten a), b), i) oder q) arbeitet; oder daß man nach Verfahrensvariante c) arbeitet, wobei $R^{21}$ Dimethylamino und $R^{12}$ Wasserstoff, Mercapto, niederes Alkyl oder $NR^8R^9$ bedeuten, mit der Maßgabe, daß p 0 ist, wenn $R^{12}$ Mercapto bedeutet oder wenn $R^{12}$ Amino, X Wasserstoff, Halogen mit einer Atomnummer von höchstens 35 oder Trifluormethyl und Y Wasserstoff oder Halogen mit einer Atomnummer von höchstens 35 bedeuten; oder daß man nach Verfahrensvariante d) oder e) arbeitet; wobei $R^1$ Wasserstoff, niederes Alkyl oder $NR^8R^9$ bedeutet; oder daß man nach Verfahrensvariante f) arbeitet, wobei $R^{13}$ Wasserstoff, niederes Alkyl oder $NR^8R^9$ bedeutet; oder daß man nach Verfahrensvariante g) oder h) arbeitet, wobei p 0 ist; oder daß man nach Verfahrensvariante k) arbeitet, wobei eine Verbindung der Formel Ig, worin p 0 ist, in eine entsprechende Verbindung der Formel I, worin $R^1$ niederes Alkoxy, niederes Alkylamino, Di-niederalkylamino, Di-(nieder)-alkylamino-(nieder)-alkylamino oder die Gruppe $-CH_2COR^{71}$ bedeutet, übergeführt wird; oder daß man nach der Verfahrensvariante m) oder n) arbeitet, wobei $R^{16}$ Wasserstoff, niederes Alkyl oder $NR^8R^9$ bedeutet.

4. Verfahren nach Anspruch 1, dadurch gekennzeichnet, daß man Verbindungen der Formel I herstellt, worin A Gruppe (a) und n 0 bedeuten.

5. Verfahren nach Anspruch 4, dadurch gekennzeichnet, daß man Verbindungen der Formel I herstellt, worin $R^3$ Wasserstoff und $R^1$ Wasserstoff, niederes Alkyl, die Gruppe $NR^4R^5$ (worin $R^4$ und $R^5$ je Wasserstoff oder niederes Alkyl sind), Hydroxy, Chlor, Brom, die Gruppe $-NH(CH_2)_mNR^8R^9$ (worin $R^8$ und $R^9$ je niederes Alkyl sind) oder die Gruppe $-CH_2-CO-R^7$ bedeuten.

6. Verfahren nach Anspruch 5, dadurch gekennzeichnet, daß man Verbindungen der Formel I herstellt, worin $R^1$ Wasserstoff, Amino oder niederes Alkyl bedeutet.

7. Verfahren nach Anspruch 4, dadurch gekennzeichnet, daß man Verbindungen der Formel I herstellt, worin $R^3$ Hydroxy und $R^1$ Wasserstoff, niederes Alkyl oder die Gruppe $NR^4R^5$ (worin $R^4$ und $R^5$ je Wasserstoff oder niederes Alkyl sind) bedeuten.

8. Verfahren nach einem der Ansprüche 4—7, dadurch gekennzeichnet, daß man Verbindungen der Formel I herstellt, worin X Halogen bedeutet.

9. Verfahren nach Anspruch 8, dadurch gekennzeichnet, daß man Verbindungen der Formel I herstellt, worin X Chlor bedeutet.

10. Verfahren nach einem der Ansprüche 4—9, dadurch gekennzeichnet, daß man Verbindungen der Formel I herstellt, worin Y Wasserstoff, Chlor oder Fluor bedeutet.

11. Verfahren nach Anspruch 1, dadurch gekennzeichnet, daß man 9-Chlor-7-(2-chlorphenyl)-5H-pyrimido[5,4-d] [2]benzazepin herstellt.

12. Verfahren nach Anspruch 1, dadurch gekennzeichnet, daß man 9-Chlor-7-(2-fluorphenyl)-5H-pyrimido[5,4-d] [2]benzazepin herstellt.

13. Verfahren nach Anspruch 1, dadurch gekennzeichnet, daß man 9-Chlor-7-(2-chlorphenyl)-2-methyl-5H-pyrimido[5,4-d] [2]benzazepin herstellt.

14. Verfahren nach Anspruch 1, dadurch gekennzeichnet, daß man 9-Chlor-7-(2-fluorphenyl)-5H-pyrimido[5,4-d] [2]benzazepin-2-ol herstellt.

15. Verfahren nach Anspruch 1, dadurch gekennzeichnet, daß man 9-Chlor-N,N-dimethyl-7-(2-fluorphenyl)-5H-pyrimido[5,4-d] [2]benzazepin-2-amin herstellt.

**Claims for the Contracting States: BE, CH, DE, FR, IT, LI, LU, NL, SE**

1. Pyrimido-2-benzazepines of the general formula

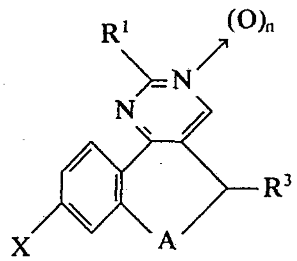

(I)

wherein A signifies one of the groups

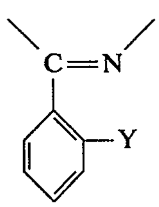

(a)

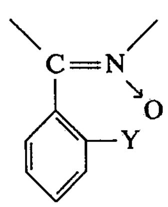

(b)

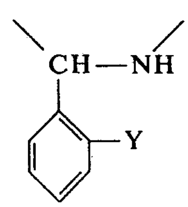

(c)

and

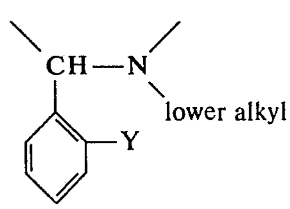

(d)

$R^1$ signifies hydrogen, chlorine, bromine, lower alkyl, the group $NR^4R^5$, the group $-CH_2-CO-R^7$, the group $-NH(CH_2)_mNR^8R^9$, hydroxy, lower alkoxy, mercapto or lower alkylmercapto, $R^3$ signifies hydrogen, lower acyloxy or hydroxy, X signifies hydrogen, halogen, trifluoromethyl, ethyl, $\alpha$-hydroxyethyl or acetyl, Y signifies hydrogen or halogen, $R^4$ and $R^5$ each signify hydrogen or lower alkyl or, together with the nitrogen atom, signify a 5- to 7-membered heterocycle which can contain an oxygen or sulphur atom or the group N lower alkyl, $R^7$ signifies hydroxy, lower alkoxy or $NR^8R^9$, $R^8$ and $R^9$

each signify hydrogen or lower alkyl, n signifies 0 or 1 and m signifies 1 to 7, with the proviso that the groups denoted as »lower« have at most 7 carbon atoms and with the following provisos:

(i) when $R^3$ signifies lower acyloxy or hydroxy, then A signifies group (a), X signifies hydrogen, halogen, trifluoromethyl, ethyl or acetyl and, if $R^1$ signifies the group $-NH(CH_2)_mNR^8R^9$, then $R^8$ and $R^9$ each signify lower alkyl;
(ii) when A signifies group (d) and $R^1$ signifies group $-NH(CH_2)_mNR^8R^9$, then $R^8$ and $R^9$ each signify lower alkyl; and
(iii) when n signifies 1, then either A signifies group (b) and $R^1$ signifies hydrogen, lower alkyl, hydroxy, lower alkoxy, $NR^{41}R^{51}$ (wherein $R^{41}$ and $R^{51}$ each signify hydrogen or lower alkyl or, together with the nitrogen atom, signify a 5- to 7-membered heterocycle wich can contain an oxygen atom), chlorine, bromine or the group $-CH_2-CO-R^7$ or A signifies group (a) and $R^1$ signifies hydrogen, lower alkyl, lower alkoxy, chlorine, bromine or group $-CH_2-CO-R^7$;

and pharmaceutically acceptable acid addition salts thereof.

2. Compounds according to claim 1, wherein A signifies group (b), $R^1$ signifies amino, n signifes 0, X signifies hydrogen, halogen having an atomic number of at most 35 or trifluoromethyl and Y signifies hydrogen or halogen having an atomic number of at most 35.

3. Compounds according to claim 1, wherein $R^1$ signifies hydrogen, chlorine, bromine, lower alkyl, the group $NR^8R^9$ (wherein $R^8$ and $R^9$ each are hydrogen or lower alkyl), the group $-CH_2-CO-R^7$ (wherein $R^7$ is lower alkoxy), di-(lower)-alkylamino-(lower)-alkylamino, hydroxy, lower alkoxy, mercapto or lower alkylmercapto, with the following provisos:

(i) when A is group (b), (c) or (d) or when A is group (a) and $R^3$ is lower acyloxy or hydroxy, then $R^1$ signifies hydrogen, lower alkyl or $NR^8R^9$;
(ii) when n is 1, then A signifies group (b); and
(iii) when A is group (b), $R^1$ is amino and Y is hydrogen or halogen having an atomic number of at most 35, then X does not signify hydrogen, halogen having an atomic number of at most 35 or trifluoromethyl.

4. Compounds according to claim 1, wherein A signifies the group (a) and n signifies 0.

5. Compounds according to claim 4, wherein $R^3$ signifies hydrogen and $R^1$ signifies hydrogen, lower alkyl, the group $NR^4R^5$ (wherein $R^4$ and $R^5$ each are hydrogen or lower alkyl), hydroxy, chlorine, bromine, the group $-NH(CH_2)_mNR^8R^9$ (wherein $R^8$ and $R^9$ each are lower alkyl) or the group $-CH_2-CO-R^7$.

6. Compounds according to claim 5, wherein $R^1$ signifies hydrogen, amino or lower alkyl.

7. Compounds according to claim 4, wherein $R^3$ signifies hydroxy and $R^1$ signifies hydrogen, lower alkyl or the group $NR^4R^5$ (wherein $R^4$ and $R^5$ each are hydrogen or lower alkyl).

8. Compounds according to any one of claims 4—7, wherein X signifies halogen.

9. Compounds according to claim 8, wherein X signifies chlorine.

10. Compounds according to any one of claims 4—9, wherein Y signifies hydrogen, chlorine or fluorine.

11. 9-Chloro-7-(2-chlorophenyl)-5H-pyrimido[5,4-d] [2]benzazepine.

12. 9-Chloro-7-(2-fluorophenyl)-5H-pyrimido[5,4-d] [2]benzazepine.

13. 9-Chloro-7-(2-chlorophenyl)-2-methyl-5H-pyrimido[5,4-d] [2]benzazepine.

14. 9-Chloro-7-(2-fluorophenyl)-5H-pyrimido[5,4-d] [2]benzazepin-2-ol.

15. 9-Chloro-N,N-dimethyl-7-(2-fluorophenyl)-5H-pyrimido[5,4-d] [2]benzazepin-2-amine.

16. Compounds of the general formula

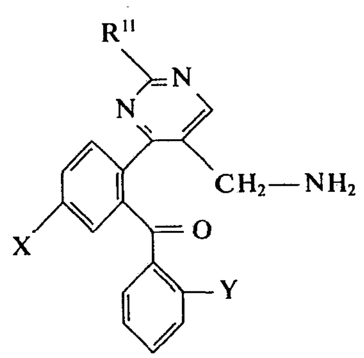

(II)

and

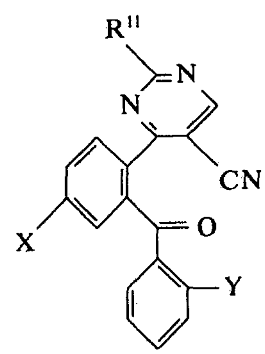

(XII)

wherein X and Y have the significance given in claim 1 and $R^{11}$ signifies hydrogen, lower alkyl or $NR^8R^9$ in which $R^8$ and $R^9$ have the significance given in claim 1.

17. Compounds according to claim 16, wherein X and Y each signify chlorine and $R^{11}$ signifies hydrogen.

18. Compounds according to any one of claims 1—10 and 12—15 as pharmaceutically active substances.

19. Compounds according to any one of claims 1—10 and 12—15 as anxiolytics and sedatives.

20. 9-Chloro-7-(2-chlorophenyl)-5H-pyrimido[5,4-d] [2]benzazepine as a pharmaceutically active substance.

21. 9-Chloro-7-(2-chlorophenyl)-5H-pyrimido[5,4-d] [2]benzazepine as an anxiolytic and sedative.

22. A process for the manufacture of compounds according to any one of claims 1—15, characterized by

a) cyclizing a compound of the general formula

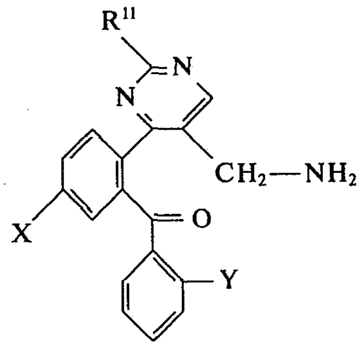

(II)

wherein X and Y have the significance given in claim 1 and $R^{11}$ signifies hydrogen, lower alkyl or $NR^8R^9$ in which $R^8$ and $R^9$ have the significance given in claim 1,

or

b) dehydrogenating a compound of the general formula

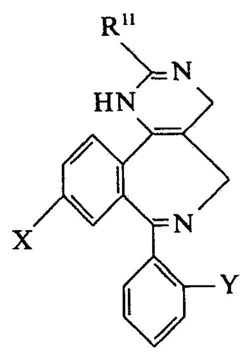

(III)

wherein X and Y have the significance given in claim 1 and $R^{11}$ has the above significance,

or

c) reacting a compound of the general formula

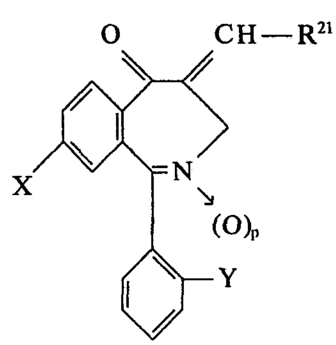

(IV)

wherein X and Y have the significance given in claim 1 and p signifies 0 or 1 and $R^{21}$ signifies di-lower alkylamino,

with a compound of the general formula

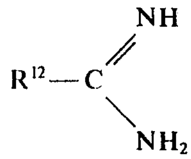

(V)

wherein $R^{12}$ signifies hydrogen, mercapto, lower alkylmercapto, lower alkyl or $NR^8R^9$ in which $R^8$ and $R^9$ have the significance given in claim 1,

or

d) reducing a compound of the general formula

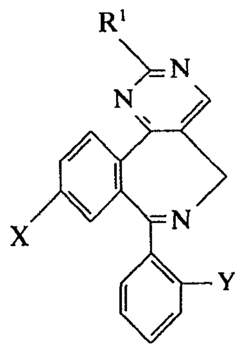

(Ia)

wherein X, Y and $R^1$ have the significance given in claim 1,

or

e) lower alkylating a compound of the general formula

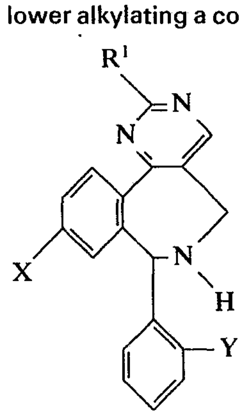

(Ib)

wherein X, Y and $R^1$ have the significance given in claim 1,

or

f) oxidizing a compound of the general formula

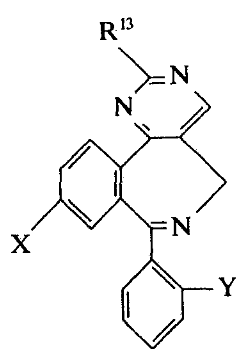

(Ic)

wherein X and Y have the significance given in claim 1 and $R^{13}$ signifies hydrogen, lower alkyl, hydroxy, lower alkoxy, $NR^{41}R^{51}$ (wherein $R^{41}$ and $R^{51}$ each are hydrogen or lower alkyl or, together with the nitrogen atom, are a 5- to 7-membered heterocycle which can contain an oxygen atom), chlorine, bromine or the group $-CH_2-CO-R^7$ (wherein $R^7$ has the significance given in claim 1),

or

g) lower alkylating a compound of the general formula

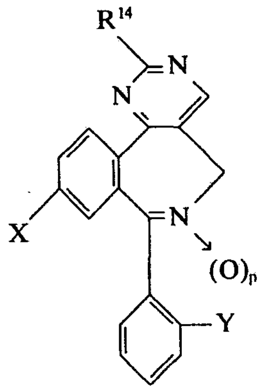

(Id)

wherein X and Y have the significance given in claim 1 and p has the above significance and $R^{14}$ signifies mercapto or hydroxy,
or

h) converting a compound of the general formula

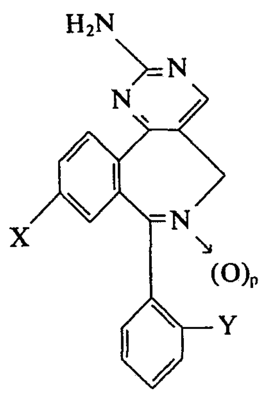

(Ie)

wherein X and Y have the significance given in claim 1 and p has the above significance, into the corresponding 2-hydroxy compound, or

i) converting a compound of the general formula

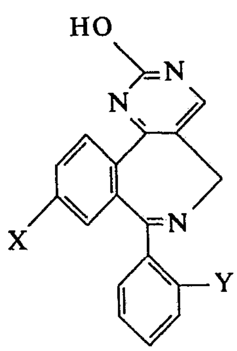

(If)

wherein X and Y have the significance given in claim 1, into a corresponding 2-chloro or 2-bromo compound, or

k) treating a compound of the general formula

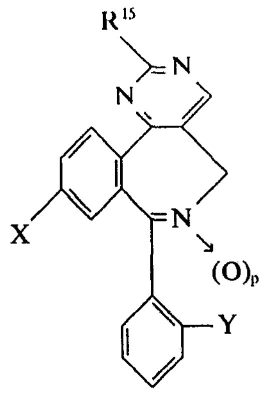

(Ig)

wherein X and Y have the significance given in claim 1 and p has the above significance and $R^{15}$ signifies chlorine or bromine,
with hydrogen sulphide, with a lower alkylmercaptan, with a lower alkanol, with a compound of the formula $HNR^4R^5$, wherein $R^4$ and $R^5$ have the significance given in claim 1, with a compound of the formula $H_2N-(CH_2)_mNR^8R^9$, wherein $R^8$, $R^9$ and m have the significance given in claim 1, or with the carbanion of a compound of the formula

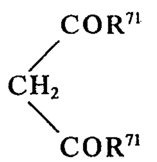

or

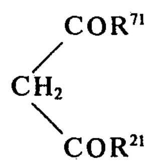

wherein $R^{71}$ signifies lower alkoxy and $R^{21}$ has the above significance,
or

l) converting a compound of the general formula

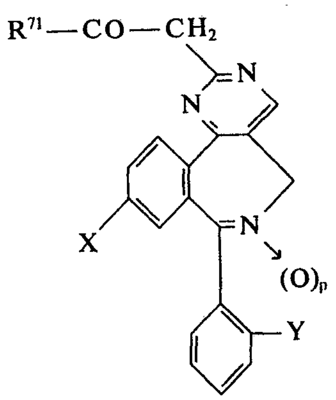

(Ih)

wherein X and Y have the significance given in claim 1 and $R^{71}$ and p have the above significance, into the corresponding free acid or into a corresponding amide, lower alkyl amide or di-lower-alkyl amide, or

m) reacting a compound of the general formula

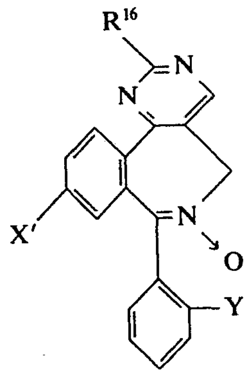

(Ii)

wherein Y has the significance given in claim 1, $R^{16}$ signifies hydrogen, chlorine, bromine, lower alkyl, the group $NR^4R^5$, the group $-CH_2-CO-R^7$, the group $NH(CH_2)_mNR^{81}R^{91}$, hydroxy, lower alkoxy, mercapto or lower alkylmercapto, X' signifies hydrogen, halogen, trifluoromethyl, ehtyl or acetyl and $R^{81}$ and $R^{91}$ each signify lower alkyl and $R^4$, $R^5$ and $R^7$ have the significance given in claim 1,
with a lower acylating agent, or

n) hydrolyzing a compound of the general formula

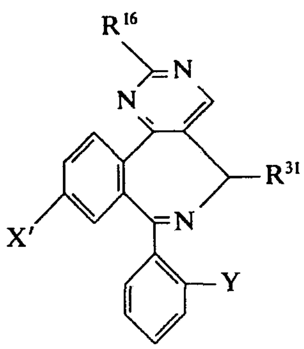

(Ik)

wherein Y has the significance given in claim 1 and X' and $R^{16}$ have the above significance and $R^{31}$ signifies lower acyloxy,
or

o) deoxygenating a compound of the general formula

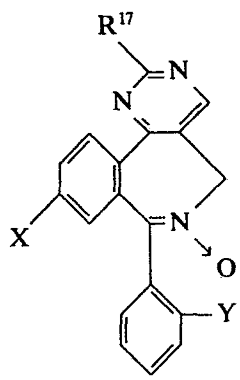

(Il)

wherein X and Y have the significance given in claim 1, $R^{17}$ signifies hydrogen, lower alkyl, the group $NR^4R^5$, the group $-CH_2-CO-R^7$, the group $NH-(CH_2)_m-NR^8R^9$, hydroxy, lower alkoxy, mercapto or lower alkylmercapto and $R^4$, $R^5$, $R^7$, $R^8$ and $R^9$ have the significance given in claim 1,
or

p) removing the elements of H—Z from a compound of the general formula

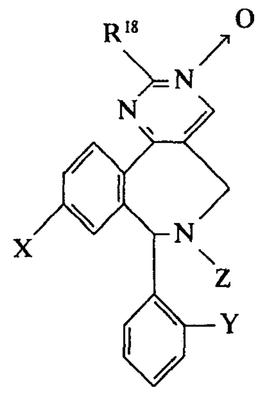

(III')

wherein X and Y have the significance given in claim 1 and $R^{18}$ signifies hydrogen, lower alkyl, lower alkoxy, chlorine, bromine or the group $-CH_2-CO-R^7$ (wherein $R^7$ has the significance given in claim 1) and Z signifies hydrogen or a readily cleavable acyl group,
or

q) converting a compound of formula I defined in claim 1 into a pharmaceutically acceptable acid addition salt.

23. A process according to claim 22, wherein 9-chloro-7-(2-chlorophenyl)-5H-pyrimido[5,4-d] [2]benzazepine is manufactured.
24. A medicament containing a compound in accordance with any one of claims 1—10 and 12—15.
25. An anxiolytic and sedative containing a compound in accordance with any one of claims 1—10 and 12—15.
26. A medicament containing 9-Chloro-7-(2-chlorophenyl)-5H-pyrimido[5,4-d] [2]benzazepine or a pharmaceutically acceptable acid addition salt thereof.
27. An anxiolytic and sedative containg 9-chloro-7-(2-chlorophenyl)-5H-pyrimido[5,4-d] [2]benzazepine or a pharmaceutically acceptable acid addition salt thereof.

**Claims for the Contracting State: AT**

1. A process for the manufacture of pyrimido-2-benzazepines of the general formula

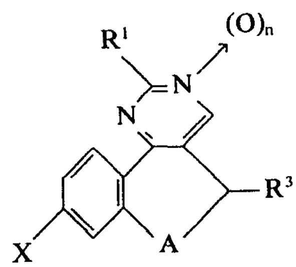

(I)

wherein A signifies one of the groups

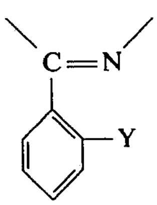

(a)

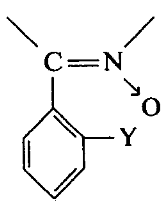

(b)

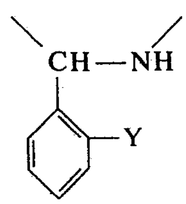

(c)

and

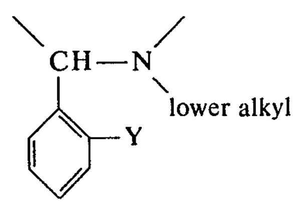

(d)

$R^1$ signifies hydrogen, chlorine, bromine, lower alkyl, the group $NR^4R^5$, the group $-CH_2-CO-R^7$, the group $-NH(CH_2)_mNR^8R^9$, hydroxy, lower alkoxy, mercapto or lower alkylmercapto, $R^3$ signifies hydrogen, lower acyloxy or hydroxy, X signifies hydrogen, halogen, trifluoromethyl, ethyl, $\alpha$-hydroxyethyl or acetyl, Y signifies hydrogen or halogen, $R^4$ and $R^5$ each signify hydrogen or lower alkyl or, togehter with the nitrogen atom, signify a 5- to 7-membered heterocycle which can contain an oxygen or sulphur atom or the group >N-lower alkyl, $R^7$ signifies hydroxy, lower alkoxy or $NR^8R^9$, $R^8$ and $R^9$

each signify hydrogen or lower alkyl, n signifies 0 or 1 and m signifes 1 to 7, with the proviso that the groups denoted as »lower« have at most 7 carbon atoms and with the following provisos:

(i) when $R^3$ signifies lower acyloxy or hydroxy, then A signifies group (a), X signifies hydrogen, halogen, trifluoromethyl, ethyl or acetyl and, if $R^1$ signifies the group $-NH(CH_2)_mNR^8R^9$, then $R^8$ and $R^9$ each signify lower alkyl;
(ii) when A signifies group (d) and $R^1$ signifies group $-NH(CH_2)_mNR^8R^9$, then $R^8$ and $R^9$ each signify lower alkyl; and
(iii) when n signifies 1, then either A signifies group (b) and $R^1$ signifies hydrogen, lower alkyl, hydroxy, lower alkoxy, $NR^{41}R^{51}$ (wherein $R^{41}$ and $R^{51}$ each signify hydrogen or lower alkyl or, together with the nitrogen atom, signify a 5- to 7-membered heterocycle which can contain an oxygen atom), chlorine, bromine or the group $-CH_2-CO-R^7$ or A signifies group (a) and $R^1$ signifies hydrogen, lower alkyl, lower alkoxy, chlorine, bromine or group $-CH_2-CO-R^7$;

and of pharmaceutically acceptable acid addition salts thereof, characterized by

a) cyclizing a compound of the general formula

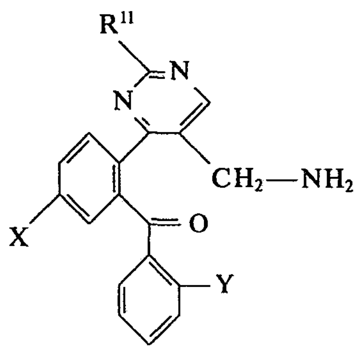

(II)

wherein X and Y have the above significance and $R^{11}$ signifies hydrogen, lower alkyl or $NR^8R^9$ in which $R^8$ and $R^9$ have the above significance,
or

b) dehydrogenating a compound of the general formula

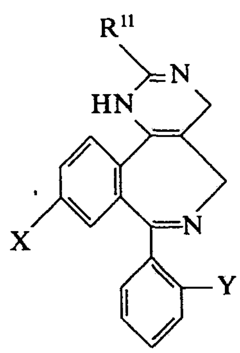

(III)

wherein X, and Y and $R^{11}$ have the above significance,
or

c) reacting a compound of the general formula

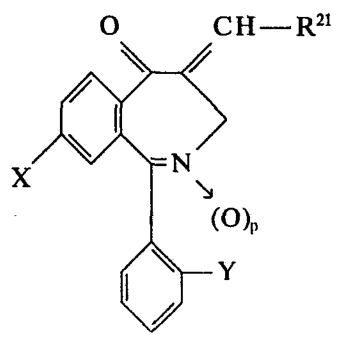

(IV)

wherein X and Y have the above significance and p signifies 0 or 1 and $R^{21}$ signifies di-lower alkylamino,
with a compound of the general formula

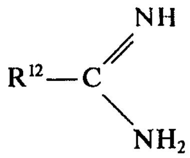

(V)

wherein $R^{12}$ signifies hydrogen, mercapto, lower alkylmercapto, lower alkyl or $NR^8R^9$ in which $R^8$ and $R^9$ have the above significance,
or

d) reducing a compound of the general formula

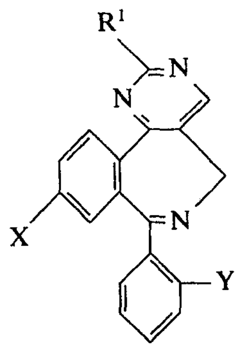

(Ia)

wherein X, Y and $R^1$ have the above significance,
or

e) lower alkylating a compound of the general formula

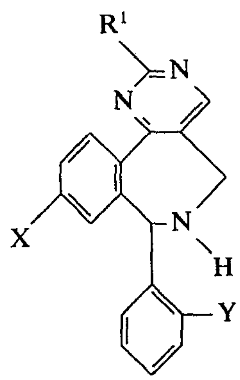

(Ib)

wherein X, Y and $R^1$ have the above significance,
or

f) oxidizing a compound of the general formula

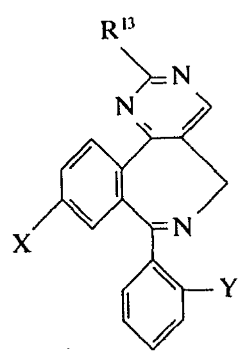

(Ic)

wherein X and Y have the above significance and $R^{13}$ signifies hydrogen, lower alkyl, hydroxy, lower alkoxy, $NR^{41}R^{51}$ (wherein $R^{41}$ and $R^{51}$ each are hydrogen or lower alkyl or, together with the

nitrogen atom, are a 5- to 7-membered heterocycle which can contain an oxygen atom), chlorine, bromine or the group $-CH_2-CO-R^7$ (wherein $R^7$ has the above significance),

or

g) lower alkylating a compound of the general formula

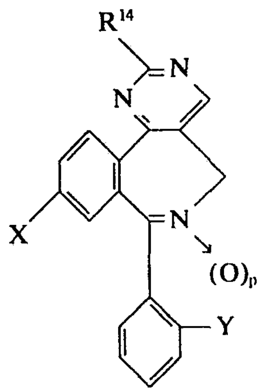

(Id)

wherein X, and Y and p have the above significance and $R^{14}$ signifies mercapto or hydroxy,

or

h) converting a compound of the general formula

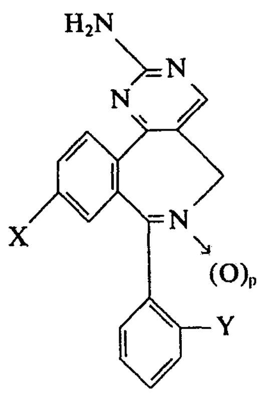

(Ie)

wherein X, and Y and p have the above significance, into the corresponding 2-hydroxy compound, or

i) converting a compound of the general formula

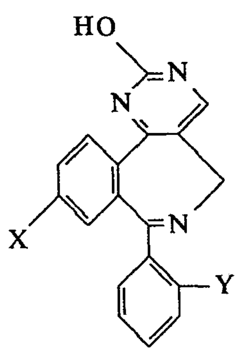

(If)

wherein X and Y have the above significance, into a corresponding 2-chloro or 2-bromo compound, or

k) treating a compound of the general formula

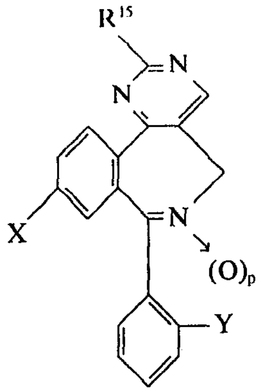

(Ig)

wherein X, Y and p have the above significance and $R^{15}$ signifies chlorine or bromine, with hydrogen sulphide, with a lower alkylmercaptan, with a lower alkanol, with a compound of the formula $HNR^4R^5$, wherein $R^4$ and $R^5$ have the above significance, with a compound of the formula $H_2N-(CH_2)_mNR^8R^9$, wherein $R^8$, $R^9$ and m have the above significance, or with the carbanion of a compound of the formula

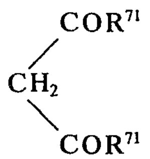

or

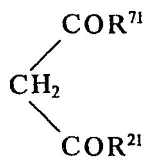

wherein $R^{71}$ signifies lower alkoxy and $R^{21}$ has the above significance,
or

l) converting a compound of the general formula

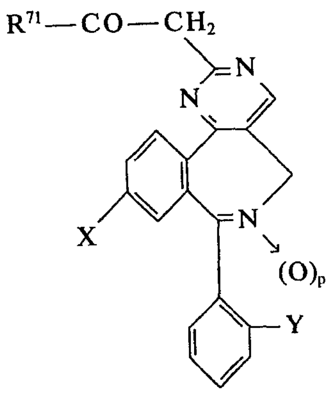

(Ih)

wherein $R^{71}$, X, Y and p have the above significance,
into the corresponding free acid or into a corresponding amide, lower alkyl amide or di-lower-alkyl amide, or

m) reacting a compound of the general formula

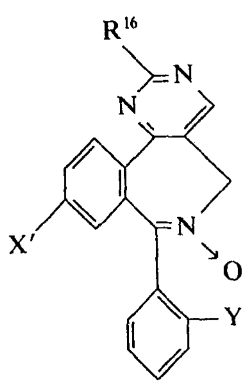

(Ii)

wherein Y has the above significance, $R^{16}$ signifies hydrogen, chlorine, bromine, lower alkyl, the group $NR^4R^5$, the group $-CH_2-CO-R^7$, the group $-NH(CH_2)_mNR^{81}R^{91}$, hydroxy, lower alkoxy, mercapto or lower alkyl mercapto, X' signifies hydrogen, halogen, trifluoromethyl, ethyl or acetyl and $R^{81}$ and $R^{91}$ each signify lower alkyl and $R^4$, $R^5$ and $R^7$ have the above significance, with a lower acylating agent, or

n) hydrolyzing a compound of the general formula

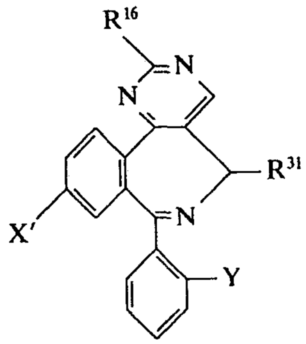

(Ik)

wherein X', Y and $R^{16}$ have the above significance and $R^{31}$ signifies lower acyloxy, or

o) deoxygenating a compound of the general formula

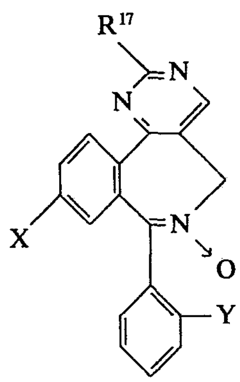

(II)

wherein X and Y have the above significance, $R^{17}$ signifies hydrogen, lower alkyl, the group $NR^4R^5$, the group $-CH_2-CO-R^7$, the group $-NH-(CH_2)_m-NR^8R^9$, hydroxy, lower alkoxy, mercapto or lower alkylmercapto and $R^4$, $R^5$, $R^7$, $R^8$ and $R^9$ have the above significance, or

p) removing the elements of H—Z from a compound of the general formula

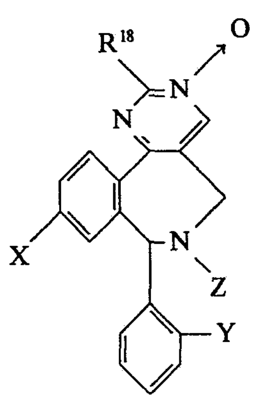

(III')

wherein X and Y have the above significance and $R^{18}$ signifies hydrogen, lower alkyl, lower alkoxy, chlorine, bromine or the group $-CH_2-CO-R^7$ (wherein $R^7$ has the above significance) and Z signifies hydrogen or a readily cleavable acyl group, or

q) converting a compound of formula I into a pharmaceutically acceptable acid addition salt.

2. A process according to claim 1, characterized in that compounds of formula I in which A signifies group (b), $R^1$ signifies amino, n signifies O, X signifies hydrogen, halogen having an atomic number of at most 35 or trifluoromethyl and Y signifies hydrogen or halogen having an atomic number of at most 35 are manufactured and in that these compounds are manufactured in accordance with process variant c) in which X and Y have the above significance and $R^{21}$ signifies dimethylamino, p signifies 1 and $R^{12}$ signifies amino.

3. A process according to claim 1, characterized in that compounds of formula I wherein $R^1$ signifies hydrogen, chlorine, bromine, lower alkyl, the group $NR^8R^9$ (wherein $R^8$ and $R^9$ each are hydrogen or lower alkyl), the group $-CH_2-CO-R^7$ (wherein $R^7$ is lower alkoxy), di-(lower)-alkylamino-(lower)-alkylamino, hydroxy, lower alkoxy, mercapto or lower alkylmercapto, with the following provisos:

(i) when A is group (b), (c) or (d) or when A is group (a) and $R^3$ is lower acyloxy or hydroxy, then $R^1$ signifies hydrogen, lower alkyl or $NR^8R^9$; and

(ii) when n is 1, then A signifies group (b);

or pharmaceutically acceptable acid addition salts thereof are manufactured according to process variants a), b), i) or q); or according to process variant c) in which $R^{21}$ signifies dimethylamino and $R^{12}$ signifies hydrogen, mercapto, lower alkyl or $NR^8R^9$, with the proviso that p is 0 when $R^{12}$ signifies mercapto or when $R^{12}$ signifies amino, X signifies hydrogen, halogen having an atomic number of at most 35 or trifluoromethyl and Y signifies hydrogen or halogen having an atomic number of at most 35; or according to process variant d) or e); in which $R^1$ signifies hydrogen, lower alkyl or $NR^8R^9$; or according to process variant f) in which $R^{13}$ signifies hydrogen, lower alkyl or $NR^8R^9$; or according to process variant g) or h) in which p is 0; or according to process variant k) in which a compound of formula Ig in which p is 0 is converted into a corresponding compound of formula I in which $R^1$ is lower alkoxy, lower alkylamino, di-lower alkylamino, di-(lower)-alkylamino-(lower)-alkylamino or the group $-CH_2CO^{71}$; or according to process variant m) or n) in which $R^{16}$ signifies hydrogen, lower alkyl or $NR^8R^9$.

4. A process according to claim 1, characterized in that compounds of formula I in which A signifies group (a) and n signifies 0 are manufactured.

5. A process according to claim 4, characterized in that compounds of formula I in which $R^3$ signifies hydrogen and $R^1$ signifies hydrogen, lower alkyl, the group $NR^4R^5$ (wherein $R^4$ and $R^5$ each are hydrogen or lower alkyl), hydroxy, chlorine, bromine, the group $-NH(CH_2)_mNR^8R^9$ (wherein $R^8$ and $R^9$ each are lower alkyl) or the group $-CH_2-CO-R^7$ are manufactured.

6. A process according to claim 5, characterized in that compounds of formula I in which $R^1$ signifies hydrogen, amino or lower alkyl are manufactured.

7. A process according to claim 4, characterized in that compounds of formula I in which $R^3$ signifies hydroxy and $R^1$ signifies hydrogen, lower alkyl or the group $NR^4R^5$ (wherein $R^4$ and $R^5$ each are hydrogen or lower alkyl) are manufactured.

8. A process according to any one of claims 4—7, characterized in that compounds of formula I in which X signifies halogen are manufactured.

9. A process according to claim 8, characterized in that compounds of formula I in which X signifies chlorine are manufactured.

10. A process according to any one of claims 4—9, characterized in that compounds of formula I in which Y signifies hydrogen, chlorine or fluorine are manufactured.

11. A process according to claim 1, characterized in that 9-chloro-7-(2-chlorophenyl)-5H-pyrimido[5,4-d] [2]benzazepine is manufactured.

12. A process according to claim 1, characterized in that 9-chloro-7-(2-fluorophenyl)-5H-pyrimido[5,4-d] [2]benzazepine is manufactured.

13. A process according to claim 1, characterized in that 9-chloro-7-(2-chlorophenyl)-2-methyl-5H-pyrimido[5,4-d] [2]benzazepine is manufactured.

14. A process according to claim 1, characterized in that 9-chloro-7-(2-fluorophenyl)-5H-pyrimido[5,4-d] [2]benzazepin-2-ol is manufactured.

15. A process according to claim 1, characterized in that 9-chloro-N,N-dimethyl-7-(2-fluorophenyl)-5H-pyrimido[5,4-d] [2]benzazepin-2-amine is manufactured.

**Revendications pour les Etats contractants: BE, CH, DE, FR, IT, LI, LU, NL, SE**

1. Pyrimido-2-benzazépines de formule générale

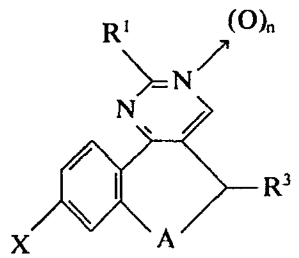

(I)

dans laquelle A représente l'un des groupes

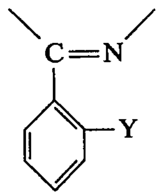 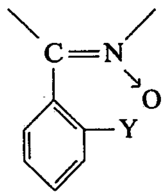 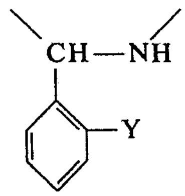 et 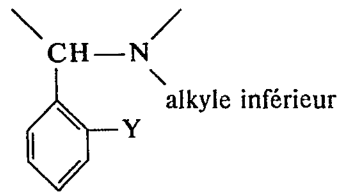

(a) (b) (c) (d)

$R^1$ représente l'hydrogène, le chlore, le brome, un groupe alkyle inférieur, le groupe $NR^4R^5$, le groupe $-CH_2-CO-R^7$, le groupe $-NH(CH_2)_mNR^8R^9$, un groupe hydroxy, alcoxy, inférieur, mercapto ou alkylmercapto inférieur, $R^3$ représente l'hydrogène, un groupe acyloxy inférieur ou hydroxy, X représente l'hydrogène, un halogène, un groupe trifluorométhyle, éthyle, alpha-hydroxyéthyle ou acétyle, Y représente l'hydrogène ou un halogène, $R^4$ et $R^5$ représentent chacun l'hydrogène ou un groupe alkyle inférieur ou bien forment ensemble et avec l'atome d'azote un hétérocycle de 5 à 7 chaînons qui peut contenir un atome d'oxygène ou de soufre ou le groupe $>$N-alkyle inférieur, $R^7$ représente un groupe hydroxy, alcoxy inférieur ou $NR^8R^9$, $R^8$ et $R^9$ représentent chacun l'hydrogène ou un groupe alkyle inférieur, n est égal à 0 ou 1 et m est un nombre de 1 à 7, étant spécifié que les groupes appelés »inférieurs« contiennent au maximum 7 atomes de charbone, et en outre que:

(i) lorsque $R^3$ représente un groupe acyloxy inférieur ou hydroxy, A représente le groupe (a), X représente l'hydrogène, un halogène, un groupe trifluorométhyle, éthyle ou acétyle, et, si $R^1$ représente le groupe $-NH(CH_2)_mNR^8R^9$, $R^8$ et $R^9$ représentent chacum un groupe alkyle inférieur;
(ii) lorsque A représente le groupe (d) et $R^1$ le groupe $-NH(CH_2)_mNR^8R^9$, $R^8$ et $R^9$ représentent chacun un groupe alkyle inférieur; et
(iii) lorsque n est égal à 1, ou bien A représente le groupe (b) et $R^1$ représente l'hydrogène, un groupe alkyle inférieur, hydroxy, alcoxy inférieur, $NR^{41}R^{51}$ (dans lequel $R^{41}$ et $R^{51}$ représentent chacun l'hydrogène ou un groupe alkyle inférieur ou bien forment ensemble et avec l'atome d'azote un hétérocycle de 5 à 7 chaînons qui peut contenir un atome d'oxygène), le chlore, le brome ou le groupe $-CH_2-CO-R^7$, ou bien A représente le groupe (a) et $R^1$ représente l'hydrogène, un groupe alkyle inférieur, alcoxy inférieur, le chlore, le brome ou le groupe $-CH_2-CO-R^7$;

et leurs sels formés par addition avec des acides acceptables pour l'usage pharmaceutique.

2. Composés selon la revendication 1, dans lesquels A représente le groupe (b), $R^1$ représente un groupe amino, n est égal à 0, X représente l'hydrogène, un halogène de numéro atomique 35 au maximum ou un groupe trifluorométhyle et Y représente l'hydrogène ou un halogène de numéro atomique 35 au maximum.

3. Composés selon la revendication 1, dans lesquels $R^1$ représente l'hydrogène, le chlore, le brome, un groupe alkyle inférieur, le groupe $NR^8R^9$ (dans lequel $R^8$ et $R^9$ représentent chacun l'hydrogène ou un groupe alkyle inférieur), le groupe $-CH_2-CO-R^7$ (dans lequel $R^7$ est un groupe alcoxy inférieur), di-(alkyle inférieur)-amino-(alkyle inférieur)-amino, hydroxy, alcoxy inférieur, mercapto ou alkylmercapto inférieur, étant spécifié que:

(i) lorsque A représente le groupe (b), (c) ou (d) ou bien lorsque A représente le groupe (a) et que $R^3$ représente un groupe acyloxy inférieur ou hydroxy, $R^1$ représente l'hydrogène, un groupe alkyle inférieur ou $NR^8R^9$;
(ii) lorsque n est égal à 1, A représente le groupe (b); et
(iii) lorsque A représente le groupe (b), $R^1$ un groupe amino et Y l'hydrogène ou un halogène de numéro atomique 35 qu maximum, X ne peut représenter l'hydrogène, un halogène de numéro atomique 35 au maximum ou un groupe trifluorométhyle.

4. Composés selon la revendication 1, dans lesquels A représente le groupe (a) et n est égal à 0.

5. Composés selon la revendication 4, dans lesquels $R^3$ représente l'hydrogène et $R^1$ l'hydrogène, un groupe alkyle inférieur, le groupe $NR^4R^5$ (dans lequel $R^4$ et $R^5$ représentent chacun l'hydrogène ou un groupe alkyle inférieur), hydroxy, le chlore, le brome, le groupe $-NH(CH_2)_mNR^8R^9$ (dans lequel $R^8$ et $R^9$ représentent chacun un groupe alkyle inférieur) ou le groupe $-CH_2-CO-R^7$.

6. Composés selon la revendication 5, dans lesquels $R^1$ représente l'hydrogène, un groupe amino ou alkyle inférieur.

7. Composés selon la revendication 4, dans lesquels $R^3$ représente un groupe hydroxy et $R^1$ l'hydrogène, un groupe alkyle inférieur ou le groupe $NR^4R^5$ (dans lequel $R^4$ et $R^5$ représentent chacun l'hydrogène ou un groupe alkyle inférieur).

8. Composés selon l'une des revendications 4 à 7, dans lesquels X représente un halogène.

9. Composés selon la revendication 8, dans lesquels X représente le chlore.

10. Composés selon l'une des revendications 4 à 9, dans lesquels Y représente l'hydrogène, le chlore ou le fluor.

11. La 9-chloro-7-(2-chlorophényl)-5H-pyrimido[5,4-d] [2]benzazépine.

12. La 9-chloro-7-(2-fluorophényl)-5H-pyrimido[5,4-d] [2]benzazépine.

13. La 9-chloro-7-(2-chlorophényl)-2-méthyl-5H-pyrimido[5,4-d] [2]benzazépine.

14. La 9-chloro-7-(2-fluorophényl)-5H-pyrimido[5,4-d] [2]benzazépine-2-ol.

15. La 9-chloro-N,N-diméthyl-7-(2-fluorophényl)-5H-pyrimido[5,4-d] [2]benzazépine-2-amine.

16. Composés de formules générales

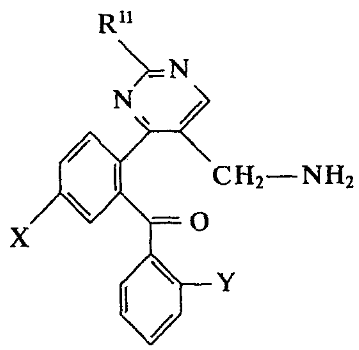

(II)

et

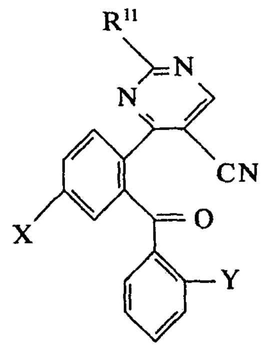

(XII)

dans lesquelles X et Y ont les significations indiquées dans la revendication 1 et $R^{11}$ représente l'hydrogène, un groupe alkyle inférieur ou $NR^8R^9$ dans lequel $R^8$ et $R^9$ ont les significations indiquées dans la revendication 1.

17. Composés selon la revendication 16, dans lesquels X et Y représentent chacun le chlore et $R^{11}$ l'hydrogène.

18. Composés selon l'une des revendications 1 à 10 et 12 à 15, en tant que substances actives pharmaceutiques.

19. Composés selon l'une des revendications 1 à 10 et 12 à 15, en tant qu'anxiolytiques et sédatifs.

20. La 9-chloro-7-(2-chlorophényl)-5H-pyrimido[5,4-d] [2]benzazépine en tant que substance active pharmaceutique.

21. La 9-chloro-7-(2-chlorophényl)-5H-pyrimido[5,4-d] [2]benzazépine en tant qu'anxiolytique et sédatif.

22. Procédé de préparation des composés selon l'une des revendications 1 à 15, caractérisé en ce que:

a) on cyclise un composé de formule générale

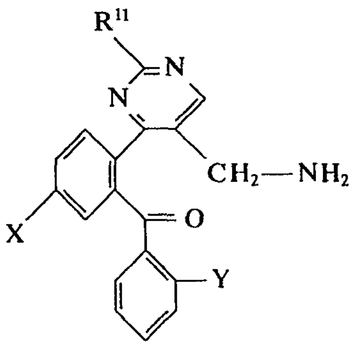

(II)

dans laquelle X et Y ont les significations indiquées dans la revendication 1, et $R^{11}$ représente l'hydrogène, un groupe alkyle inférieur ou $NR^8R^9$ dans lequel $R^8$ et $R^9$ ont les significations indiquées dans la revendication 1, ou bien

b) on soumet à déshydrogénation un composé de formule générale

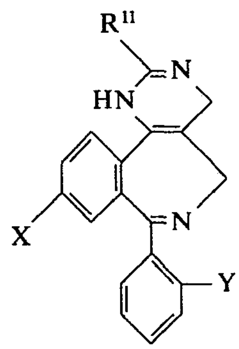

(III)

dans laquelle X et Y ont les significations indiquées dans la revendication 1 et $R^{11}$ la signification ci-dessus, ou bien

c) on fait réagir un composé de formule générale

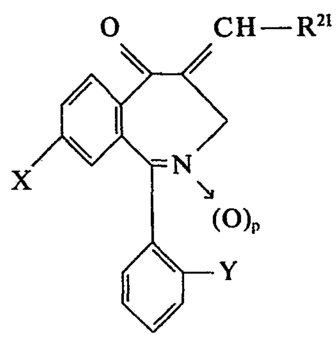

(IV)

dans laquelle X et Y ont les significations indiquées dans la revendication 1, p est égal à 0 ou 1 et $R^{21}$ représente un groupe di-(alkyle inférieur)-amino, avec un composé de formule générale

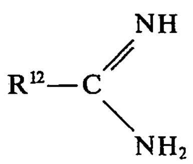

(V)

dans laquelle $R^{12}$ représente l'hydrogène, un groupe mercapto, alkylmercapto inférieur, alkyle inférieur ou $NR^8R^9$ dans lequel $R^8$ et $R^9$ ont les significations indiquées dans la revendication 1, ou bien

d) on réduit un composé de formule générale

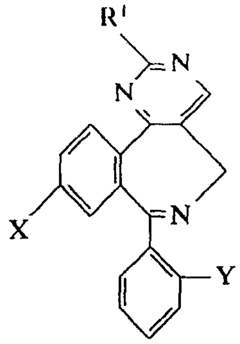

(Ia)

dans laquelle X, Y et $R^1$ ont les significations indiquées dans la revendication 1, ou bien

e) on introduit un groupe alkyle inférieur dans un composé de formule générale

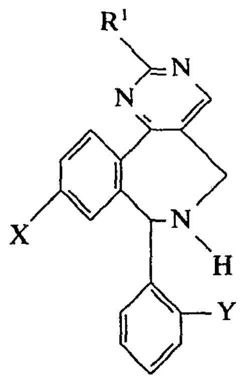

(Ib)

dans laquelle X, Y et $R^1$ ont les significations indiquées dans la revendication 1, ou bien

f) on oxyde un composé de formule générale

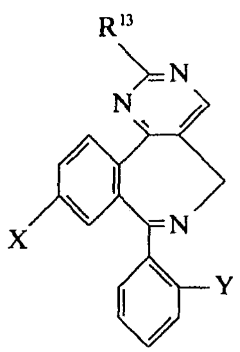

(Ic)

dans laquelle X et Y ont les significations indiquées dans la revendication 1, et $R^{13}$ représente l'hydrogène, un groupe alkyle inférieur, hydroxy, alcoxy, inférieur, $NR^{41}R^{51}$ (dans lequel $R^{41}$ et $R^{51}$ représentent chacun l'hydrogène ou un groupe alkyle inférieur ou bien forment ensemble et avec l'atome d'azote un hétérocycle de 5 à 7 chaînons que peut contenir un atome d'oxygène), le chlore, le brome ou le groupe $-CH_2-CO-R^7$ (dans lequel $R^7$ a la signification indiquée dans la revendication 1), ou bien

g) on introduit un substituant alkyle inférieur dans un composé de formule générale

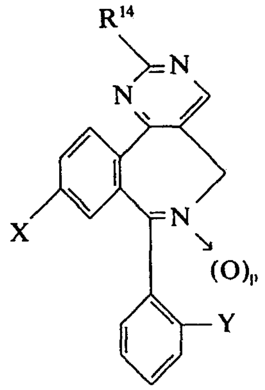

(Id)

dans laquelle X et Y ont les significations indiquées dans la revendication 1, p a la signification indiquée ci-dessus et $R^{14}$ représente un groupe mercapto ou hydroxy, ou bien

h) on convertit un composé de formule générale

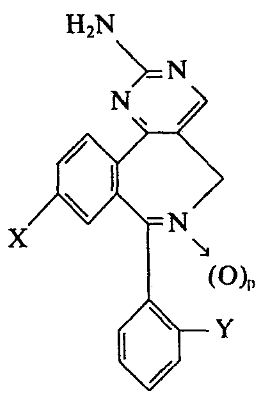

(Ie)

dans laquelle X et Y ont les significations indiquées dans la revendication 1 et p la signification ci-dessus, en le composé correspondant hydroxylé en position 2, ou bien

i) on convertit un composé de formule générale

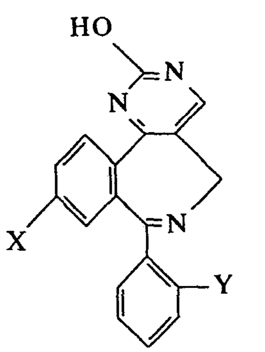

(If)

dans laquelle X et Y ont les significations indiquées dans la revendication 1, en le composé correspondant chloré ou bromé en position 2, ou bien

k) on traite un composé de formule générale

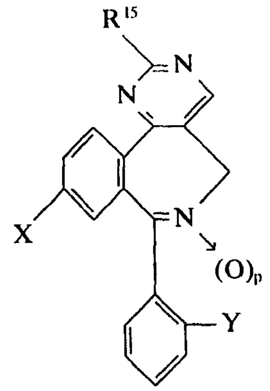

(Ig)

dans laquelle X et Y ont les significations indiquées dans la revendication 1, p la signification ci-dessus et $R^{15}$ représente le chlore ou le brome,
par le sulfure d'hydrogène, par un alkylmercaptan inférieur, par un alcanol inférieur, par un composé de formule $HNR^4R^5$ dans laquelle $R^4$ et $R^5$ ont les significations indiquées dans la revendication 1, par un composé de formule $H_2N-(CH_2)_mNR^8R^9$ dans laquelle $R^8$, $R^9$ et m ont les signification indiquées dans la revendication 1, ou par le carbanion d'un composé de formule

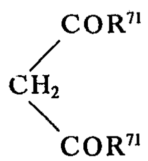 ou 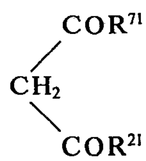

dans laquelle $R^{71}$ représente un groupe alcoxy inférieur et $R^{21}$ a la signification indiquée ci-dessus ou bien.

l) on convertit un composé de formule générale

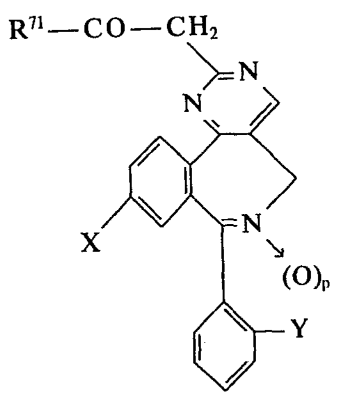

(Ih)

dans laquelle X et Y ont les significations indiquées dans la revendication 1, et $R^{71}$ et p les significations indiquées ci-dessus,
en l'acide libre correspondant ou en un amide, alkylamide inférieur ou di-(alkyle inférieur)-amide correspondant, ou bien

m) on fait réagir un composé de formule générale

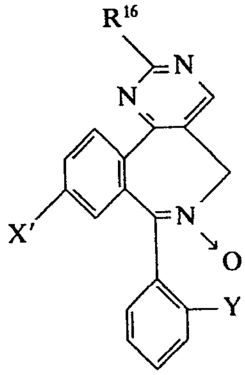

(Ii)

dans laquelle Y a la signification indiquée dans la revendication 1, $R^{16}$ représente l'hydrogène, le chlore, le brome, un groupe alkyle inférieur, le groupe $NR^4R^5$, le groupe $-CH_2-CO-R^7$, le groupe $NH(CH_2)_mNR^{81}R^{91}$, un groupe hydroxy, alcoxy inférieur, mercapto ou alkylmercapto inférieur, X' représente l'hydrogène, un halogène, un groupe trifluorométhyle, éthyle ou acétyle et $R^{81}$ et $R^{91}$ représentent chacun un groupe alkyle inférieur, $R^4$, $R^5$ et $R^7$ ayant les significations indiquées dans la revendication 1, avec un agent acylant inférieur, ou bien

n) on hydrolyse un composé de formule générale

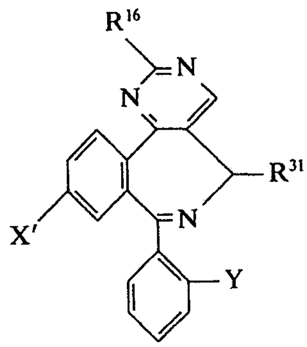

(Ik)

dans laquelle Y a la signification indiquée dans la revendication 1 et X' et $R^{16}$ les significations ci-dessus, $R^{31}$ représentant un groupe acyloxy inférieur, ou bin

o) on soumet à désoxydation un composé de formule générale

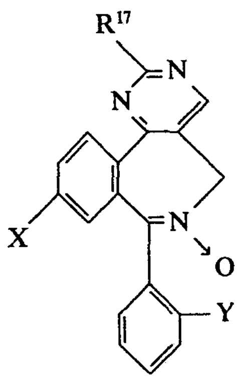

(Il)

dans laquelle X et Y ont les significations indiquées dans la revendication 1, $R^{17}$ représente l'hydrogène un groupe alkyle inférieur, le groupe $NR^4R^5$, le groupe $-CH_2-CO-R^7$, le groupe $NH-(CH_2)_m-NR^8R^9$, un groupe hydroxy, alcoxy inférieur, mercapto ou alkyl-mercapto inférieur et $R^4$, $R^5$, $R^7$, $R^8$ et $R^9$ ont les significations indiquées dans la revendication 1, ou bien

p) dans un composé de formule générale

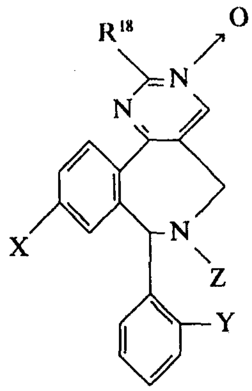

(III')

dans laquelle X et Y ont les significations indiquées dans la revendication 1, et $R^{18}$ représente l'hydrogène, un groupe alkyle inférieur, alcoxy inférieur, le chlore, le brome ou le groupe $-CH_2-CO-R^7$ (dans lequel $R^7$ a la signification indiquée dans la revendication 1) et Z représente l'hydrogène ou un groupe acyle facile à éliminer, on élimine les éléments de H—Z, ou bien

q) on convertit un composé de formule I définie dans la revendication 1 en un sel par addition avec un acide acceptable pour l'usage pharmaceutique.

23. Procédé selon la revendication 22, dans lequel on prépare la 9-chloro-7-(2-chlorophényl)-5H-pyrimido[5,4-d] [2]benzazépine.

24. Médicament contenant un composé selon l'une des revendications 1 à 10 et 12 à 15.

25. Anxiolytique et sédatif contenant un composé selon l'une des revendications 1 à 10 et 12 à 15.

26. Médicament contenant de la 9-chloro-7-(2-chlorophényl)-5H-pyrimido[5,4-d] [2]benzazépine ou un sel de ce composé formé par addition avec un acide acceptable pour l'usage pharmaceutique.

27. Anxiolytique et sédatif contenant de la 9-chloro-7-(2-chlorophényl)-5H-pyrimido[5,4-d] [2]benzazépine ou un sel de ce composé formé par addition avec un acide acceptable pour l'usage pharmaceutique.

**Revendications pour l'Etat contractant: AT**

1. Procédé de préparation de pyrimido-2-benzazépines de formule générale

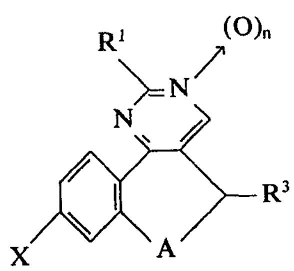

(I)

dans laquelle A représente l'un des groupes

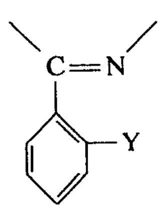

(a)

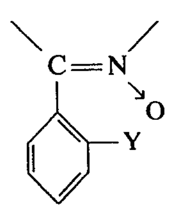

(b)

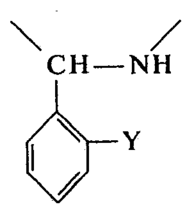

(c)

et

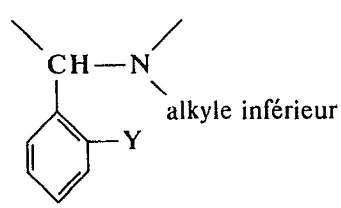

(d)

$R^1$ représente l'hydrogène, le chlore, le brome, un groupe alkyle inférieur, le groupe $NR^4R^5$, le groupe $-CH_2-CO-R^7$, le groupe $-NH(CH_2)_mNR^8R^9$, un groupe hydroxy, alcoxy inférieur, mercapto ou alkylmercapto inférieur, $R^3$ représente l'hydrogène, un groupe acyloxy inférieur ou hydroxy, X représente l'hydrogène, un halogène, un groupe trifluorométhyle, éthyle, alpha-hydroxyéthyle ou acétyle, Y représente l'hydrogène ou un halogène, $R^4$ et $R^5$ représentent chacun l'hydrogène ou un groupe alkyle inférieur ou bien forment ensemble et avec l'atome d'azote un hétérocycle de 5 à 7 chaînons qui peut

contenir un atome d'oxygène ou de soufre ou le groupe >N-alkyle inférieur, $R^7$ représente un groupe hydroxy, alcoxy inférieur ou $NR^8R^9$, $R^8$ et $R^9$ représentent chacun l'hydrogène ou un groupe alkyle inférieur, n est égal à 0 ou 1 et m est un nombre de 1 à 7, étant spécifié que les groupes appelés »inférieurs« contiennent au maximum 7 atomes de carbone, et en outre que:

(i) lorsque $R^3$ représente un groupe acyloxy inférieur ou hydroxy, A représente le groupe (a), X représente l'hydrogène, un halogène, un groupe trifluorométhyle, éthyle ou acétyle, et, si $R^1$ représente le groupe $-NH(CH_2)_mNR^8R^9$, $R^8$ et $R^9$ représentent chacun un groupe alkyle inférieur;
(ii) lorsque A représente le groupe (d) et $R^1$ le groupe $-NH(CH_2)_mNR^8R^9$, $R^8$ et $R^9$ représente chacun un groupe alkyle inférieur; et
(iii) lorsque n est égal à 1, ou bien A représente le groupe (b) et $R^1$ représente l'hydrogène, un groupe alkyle inférieur,hydroxy, alcoxy inférieur, $NR^{41}R^{51}$ (dans lequel $R^{41}$ et $R^{51}$ représentent chacun l'hydrogène ou un groupe alkyle inférieur ou bien forment ensemble et avec l'atome d'azote un hétérocycle de 5 à 7 chaînons qui peut contenir un atome d'oxygène), le chlore, le brome ou le groupe $-CH_2-CO-R^7$, ou bien A représente le groupe (a) et $R^1$ représente l'hydrogène, un groupe alklye inférieur, alcoxy inférieur, le chlore, le brome ou le groupe $-CH_2-CO-R^7$;

et de leurs sels formés par addition avec des acides acceptables pour l'usage pharmaceutique, caractérisé en ce que:

a) on cyclise un composé de formule générale

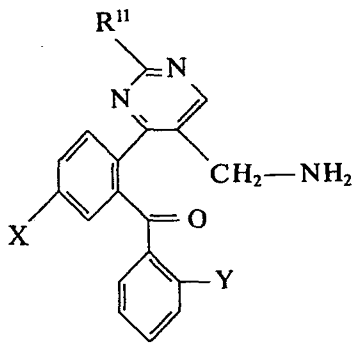

(II)

dans laquelle X et Y ont les significations indiquées ci-dessus et $R^{11}$ représente l'hydrogène, un groupe alkyle inférieur ou $NR^8R^9$ dans lequel $R^8$ et $R^9$ ont les significations indiquées ci-dessus, ou bien

b) on soumet un composé de formule générale

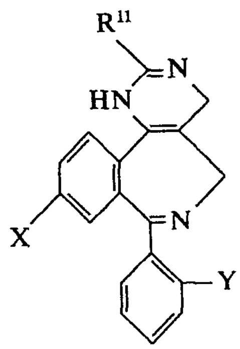

(III)

dans laquelle X, Y et $R^{11}$ ont les significations ci-dessus, à déshydrogénation, ou bien

c) on fait réagir un composé de formule générale

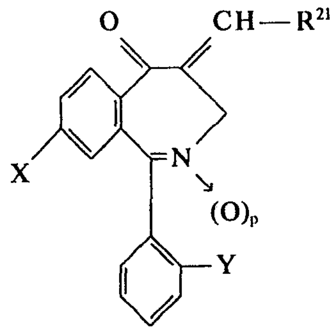

(IV)

dans laquelle X et Y ont les significations indiquées ci-dessus, p est égal à 0 ou 1 et $R^{21}$ représente un groupe di-(alkyle inférieur)-amino, avec un composé de formule générale

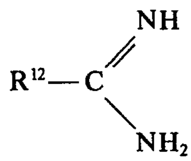

(V)

dans laquelle $R^{12}$ représente l'hydrogène, un groupe mercapto, alkylmercapto inférieur, alkyle inférieur ou $NR^8R^9$ dans lequel $R^8$ et $R^9$ ont les significations indiquées ci-dessus, ou bien

d) on réduit un composé de formule générale

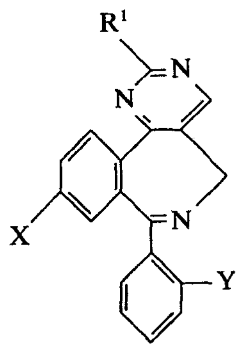

(Ia)

dans laquelle X, Y et $R^1$ ont les significations indiquées ci-dessus, ou bien

e) on introduit un groupe alkyle inférieur dans un composé de formule générale

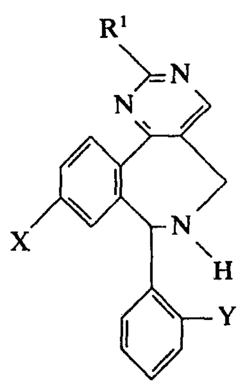

(Ib)

dans laquelle X, Y et $R^1$ ont les significations indiquées ci-dessus, ou bien

f) on oxyde un composé de formule générale

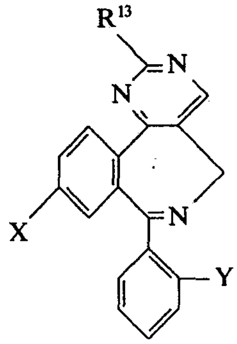

(Ic)

dans laquelle X et Y ont les significations indiquées ci-dessus et $R^{13}$ représente l'hydrogène, un groupe alkyle inférieur, hydroxy, alcoxy inférieur, $NR^{41}R^{51}$ (dans lequel $R^{41}$ et $R^{51}$ représentent chacun l'hydrogène ou un groupe alkyle inférieur ou bien forment ensemble et avec l'atome d'azote un hétérocycle de 5 à 7 chaînons qui peut contenir un atome d'oxygène), le chlore, le brome ou le groupe $-CH_2-CO-R^7$ (dans lequel $R^7$ a les significations indiquées ci-dessus), ou bien

g) on introduit un groupe alkyle inférieur dans un composé de formule générale

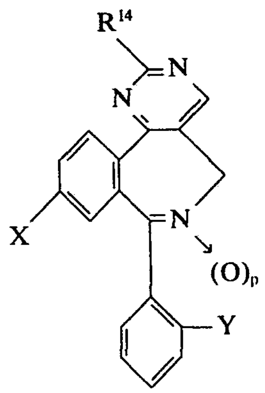

(Id)

dans laquelle X, Y et p ont les significations indiquées ci-dessus et $R^{14}$ représente un groupe mercapto ou hydroxy, ou bien

h) on convertit un composé de formule générale

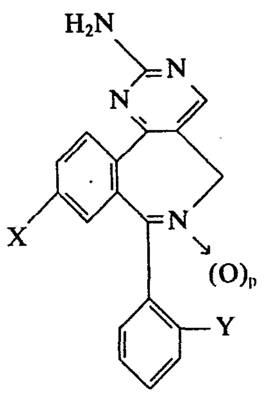

(Ie)

dans laquelle X, Y et p ont les significations ci-dessus, en le composé correspondant portant un groupe hydroxy en position 2, ou bien

i) on convertit un composé de formule générale

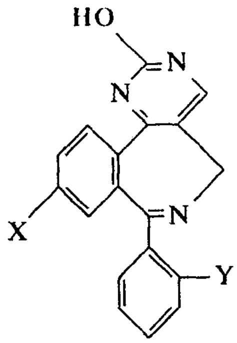

(If)

dans laquelle X et Y ont les significations indiquées ci-dessus, en un composé correspondant chloré ou bromé en position 2, ou bien

k) on traite un composé de formule générale

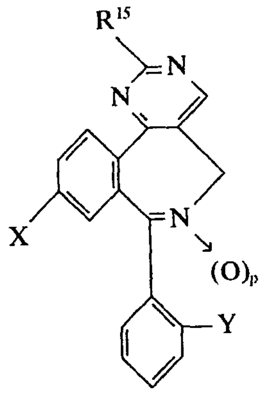

(Ig)

dans laquelle X, Y et p ont les significations indiquées ci-dessus et $R^{15}$ représente le chlore ou le brome,
avec le sulfure d'hydrogène, avec un alkylmercaptan inférieur, avec un alcanol inférieur, avec un composé de formule $HNR^4R^5$ dans laquelle $R^4$ et $R^5$ ont les significations ci-dessus, avec un composé de formule $H_2N-(CH_2)_mNR^8R^9$ dans laquelle $R^8$ et $R^9$ et m ont les signification ci-dessus, ou avec le carbanion d'un composé de formule

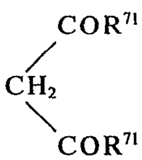 ou 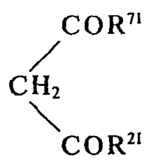

dans laquelle $R^{71}$ représente un groupe alcoxy inférieur et $R^{21}$ a la signification indiquée ci-dessus, ou bien

l) on convertit un composé de formule générale

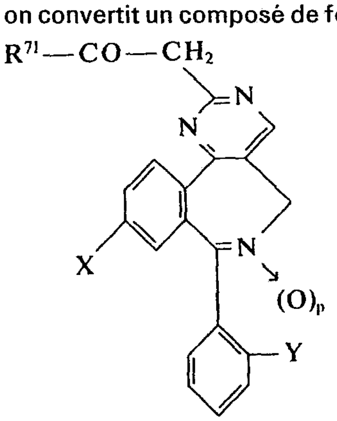

(Ih)

dans laquelle $R^{71}$, X, Y et p ont les significations indiquées ci-dessus, en l'acide libre correspondant ou en un amide, alkylamide inférieur ou di-(alkyle inférieur)-amide correspondant, ou bien

m) on fait réagir un composé de formule générale

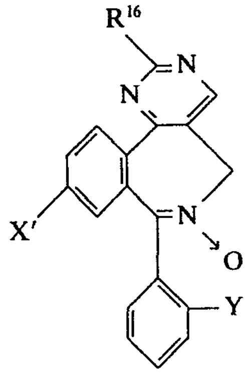

(Ii)

dans laquelle Y a la signification ci-dessus, $R^{16}$ représente l'hydrogène, le chlore, le brome, un groupe alkyle inférieur, le groupe $NR^4R^5$, le groupe $-CH_2-CO-R^7$, le groupe $NH(CH_2)_mNR^{81}R^{91}$, un groupe hydroxy, alcoxy inférieur, mercapto ou alkylmercapto inférieur, X' représente l'hydrogène, un halogène, un groupe trifluorométhyle, éthyle ou acétyle et $R^{81}$ et $R^{91}$ représentent chacun un groupe alkyle inférieur, $R^4$, $R^5$ et $R^7$ ayant les significations ci-dessus, avec un agent acylant inférieur, ou bien

n) on hydrolyse un composé de formule générale

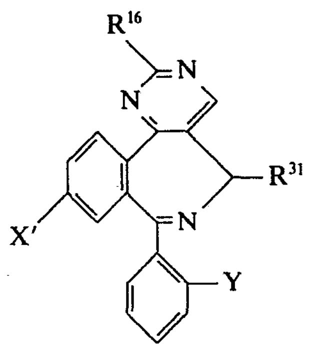

(Ik)

dans laquelle X', Y et $R^{16}$ ont les significations ci-dessus et $R^{31}$ représente un groupe acyloxy inférieur ou bien

o) on soumet à désoxygénation un composé de formule générale

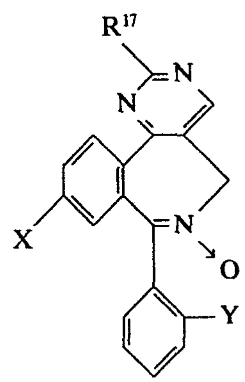

(Il)

dans laquelle X et Y ont les significations ci-dessus, $R^{17}$ représente l'hydrogène un groupe alkyle inférieur, le groupe $NR^4R^5$, le groupe $-CH_2-CO-R^7$, le groupe $NH-(CH_2)_m-NR^8R^9$, un groupe hydroxy, alcoxy inférieur, mercapto ou alkyl-mercapto inférieur et $R^4$, $R^5$, $R^7$, $R^8$ et $R^9$ ont les significations ci-dessus, ou bien

p) dans un composé de formule générale

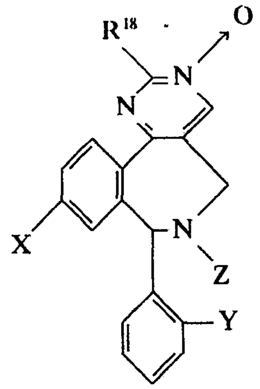

(III')

dans laquelle X et Y ont les significations ci-dessus et $R^{18}$ représente l'hydrogène, un groupe alkyle inférieur, alcoxy inférieur, le chlore, le brome ou le groupe $-CH_2-CO-R^7$ (dans lequel $R^7$ a la signification ci-dessus) et Z représente l'hydrogène ou un groupe acyle facile à éliminer, on élimine les éléments de $H-Z$, ou bien

q) on convertit un composé de formule I en un sel par addition avec un acide acceptable pour l'usage pharmaceutique.

2. Procédé selon la revendication 1, caractérisé en ce que l'on prépare des composés de formule I dans laquelle A représente le groupe (b), $R^1$ représente un groupe amino, n est égal à 0, X représente l'hydrogène, un halogène de numéro atomique 35 au maximum ou un groupe trifluorométhyle, et Y représente l'hydrogène ou un halogène de numéro atomatique 35 au maximum, et en ce que l'on prépare ces composés par la variante opératoire (c) pour laquelle X et Y ont les significations indiquées ci-dessus et $R^{21}$ représente un groupe diméthylamino, p est égal à 1 et $R^{12}$ représente un groupe amino.

3. Procédé selon la revendication 1, caractérisé en ce que l'on prépare des composés de formule I dans laquelle $R^1$ représente l'hydrogène, le chlore, le brome, un groupe alkyle inférieur, le groupe $NR^8R^9$ (dans lequel $R^8$ et $R^9$ représentent chacun l'hydrogène ou un groupe alkyle inférieur), le groupe $-CH_2-CO-R^7$ (dans lequel $R^7$ représente un groupe alcoxy inférieur), un groupe di-(alkyle inférieur)-amino-(alkyle inférieur)-amino, hydroxy, alcoxy inférieur, mercapto ou alkylmercapto inférieur, étant spécifié que:

(i) lorsque A représente le groupe (b), (c) ou (d) ou lorsque A représente le groupe (a) et $R^3$ un groupe acyloxy inférieur ou hydroxy, $R^1$ représente l'hydrogène, un groupe alkyle inférieur ou $NR^8R^9$; et
(ii) lorsque n est égal à 1, A représente le groupe (b);

ou leurs sels formés par addition avec des acides acceptables pour l'usage pharmaceutique et en ce que l'on opère par les variantes opératoires (a), (b), (i) ou (q); ou bien on opère par la variante opératoire (c) pour laquelle $R^{21}$ représente un groupe diméthylamino et $R^{12}$ l'hydrogène, un groupe mercapto, alkyle inférieur ou $NR^8R^9$, étant spécifié que p est égal à 0 lorsque $R^{12}$ représente un groupe mercapto ou bien lorsque $R^{12}$ représente un groupe amino, X l'hydrogène, un halogène de numéro atomique 35 au maximum ou un groupe trifluorométhyle et Y l'hydrogène ou un halogène de numéro atomique 35 au maximum; ou bien on opère selon la variante opératoire (d) ou (e), pour laquelle $R^1$ représente l'hydrogène, un groupe alklye inférieur ou $NR^8R^9$; ou bien on opère selon la variante opératoire (f), pour laquelle $R^{13}$ représente l'hydrogène, un groupe alkyle inférieur ou $NR^8R^9$; ou bien on opère selon la variante opératoire (g) ou (h), pour laquelle p est égal à 0; ou bien on opère par la variante opératoire (k), en convertissant un composé de formule Ig dans laquelle p est égal à 0 en un composé correspondant de formule I dans laquelle $R^1$ représente un groupe alcoxy inférieur, alkylamino inférieur, di-(alkyle inférieur)-amino, di-(alkyle inférieur)-amino-(alkyle inférieur)-amino ou bien le groupe $-CH_2COR^{71}$; ou bien on opère par la variante opératoire (m) ou (n), pour laquelle $R^{16}$ représente l'hydrogène, un groupe alkyle inférieur ou $NR^8R^9$.

4. Procédé selon la revendication 1, caractérisé en ce que l'on prépare des composés de formule I dans laquelle A représente le groupe (a) et n est égal à 0.

5. Procédé selon la revendication 4, caractérisé en ce que l'on prépare des composés de formule I dans laquelle $R^3$ représente l'hydrogène et $R^1$ l'hydrogène, un groupe alkyle inférieur, le groupe $NR^4R^5$ (dans lequel $R^4$ et $R^5$ représentent chacun l'hydrogène ou un groupe alkyle inférieur), un groupe hydroxy, le chlore, le brome, le groupe $-NH(CH_2)_mNR^8R^9$ (dans lequel $R^8$ et $R^9$ représentent chacun un groupe alkyle inférieur) ou le groupe $-CH_2-CO-R^7$.

6. Procédé selon la revendication 5, caractérisé en ce que l'on prépare des composés de formule I dans laquelle $R^1$ représente l'hydrogène, un groupe amino ou alkyle inférieur.

7. Procédé selon la revendication 4, caractérisé en ce que l'on prépare des composés de formule I dans laquelle $R^3$ représente un groupe hydroxy et $R^1$ l'hydrogène, un groupe alkyle inférieur ou le groupe $NR^4R^5$ (dans lequel $R^4$ et $R^5$ représentent chacun l'hydrogène ou un groupe alkyle inférieur).

8. Procédé selon l'une des revendications 4 à 7, caractérisé en ce que l'on prépare des composés de formule I dans laquelle X représente un halogène.

9. Procédé selon la revendication 8, caractérisé en ce que l'on prépare des composés de formule I dans laquelle X représente le chlore.

10. Procédé selon des revendications 4 à 9, caractérisé en ce que l'on prépare des composés de formule I dans laquelle Y représente l'hydrogène, le chlore ou le fluor.

11. Procédé selon la revendication 1, caractérisé en ce que l'on prépare la 9-chloro-7-(2-chlorophényl)-5H-pyrimido[5,4-d] [2]benzazépine.

12. Procédé selon la revendication 1, caractérisé en ce que l'on prépare la 9-chloro-7-(2-fluorophényl)-5H-pyrimido[5,4-d] [2]benzazépine.

13. Procédé selon la revendication 1, caractérisé en ce que l'on prépare la 9-chloro-7-(2-chlorophényl)-2-méthyl-5H-pyrimido[5,4-d] [2]benzazépine.

14. Procédé selon la revendication 1, caractérisé en ce que l'on prépare le 9-chloro-7-(2-fluorophényl)-5H-pyrimido[5,4-d] [2]benzazépine-2-ol.

15. Procédé selon la revendication 1, caractérisé en ce que l'on prépare la 9-chloro-N,N-diméthyl-7-(2-fluorophényl)-5H-pyrimido[5,4-d] [2]benzazépine-2-amine.